# EUROPEAN PATENT APPLICATION

(11) **EP 4 834 374 A1**
(43) Date of publication of application: **04.11.2026**
(21) Application number: 24911533.8
(22) Date of filing: 27.12.2024
(51) Int. Cl.: A61K 47/68, A61K 38/16, A61K 38/26, A61P 3/00, C07D 519/00

(54) **ANTIBODY-POLYPEPTIDE CONJUGATE AND PHARMACEUTICAL USE THEREOF**

(30) Priority: 29.12.2023 CN 202311853381
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: MA, Xiaochuan, Shanghai 200245 (CN); LI, Ruihua, Shanghai 200245 (CN); WU, Fangzhou, Lianyungang, Jiangsu 222047 (CN); YE, Xin, Shanghai 200245 (CN); WANG, Lei, Lianyungang, Jiangsu 222047 (CN); ZHANG, Lianshan, Lianyungang, Jiangsu 222047 (CN); HE, Feng, Shanghai 200245 (CN)
(74) Representative: Regimbeau
(86) International application number: PCT/CN2024/143340
(87) International publication number: WO 2025/140617

(57) **Abstract**

An antibody-polypeptide conjugate and pharmaceutical use thereof. Specifically, the present invention relates to an antibody-polypeptide conjugate or a pharmaceutically acceptable salt thereof and pharmaceutical use thereof.

## Description

### Technical Field

The present disclosure belongs to the field of biotechnology, and relates to an antibody-polypeptide conjugate and the pharmaceutical use thereof.

### Background Art

The statements herein are merely to provide background information relevant to the present disclosure and do not necessarily constitute the prior art.

FGF21 is a multifunctional regulator that is produced primarily by the liver and is also expressed in adipose tissue, pancreas and skeletal muscle. It is secreted into the blood and is involved in regulating the body's metabolism. By binding its cell membrane surface receptor KLB, FGF21 can activate KLB&FGFR1c and its downstream signalling pathway, playing a role in regulating carbohydrate metabolism, lipid metabolism, and energy expenditure, etc.

Endocrine peptides such as glucagon-like peptide-1 (GLP-1), glucagon (GCG), and glucose-dependent insulinotropic polypeptide (GIP), which are secreted by the intestine or liver, can broadly regulate blood glucose levels, lipid metabolism, feeding behaviour and other important physiological processes related to metabolism by activating GLP-1R, GCGR and GIPR, respectively.

Clinically, FGF21 fusion proteins have shown some clinical efficacy in the treatment of non-alcoholic steatohepatitis (NASH), while improvements in NASH patients treated with GLP-1/GIP/GCG mono- or multi-activating peptides have also been demonstrated.

Both preclinical and clinical studies have shown that FGF21 signalling and GLP-1 are mechanistically complementary (Qi Pan et al., EBioMedicine, 63, 2021, 103202; Allegra Kaufman et al., Cell Reports Medicine, 2020, 1 (4): 100057; Leiluo Geng et al., Nature Reviews Endocrinology, 2020, 16 (11): 654-667; Kyle H. Flippo et al., Nature metabolism, 2021, 3 (3): 309-317), and their combination may have a better therapeutic effect. Currently, the industry has focused largely on developing bifunctional molecules in the form of fusions (e.g., WO 2022003169 A1). These drug forms have a short half-life and can only be administered once a week. Therefore, there is a strong clinical need to develop multifunctional molecules with less frequent dosing, while avoiding issues such as druggability of fusion proteins.

### Summary of the Invention

The present disclosure provides an antibody-polypeptide conjugate or a pharmaceutically acceptable salt thereof, having the structure as represented by general formula (I):

Ab-(L-P)ₘ (I)

wherein
Ab is an antibody;
P is a glucagon-like peptide-1 receptor agonist peptide or an analogue thereof;
L is a linker connecting Ab and P;
wherein Ab binds directly to L via the amino acid residue at position 297, or Ab binds to L via the glycan chain or remodeled glycan chain at position 297;
m represents an integer from 0 to 10.

The present disclosure provides an antibody-polypeptide conjugate or a pharmaceutically acceptable salt thereof, having the structure as represented by general formula (I):

Ab-(L-P)ₘ (I)

wherein
Ab is an antibody;
P is a glucagon-like peptide-1 receptor agonist peptide or an analogue thereof;
L is a linker connecting Ab and P;
wherein Ab binds directly to L via the amino acid residue at position 297, or Ab binds to L via the glycan chain or remodeled glycan chain at position 297;
m represents an integer from 1 to 10.

In some embodiments, in the antibody-polypeptide conjugate or the pharmaceutically acceptable salt thereof as described above, Ab binds to L via the remodeled glycan chain at Asn297.

In some embodiments, in the antibody-polypeptide conjugate or the pharmaceutically acceptable salt thereof according to any one of the foregoing, the structure of the remodeled glycan chain is: 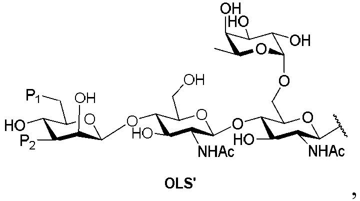
in the formula, the wavy line 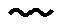 indicates binding to Asn at position 297 of the Pc heavy chain;
P₁ and P₂ are identical or different, and are each independently selected from the group consisting of hydroxyl, *-(CR^{p}R^{q}-CR^{s}R^{t}-O)s¹- and *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino; the asterisk * indicates binding to the linker L;
s¹ is 1-10, preferably 1-5; s² is 0-10, preferably 1-5; s³ is 1-10, preferably 1-5; s⁴ is 0-10, preferably 1-5;
provided that P₁ and P₂ are not both hydroxyl or *-(CH₂CH₂O)s¹-.

In some embodiments, in the antibody-polypeptide conjugate or the pharmaceutically acceptable salt thereof according to any one of the foregoing, wherein P₁ is hydroxyl, and P₂ is *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently selected from the group consisting of hydrogen atom, halogen, C₁₋₆ alkyl and C₁₋₆ haloalkyl, s² is 1-5, s³ is 1-5, and s⁴ is 1-5.

In some embodiments, in the antibody-polypeptide conjugate or the pharmaceutically acceptable salt thereof according to any one of the foregoing, wherein P₁ is hydroxyl, and P₂ is *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently selected from the group consisting of hydrogen, halogen, C₁₋₆ alkyl and C₁₋₆ haloalkyl, s² is 1, s³ is 1, and s⁴ is 1.

In some embodiments, in the antibody-polypeptide conjugate or the pharmaceutically acceptable salt thereof according to any one of the foregoing, wherein P₁ is hydroxyl, and P₂ is *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently hydrogen or C₁₋₆ alkyl, s² is 1-5, s³ is 1-5, and s⁴ is 1-5.

In some embodiments, in the antibody-polypeptide conjugate or the pharmaceutically acceptable salt thereof according to any one of the foregoing, wherein P₁ is hydroxyl, and P₂ is *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently hydrogen or C₁₋₆ alkyl, s² is 1, s³ is 1, and s⁴ is 1.

In some embodiments, in the antibody-polypeptide conjugate or the pharmaceutically acceptable salt thereof according to any one of the foregoing, the structure of the remodeled glycan chain is:

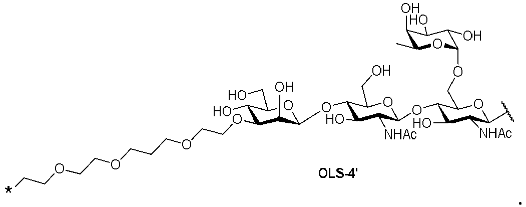

In some embodiments, the antibody-polypeptide conjugate or the pharmaceutically acceptable salt thereof according to any one of the foregoing has the structure as represented by general formula (II): 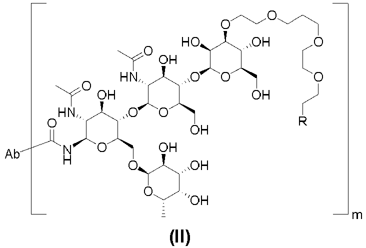
wherein R is -L-P;
Ab, L, P and m are as defined in (I).

In some embodiments, in the antibody-polypeptide conjugate or the pharmaceutically acceptable salt thereof according to any one of the foregoing, P is a glucagon-like peptide-1 receptor agonist peptide or an analogue thereof.

In some embodiments, in the antibody-polypeptide conjugate or the pharmaceutically acceptable salt thereof according to any one of the foregoing, P is a GLP-1R monoagonist peptide or a GLP-1R/GIPR/GCGR triagonist peptide or an analogue thereof.

In some embodiments, in the antibody-polypeptide conjugate or the pharmaceutically acceptable salt thereof according to any one of the foregoing, P is a GLP-1R monoagonist peptide or an analogue thereof.

In some embodiments, in the antibody-polypeptide conjugate or the pharmaceutically acceptable salt thereof according to any one of the foregoing, P is a GLP-1R/GIPR dual agonist peptide or an analogue thereof.

In some embodiments, in the antibody-polypeptide conjugate or the pharmaceutically acceptable salt thereof according to any one of the foregoing, P is a GLP-1R/GCGR dual agonist peptide or an analogue thereof.

In some embodiments, in the antibody-polypeptide conjugate or the pharmaceutically acceptable salt thereof according to any one of the foregoing, P is a GLP-1R/GIPR/GCGR triagonist peptide or an analogue thereof.

In some embodiments, in the antibody-polypeptide conjugate or the pharmaceutically acceptable salt thereof according to any one of the foregoing, P comprises the amino acid sequence of SEQ ID NO: 20, SEQ ID NO: 21, or SEQ ID NO: 22.

In some embodiments, in the antibody-polypeptide conjugate or the pharmaceutically acceptable salt thereof according to any one of the foregoing, the amino acid sequence of P is as set forth in SEQ ID NO: 20 or SEQ ID NO: 21.

In some embodiments, in the antibody-polypeptide conjugate or the pharmaceutically acceptable salt thereof according to any one of the foregoing, the amino acid sequence of P is as set forth in SEQ ID NO: 22.

In some embodiments, in the antibody-polypeptide conjugate or the pharmaceutically acceptable salt thereof according to any one of the foregoing, Ab is selected from the group consisting of IgG1, IgG2, IgG3 and IgG4 antibodies; preferably, Ab is an IgG1 antibody.

In some embodiments, in the antibody-polypeptide conjugate or the pharmaceutically acceptable salt thereof according to any one of the foregoing, Ab is a KLB antibody or an ActRIIA/B antibody.

In some embodiments, in the antibody-polypeptide conjugate or the pharmaceutically acceptable salt thereof according to any one of the foregoing, Ab is a KLB antibody.

In some embodiments, in the antibody-polypeptide conjugate or the pharmaceutically acceptable salt thereof according to any one of the foregoing, Ab is a KLB antibody; preferably, the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11.

In some embodiments, in the antibody-polypeptide conjugate or the pharmaceutically acceptable salt thereof according to any one of the foregoing, Ab is a KLB antibody; preferably, the amino acid sequence of the HCDR1 of the heavy chain variable region of Ab is as set forth in SEQ ID NO: 6, the amino acid sequence of the HCDR2 is as set forth in SEQ ID NO: 7, the amino acid sequence of the HCDR3 is as set forth in SEQ ID NO: 8, the amino acid sequence of the LCDR1 of the light chain variable region is as set forth in SEQ ID NO: 9, the amino acid sequence of the LCDR2 is as set forth in SEQ ID NO: 10, and the amino acid sequence of the LCDR3 is as set forth in SEQ ID NO: 11.

In some embodiments, in the antibody-polypeptide conjugate or the pharmaceutically acceptable salt thereof according to any one of the foregoing, Ab is an ActRIIA/B antibody.

In some embodiments, in the antibody-polypeptide conjugate or the pharmaceutically acceptable salt thereof according to any one of the foregoing, Ab is an ActRIIA/B antibody; preferably, the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28.

In some embodiments, in the antibody-polypeptide conjugate or the pharmaceutically acceptable salt thereof according to any one of the foregoing, Ab is an ActRIIA/B antibody; preferably, the amino acid sequence of the HCDR1 of the heavy chain variable region of Ab is as set forth in SEQ ID NO: 23, the amino acid sequence of the HCDR2 is as set forth in SEQ ID NO: 24, the amino acid sequence of the HCDR3 is as set forth in SEQ ID NO: 25, the amino acid sequence of the LCDR1 of the light chain variable region is as set forth in SEQ ID NO: 26, the amino acid sequence of the LCDR2 is as set forth in SEQ ID NO: 27, and the amino acid sequence of the LCDR3 is as set forth in SEQ ID NO: 28.

In some embodiments, in the antibody-polypeptide conjugate or the pharmaceutically acceptable salt thereof according to any one of the foregoing, Ab is a murine antibody, a chimeric antibody, a humanised antibody or a fully human antibody. In some embodiments, Ab is a chimeric antibody or a humanised antibody. In some embodiments, Ab is a humanised antibody.

In some embodiments, in the antibody-polypeptide conjugate or the pharmaceutically acceptable salt thereof according to any one of the foregoing, Ab comprises a framework region (FR) of a human antibody.

In some embodiments, in the antibody-polypeptide conjugate or the pharmaceutically acceptable salt thereof according to any one of the foregoing, Ab is a KLB antibody; preferably, the heavy chain variable region of Ab comprises the amino acid sequence of SEQ ID NO: 12, and the light chain variable region comprises the amino acid sequence of SEQ ID NO: 13.

In some embodiments, in the antibody-polypeptide conjugate or the pharmaceutically acceptable salt thereof according to any one of the foregoing, Ab is a KLB antibody; preferably, the amino acid sequence of the heavy chain variable region of Ab is as set forth in SEQ ID NO: 12, and the amino acid sequence of the light chain variable region is as set forth in SEQ ID NO: 13.

In some embodiments, in the antibody-polypeptide conjugate or the pharmaceutically acceptable salt thereof according to any one of the foregoing, Ab is an ActRIIA/B antibody; preferably, the heavy chain variable region of Ab comprises the amino acid sequence of SEQ ID NO: 29, and the light chain variable region comprises the amino acid sequence of SEQ ID NO: 30.

In some embodiments, in the antibody-polypeptide conjugate or the pharmaceutically acceptable salt thereof according to any one of the foregoing, Ab is an ActRIIA/B antibody; preferably, the amino acid sequence of the heavy chain variable region of Ab is as set forth in SEQ ID NO: 29, and the amino acid sequence of the light chain variable region is as set forth in SEQ ID NO: 30.

In some embodiments, in the antibody-polypeptide conjugate or the pharmaceutically acceptable salt thereof according to any one of the foregoing, Ab comprises a heavy chain constant region and a light chain constant region. In some embodiments, the heavy chain constant region is a human IgG1, IgG2, IgG3 or IgG4 heavy chain constant region. In some embodiments, the light chain constant region is a human κ or λ light chain constant region.

In some embodiments, in the antibody-polypeptide conjugate or the pharmaceutically acceptable salt thereof according to any one of the foregoing, the heavy chain constant region comprises the amino acid sequence of SEQ ID NO: 14 or 18, and the light chain constant region comprises the amino acid sequence of SEQ ID NO: 15. In some embodiments, in the antibody-polypeptide conjugate or the pharmaceutically acceptable salt thereof according to any one of the foregoing, the heavy chain constant region comprises the amino acid sequence of SEQ ID NO: 14, and the light chain constant region comprises the amino acid sequence of SEQ ID NO: 15. In some embodiments, the amino acid sequence of the heavy chain constant region is as set forth in SEQ ID NO: 14, and the amino acid sequence of the light chain constant region is as set forth in SEQ ID NO: 15.

In some embodiments, in the antibody-polypeptide conjugate or the pharmaceutically acceptable salt thereof according to any one of the foregoing, the heavy chain constant region comprises the amino acid sequence of SEQ ID NO: 18, and the light chain constant region comprises the amino acid sequence of SEQ ID NO: 15. In some embodiments, the amino acid sequence of the heavy chain constant region is as set forth in SEQ ID NO: 18, and the amino acid sequence of the light chain constant region is as set forth in SEQ ID NO: 15.

In some embodiments, in the antibody-polypeptide conjugate or the pharmaceutically acceptable salt thereof according to any one of the foregoing, the heavy chain constant region comprises the amino acid sequence of SEQ ID NO: 31, and the light chain constant region comprises the amino acid sequence of SEQ ID NO: 32. In some embodiments, the amino acid sequence of the heavy chain constant region is as set forth in SEQ ID NO: 31, and the amino acid sequence of the light chain constant region is as set forth in SEQ ID NO: 32.

In some embodiments, in the antibody-polypeptide conjugate or the pharmaceutically acceptable salt thereof according to any one of the foregoing, Ab is a KLB antibody; preferably, the heavy chain of Ab comprises the amino acid sequence of SEQ ID NO: 16 or 19, and the light chain comprises the amino acid sequence of SEQ ID NO: 17.

In some embodiments, in the antibody-polypeptide conjugate or the pharmaceutically acceptable salt thereof according to any one of the foregoing, Ab is a KLB antibody; preferably, the amino acid sequence of the heavy chain of Ab is as set forth in SEQ ID NO: 16 or 19, and the amino acid sequence of the light chain is as set forth in SEQ ID NO: 17.

In some embodiments, in the antibody-polypeptide conjugate or the pharmaceutically acceptable salt thereof according to any one of the foregoing, Ab is a KLB antibody; preferably, the amino acid sequence of the heavy chain of Ab is as set forth in SEQ ID NO: 16, and the amino acid sequence of the light chain is as set forth in SEQ ID NO: 17.

In some embodiments, in the antibody-polypeptide conjugate or the pharmaceutically acceptable salt thereof according to any one of the foregoing, Ab is a KLB antibody; preferably, the amino acid sequence of the heavy chain of Ab is as set forth in SEQ ID NO: 19, and the amino acid sequence of the light chain is as set forth in SEQ ID NO: 17.

In some embodiments, in the antibody-polypeptide conjugate or the pharmaceutically acceptable salt thereof according to any one of the foregoing, Ab is an ActRIIA/B antibody; preferably, the heavy chain of Ab comprises the amino acid sequence of SEQ ID NO: 33, and the light chain comprises the amino acid sequence of SEQ ID NO: 34.

In some embodiments, in the antibody-polypeptide conjugate or the pharmaceutically acceptable salt thereof according to any one of the foregoing, Ab is an ActRIIA/B antibody; preferably, the amino acid sequence of the heavy chain of Ab is as set forth in SEQ ID NO: 33, and the amino acid sequence of the light chain is as set forth in SEQ ID NO: 34.

In some embodiments, in the antibody-polypeptide conjugate or the pharmaceutically acceptable salt thereof according to any one of the foregoing, L is -L^{a}-L^{b}-L^{c}-L^{d}-L^{e}-,
L^{a} is selected from: 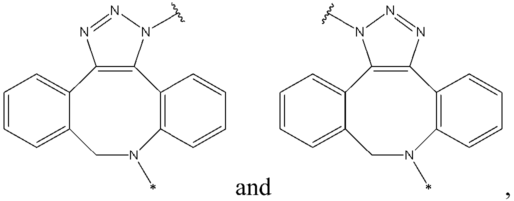 wherein the asterisk * indicates binding to L^{b}, and the wavy line 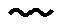 indicates binding to the remodeled glycan chain of Ab;
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino;
alternatively, R^{a} and R^{b}, together with the carbon atom to which they are attached, form cycloalkyl or heterocyclyl, R^{c} and R^{d}, together with the carbon atom to which they are attached, form cycloalkyl or heterocyclyl, R^{f} and R^{g}, together with the carbon atom to which they are attached, form cycloalkyl or heterocyclyl, Rⁱ and R^{j}, together with the carbon atom to which they are attached, form cycloalkyl or heterocyclyl, and R^{m} and Rⁿ, together with the carbon atom to which they are attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino;
alternatively, R^{a} and R^{c}, together with the carbon atoms to which they are respectively attached, form cycloalkyl or heterocyclyl, and Rⁱ and R^{m}, together with the carbon atoms to which they are respectively attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino.

In some embodiments, in the antibody-polypeptide conjugate or the pharmaceutically acceptable salt thereof according to any one of the foregoing, L is -L^{a}-L^{b}-L^{c}-L^{d}-L^{e}-,
L^{a} is selected from: 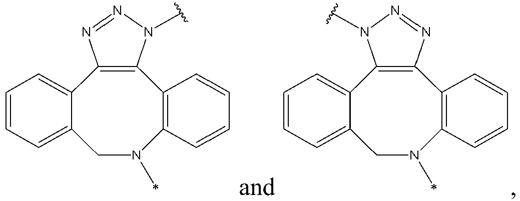 wherein the asterisk * indicates binding to L^{b}, and the wavy line 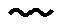 indicates binding to the remodeled glycan chain of Ab;
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, C₁₋₆ alkyl, C₁₋₆ haloalkyl, C₁₋₆ alkoxy, C₁₋₆ hydroxyalkyl, hydroxyl, cyano, amino, 3- to 6-membered cycloalkyl, 3- to 6-membered heterocyclyl, 6- to 10-membered aryl and 5- to 10-membered heteroaryl, the 3- to 6-membered cycloalkyl, 3- to 6-membered heterocyclyl, 6- to 10-membered aryl and 5- to 10-membered heteroaryl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{b}, together with the carbon atom to which they are attached, form 3- to 6-membered cycloalkyl or 3- to 6-membered heterocyclyl, R^{c} and R^{d}, together with the carbon atom to which they are attached, form 3- to 6-membered cycloalkyl or 3- to 6-membered heterocyclyl, R^{f} and R^{g}, together with the carbon atom to which they are attached, form 3- to 6-membered cycloalkyl or 3-to 6-membered heterocyclyl, Rⁱ and R^{j}, together with the carbon atom to which they are attached, form 3- to 6-membered cycloalkyl or 3- to 6-membered heterocyclyl, and R^{m} and Rⁿ, together with the carbon atom to which they are attached, form 3- to 6-membered cycloalkyl or 3- to 6-membered heterocyclyl, the 3- to 6-membered cycloalkyl or 3- to 6-membered heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{c}, together with the carbon atoms to which they are respectively attached, form 3- to 6-membered cycloalkyl or 3- to 6-membered heterocyclyl, and Rⁱ and R^{m}, together with the carbon atoms to which they are respectively attached, form 3- to 6-membered cycloalkyl or 3- to 6-membered heterocyclyl, the 3- to 6-membered cycloalkyl or 3- to 6-membered heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino.

In some embodiments, in the antibody-polypeptide conjugate or the pharmaceutically acceptable salt thereof according to any one of the foregoing, L^{b} is -C(O)-CR^{a}R^{b}-CR^{c}R^{d}-C(O)-, and R^{a}, R^{b}, R^{c} and R^{d} are identical or different, and are each independently a hydrogen atom or C₁₋₆ alkyl; preferably, L^{b} is -C(O)-CH₂-CH₂-C(O)-.

In some embodiments, in the antibody-polypeptide conjugate or the pharmaceutically acceptable salt thereof according to any one of the foregoing, L^{c} is -NR^{h}-CRⁱR^{j}-CR^{m}Rⁿ-, and R^{h}, Rⁱ, R^{j}, R^{m} and Rⁿ are identical or different, and are each independently a hydrogen atom or C₁₋₆ alkyl; preferably, L° is -NH-CH₂-CH₂-

In one embodiment, in the antibody-polypeptide conjugate or the pharmaceutically acceptable salt thereof according to any one of the foregoing, the PEG unit comprises between 1 and 36 EG units, or between 4 and 24 EG units. In one embodiment, a PEG segment comprises 2 EG units, or 4 EG units, or 6 EG units, or 8 EG units, or 10 EG units, or 12 EG units, or 14 EG units, or 16 EG units, or 18 EG units, or 20 EG units, or 22 EG units, or 24 EG units.

In some embodiments, in the antibody-polypeptide conjugate or the pharmaceutically acceptable salt thereof according to any one of the foregoing, L^{d} is 1 to 36 -O-CH₂-CH₂- units; preferably, L^{d} is 4 to 24 -O-CH₂-CH₂- units; more preferably, L^{d} is 4, 8 , 12 or 24 -O-CH₂-CH₂- units; most preferably, L^{d} is 8 -O-CH₂-CH₂- units.

In some embodiments, in the antibody-polypeptide conjugate or the pharmaceutically acceptable salt thereof according to any one of the foregoing, L is -L^{a}-L^{b}-L^{c}-L^{d}-L^{e}-,
L^{a} is selected from: 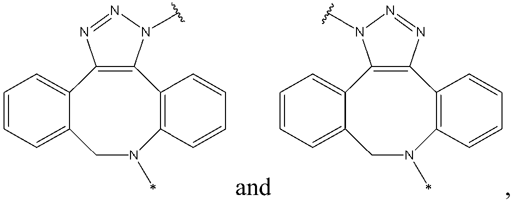 wherein the asterisk * indicates binding to L^{b}, and the wavy line 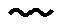 indicates binding to the remodeled glycan chain of Ab;
L^{b} is selected from -C(O)-CR^{a}R^{b}-CR^{c}R^{d}-C(O)-;
L° is -NR^{h}-CRⁱR^{j}-CR^{m}Rⁿ-;
L^{d} is 4 to 24 -O-CH₂-CH₂- units;
L^{e} is -C(O)-;
R^{a}, R^{b}, R^{c}, R^{d}, R^{h}, Rⁱ, R^{j}, R^{m} and Rⁿ are identical or different, and are each independently a hydrogen atom or C₁₋₆ alkyl.

In some embodiments, in the antibody-polypeptide conjugate or the pharmaceutically acceptable salt thereof according to any one of the foregoing, L is 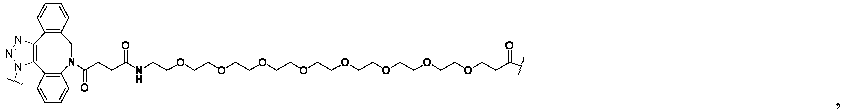 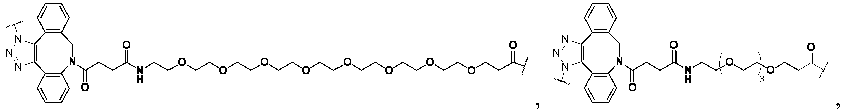 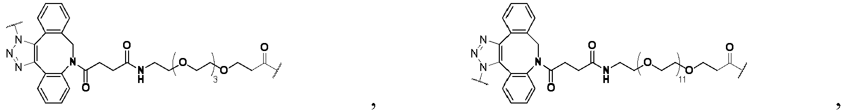 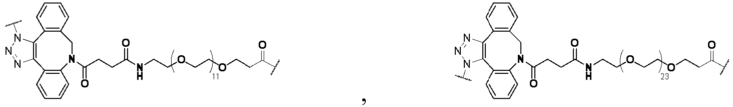 or

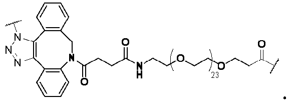

In some embodiments, in the antibody-polypeptide conjugate or the pharmaceutically acceptable salt thereof according to any one of the foregoing, L is 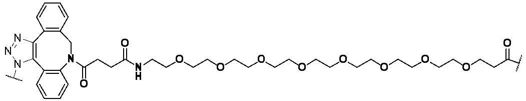 or

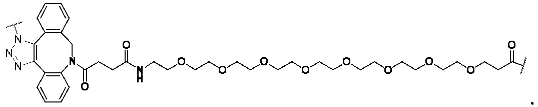

In some embodiments, the antibody-polypeptide conjugate or the pharmaceutically acceptable salt thereof according to any one of the foregoing has the structure as represented by general formula (II): 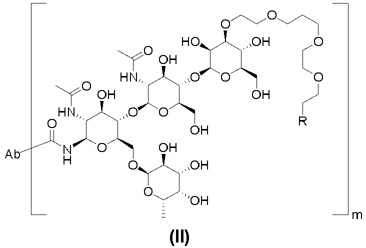 wherein 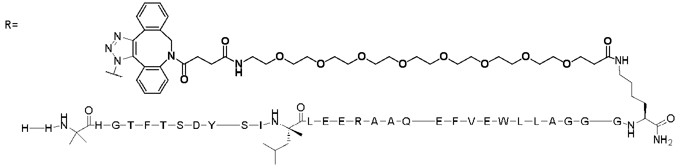 or 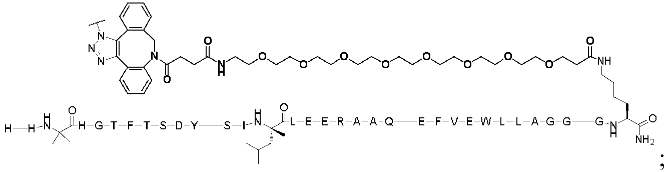 wherein Ab and m are as defined in general formula (I).

In some embodiments, the antibody-polypeptide conjugate or the pharmaceutically acceptable salt thereof according to any one of the foregoing has the structure as represented by general formula (II): 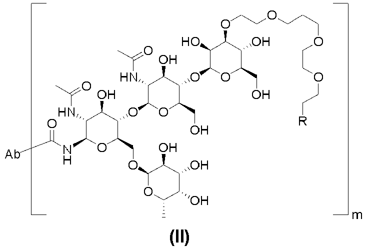 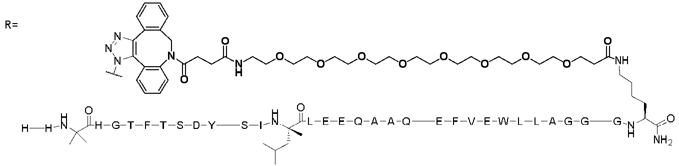 or 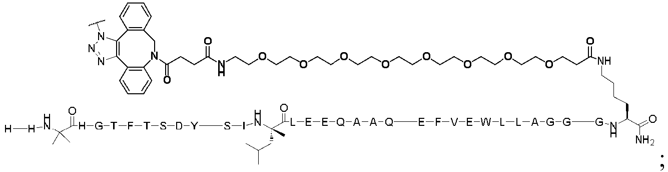 wherein Ab and m are as defined in general formula (I).

In some embodiments, the antibody-polypeptide conjugate or the pharmaceutically acceptable salt thereof according to any one of the foregoing has the structure as represented by general formula (II): 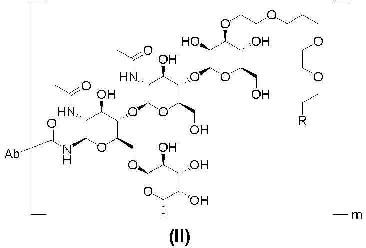 wherein 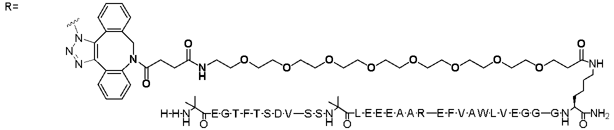 or 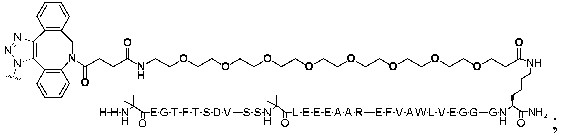 wherein Ab and m are as defined in general formula (I).

In some embodiments, the antibody-polypeptide conjugate or the pharmaceutically acceptable salt thereof according to any one of the foregoing has the structure as represented by general formula (II): 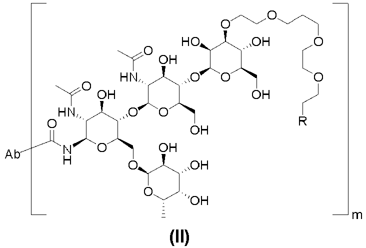 wherein 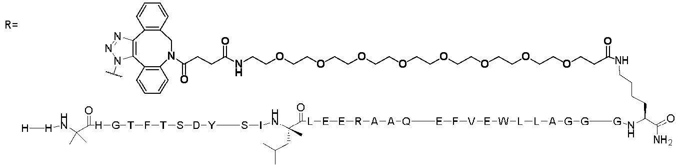 or 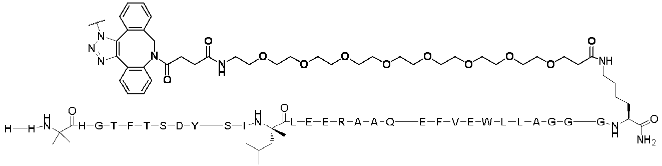
the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11;
preferably, the heavy chain variable region of Ab comprises the amino acid sequence of SEQ ID NO: 12, and the light chain variable region comprises the amino acid sequence of SEQ ID NO: 13;
more preferably, the amino acid sequence of the heavy chain of Ab is as set forth in SEQ ID NO: 16, and the amino acid sequence of the light chain is as set forth in SEQ ID NO: 17;
m is an integer from 1 to 4; preferably, m is 1 or 2; more preferably, m is 2.

In some embodiments, the antibody-polypeptide conjugate or the pharmaceutically acceptable salt thereof according to any one of the foregoing has the structure as represented by general formula (II): 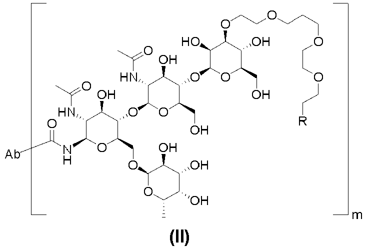 wherein 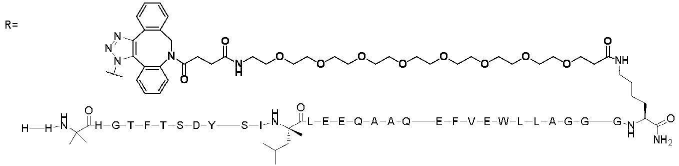 or 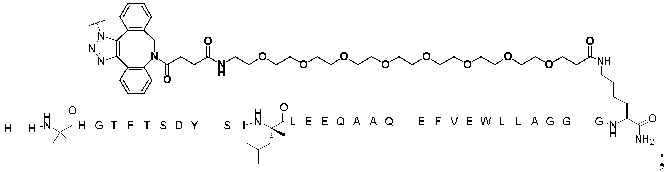
the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11;
preferably, the heavy chain variable region of Ab comprises the amino acid sequence of SEQ ID NO: 12, and the light chain variable region comprises the amino acid sequence of SEQ ID NO: 13;
more preferably, the amino acid sequence of the heavy chain of Ab is as set forth in SEQ ID NO: 16 or 19, and the amino acid sequence of the light chain is as set forth in SEQ ID NO: 17;
m is an integer from 1 to 4; preferably, m is 1 or 2; more preferably, m is 2.

In some embodiments, the antibody-polypeptide conjugate or the pharmaceutically acceptable salt thereof according to any one of the foregoing has the structure as represented by general formula (II): 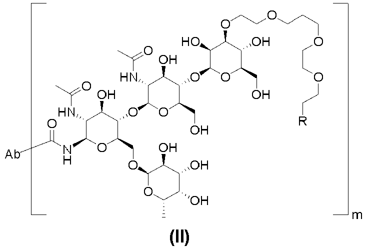 wherein 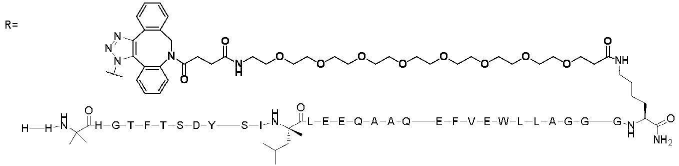 or 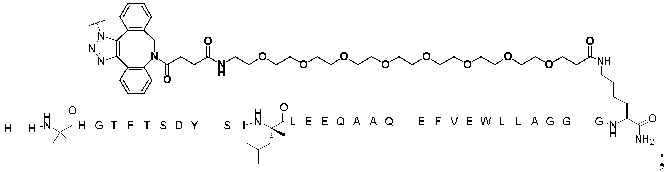
the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
preferably, the heavy chain variable region of Ab comprises the amino acid sequence of SEQ ID NO: 29, and the light chain variable region comprises the amino acid sequence of SEQ ID NO: 30;
more preferably, the heavy chain of Ab comprises the amino acid sequence of SEQ ID NO: 33, and the light chain comprises the amino acid sequence of SEQ ID NO: 34;
m is an integer from 1 to 4; preferably, m is 1 or 2; more preferably, m is 2.

In some embodiments, the antibody-polypeptide conjugate or the pharmaceutically acceptable salt thereof according to any one of the foregoing has the structure as represented by general formula (II): 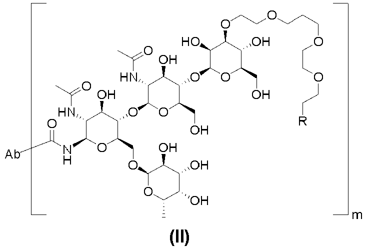 wherein 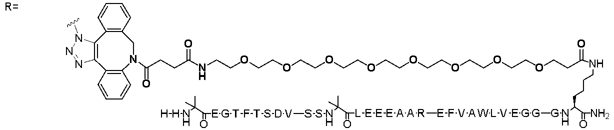 or 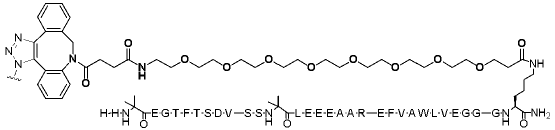
the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11;
preferably, the heavy chain variable region of Ab comprises the amino acid sequence of SEQ ID NO: 12, and the light chain variable region comprises the amino acid sequence of SEQ ID NO: 13;
more preferably, the amino acid sequence of the heavy chain of Ab is as set forth in SEQ ID NO: 16, and the amino acid sequence of the light chain is as set forth in SEQ ID NO: 17;
m is an integer from 1 to 4; preferably, m is 1 or 2; more preferably, m is 2.

In some embodiments, the antibody-polypeptide conjugate or the pharmaceutically acceptable salt thereof according to any one of the foregoing has the structure as represented by general formula (II): 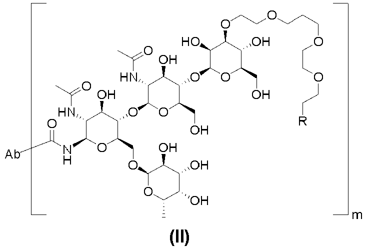 wherein 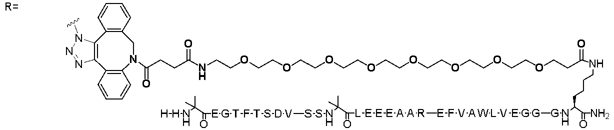 or 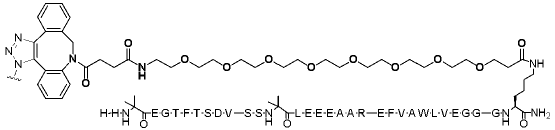
the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
preferably, the heavy chain variable region of Ab comprises the amino acid sequence of SEQ ID NO: 29, and the light chain variable region comprises the amino acid sequence of SEQ ID NO: 30;
more preferably, the heavy chain of Ab comprises the amino acid sequence of SEQ ID NO: 33, and the light chain comprises the amino acid sequence of SEQ ID NO: 34;
m is an integer from 1 to 4; preferably, m is 1 or 2; more preferably, m is 2.

In another aspect, the present disclosure provides a compound as represented by general formula (IIa) or a salt thereof: 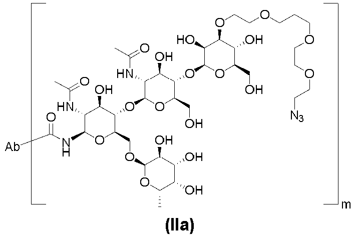 wherein Ab and m are as defined in general formula (I) or general formula (II).

In some embodiments, in the compound as represented by general formula (IIa) or the salt thereof as described above, Ab is a KLB antibody or an ActRIIA/B antibody;
preferably, (1) the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11; or
(2) the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
more preferably, (1) the heavy chain variable region of Ab comprises the amino acid sequence of SEQ ID NO: 12, and the light chain variable region comprises the amino acid sequence of SEQ ID NO: 13; or
(2) the heavy chain variable region of Ab comprises the amino acid sequence of SEQ ID NO: 29, and the light chain variable region comprises the amino acid sequence of SEQ ID NO: 30;
most preferably, (1) the heavy chain of Ab comprises the amino acid sequence of SEQ ID NO: 16 or 19, and the light chain comprises the amino acid sequence of SEQ ID NO: 17; or
(2) the heavy chain of Ab comprises the amino acid sequence of SEQ ID NO: 33, and the light chain comprises the amino acid sequence of SEQ ID NO: 34;
m is as defined in general formula (I) or general formula (II).

In another aspect, the present disclosure provides a glucagon-like peptide-1 receptor agonist peptide or an analogue thereof.

In some embodiments, the glucagon-like peptide-1 receptor agonist peptide or the analogue thereof as described above is a GLP-1R monoagonist peptide or a GLP-1R/GIPR/GCGR triagonist peptide or an analogue thereof.

In some embodiments, the glucagon-like peptide-1 receptor agonist peptide or the analogue thereof according to any one of the foregoing is a GLP-1R monoagonist peptide.

In some embodiments, the glucagon-like peptide-1 receptor agonist peptide or the analogue thereof according to any one of the foregoing is a GLP-1R/GIPR dual agonist peptide or an analogue thereof.

In some embodiments, the glucagon-like peptide-1 receptor agonist peptide or the analogue thereof according to any one of the foregoing is a GLP-1R/GCGR dual agonist peptide or an analogue thereof.

In some embodiments, the glucagon-like peptide-1 receptor agonist peptide or the analogue thereof according to any one of the foregoing is a GLP-1R/GIPR/GCGR triagonist peptide or an analogue thereof.

In some embodiments, the glucagon-like peptide-1 receptor agonist peptide or the analogue thereof according to any one of the foregoing comprises the amino acid sequence of SEQ ID NO: 20, SEQ ID NO: 21 or SEQ ID NO: 22; preferably, it comprises the amino acid sequence of SEQ ID NO: 21.

In some embodiments, the glucagon-like peptide-1 receptor agonist peptide or the analogue thereof according to any one of the foregoing comprises the amino acid sequence as set forth in SEQ ID NO: 20, SEQ ID NO: 21 or SEQ ID NO: 22; preferably, the amino acid sequence is as set forth in SEQ ID NO: 21.

In another aspect, the present disclosure provides a compound as represented by general formula (Ib) or a salt thereof:

L'-P (Ib)

wherein
P is a glucagon-like peptide-1 receptor agonist peptide or an analogue thereof; preferably, P is a GLP-1R monoagonist peptide or a GLP-1R/GIPR/GCGR triagonist peptide or an analogue thereof; more preferably, P comprises the amino acid sequence of SEQ ID NO: 20, SEQ ID NO: 21 or SEQ ID NO: 22;
L' is L^{aa}-L^{b}-L^{c}-L^{d}-L^{e}-,
L^{aa} is selected from: 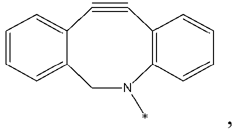 wherein the asterisk * indicates binding to L^{b};
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino;
alternatively, R^{a} and R^{b}, together with the carbon atom to which they are attached, form cycloalkyl or heterocyclyl, R^{c} and R^{d}, together with the carbon atom to which they are attached, form cycloalkyl or heterocyclyl, R^{f} and R^{g}, together with the carbon atom to which they are attached, form cycloalkyl or heterocyclyl, Rⁱ and R^{j}, together with the carbon atom to which they are attached, form cycloalkyl or heterocyclyl, and R^{m} and Rⁿ, together with the carbon atom to which they are attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino;
alternatively, R^{a} and R^{c}, together with the carbon atoms to which they are respectively attached, form cycloalkyl or heterocyclyl, and Rⁱ and R^{m}, together with the carbon atoms to which they are respectively attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino.

In another aspect, the present disclosure provides a compound as represented by general formula (Ib) or a salt thereof:

L'-P (Ib)

wherein
P is a glucagon-like peptide-1 receptor agonist peptide or an analogue thereof; preferably, P is a GLP-1R/GIPR/GCGR triagonist peptide or an analogue thereof; more preferably, the amino acid sequence of P is as set forth in SEQ ID NO: 20 or SEQ ID NO: 21;
L' is L^{aa}-L^{b}-L^{c}-L^{d}-L^{e}-,
L^{aa} is selected from: 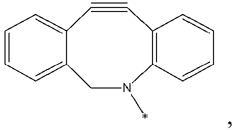 wherein the asterisk * indicates binding to L^{b};
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino;
alternatively, R^{a} and R^{b}, together with the carbon atom to which they are attached, form cycloalkyl or heterocyclyl, R^{c} and R^{d}, together with the carbon atom to which they are attached, form cycloalkyl or heterocyclyl, R^{f} and R^{g}, together with the carbon atom to which they are attached, form cycloalkyl or heterocyclyl, Rⁱ and R^{j}, together with the carbon atom to which they are attached, form cycloalkyl or heterocyclyl, and R^{m} and Rⁿ, together with the carbon atom to which they are attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino;
alternatively, R^{a} and R^{c}, together with the carbon atoms to which they are respectively attached, form cycloalkyl or heterocyclyl, and Rⁱ and R^{m}, together with the carbon atoms to which they are respectively attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino.

In some embodiments, in the compound as represented by general formula (Ib) or the salt thereof as described above, P is a glucagon-like peptide-1 receptor agonist peptide or an analogue thereof; preferably, P is a GLP-1R monoagonist peptide or a GLP-1R/GIPR/GCGR triagonist peptide or an analogue thereof; more preferably, P comprises the amino acid sequence of SEQ ID NO: 20, SEQ ID NO: 21 or SEQ ID NO: 22;
L' is L^{aa}-L^{b}-L^{c}-L^{d}-L^{e}-,
L^{aa} is selected from: 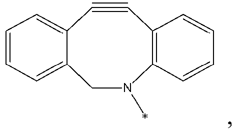 wherein the asterisk * indicates binding to L^{b};
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, C₁₋₆ alkyl, C₁₋₆ haloalkyl, C₁₋₆ alkoxy, C₁₋₆ hydroxyalkyl, hydroxyl, cyano, amino, 3- to 6-membered cycloalkyl, 3- to 6-membered heterocyclyl, 6- to 10-membered aryl and 5- to 10-membered heteroaryl, the 3- to 6-membered cycloalkyl, 3- to 6-membered heterocyclyl, 6- to 10-membered aryl and 5- to 10-membered heteroaryl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{b}, together with the carbon atom to which they are attached, form 3- to 6-membered cycloalkyl or 3- to 6-membered heterocyclyl, R^{c} and R^{d}, together with the carbon atom to which they are attached, form 3- to 6-membered cycloalkyl or 3- to 6-membered heterocyclyl, R^{f} and R^{g}, together with the carbon atom to which they are attached, form 3- to 6-membered cycloalkyl or 3-to 6-membered heterocyclyl, Rⁱ and R^{j}, together with the carbon atom to which they are attached, form 3- to 6-membered cycloalkyl or 3- to 6-membered heterocyclyl, and R^{m} and Rⁿ, together with the carbon atom to which they are attached, form 3- to 6-membered cycloalkyl or 3- to 6-membered heterocyclyl, the cycloalkyl or heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{c}, together with the carbon atoms to which they are respectively attached, form 3- to 6-membered cycloalkyl or 3- to 6-membered heterocyclyl, and Rⁱ and R^{m}, together with the carbon atoms to which they are respectively attached, form 3- to 6-membered cycloalkyl or 3- to 6-membered heterocyclyl, the 3- to 6-membered cycloalkyl or 3- to 6-membered heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino.

In some embodiments, in the compound as represented by general formula (Ib) or the salt thereof according to any one of the foregoing, P is a glucagon-like peptide-1 receptor agonist peptide or an analogue thereof; preferably, P is a GLP-1R/GIPR/GCGR triagonist peptide or an analogue thereof; more preferably, the amino acid sequence of P is as set forth in SEQ ID NO: 20 or SEQ ID NO: 21;
L' is L^{aa}-L^{b}-L^{c}-L^{d}-L^{e}-,
L^{aa} is selected from: 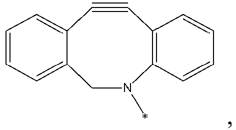 wherein the asterisk * indicates binding to L^{b};
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and -O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, C₁₋₆ alkyl, C₁₋₆ haloalkyl, C₁₋₆ alkoxy, C₁₋₆ hydroxyalkyl, hydroxyl, cyano, amino, 3- to 6-membered cycloalkyl, 3- to 6-membered heterocyclyl, 6- to 10-membered aryl and 5- to 10-membered heteroaryl, the 3- to 6-membered cycloalkyl, 3- to 6-membered heterocyclyl, 6- to 10-membered aryl and 5- to 10-membered heteroaryl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{b}, together with the carbon atom to which they are attached, form 3- to 6-membered cycloalkyl or 3- to 6-membered heterocyclyl, R^{c} and R^{d}, together with the carbon atom to which they are attached, form 3- to 6-membered cycloalkyl or 3- to 6-membered heterocyclyl, R^{f} and R^{g}, together with the carbon atom to which they are attached, form 3- to 6-membered cycloalkyl or 3-to 6-membered heterocyclyl, Rⁱ and R^{j}, together with the carbon atom to which they are attached, form 3- to 6-membered cycloalkyl or 3- to 6-membered heterocyclyl, and R^{m} and Rⁿ, together with the carbon atom to which they are attached, form 3- to 6-membered cycloalkyl or 3- to 6-membered heterocyclyl, the cycloalkyl or heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino;
alternatively, R^{a} and R^{c}, together with the carbon atoms to which they are respectively attached, form 3- to 6-membered cycloalkyl or 3- to 6-membered heterocyclyl, and Rⁱ and R^{m}, together with the carbon atoms to which they are respectively attached, form 3- to 6-membered cycloalkyl or 3- to 6-membered heterocyclyl, the 3- to 6-membered cycloalkyl or 3- to 6-membered heterocyclyl being optionally substituted with one or more substituents selected from the group consisting of oxo, halogen, C₁₋₆ alkyl, C₁₋₆ haloalkyl, C₁₋₆ alkoxy, C₁₋₆ hydroxyalkyl, hydroxyl, cyano and amino.

In some embodiments, in the compound as represented by general formula (Ib) or the salt thereof according to any one of the foregoing, L^{aa} is selected from: 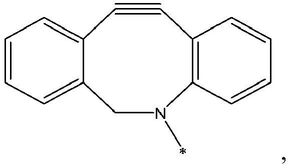 wherein the asterisk * indicates binding to L^{b}.

In some embodiments, in the compound as represented by general formula (Ib) or the salt thereof according to any one of the foregoing, L^{b} is -C(O)-CR^{a}R^{b}-CR^{c}R^{d}-C(O)-, and R^{a}, R^{b}, R^{c} and R^{d} are identical or different, and are each independently a hydrogen atom or C₁₋₆ alkyl; preferably, L^{b} is -C(O)-CH₂-CH₂-C(O)-.

In some embodiments, in the compound as represented by general formula (Ib) or the salt thereof according to any one of the foregoing, L^{c} is -NR^{h}-CRⁱR^{j}-CR^{m}Rⁿ-, and R^{h}, Rⁱ, R^{j}, R^{m} and Rⁿ are identical or different, and are each independently a hydrogen atom or C₁₋₆ alkyl; preferably, L° is -NH-CH₂-CH₂-.

In some embodiments, in the compound as represented by general formula (Ib) or the salt thereof according to any one of the foregoing, L^{d} is 1 to 36 - O-CH₂-CH₂- units; preferably, L^{d} is 4 to 24 -O-CH₂-CH₂- units; more preferably, L^{d} is 4, 8, 12 or 24 -O-CH₂-CH₂- units; most preferably, L^{d} is 8 -O-CH₂-CH₂- units.

In some embodiments, in the compound as represented by general formula (Ib) or the salt thereof according to any one of the foregoing, L^{e} is -C(O)-.

In some embodiments, in the compound as represented by general formula (Ib) or the salt thereof according to any one of the foregoing, P is a glucagon-like peptide-1 receptor agonist peptide or an analogue thereof; preferably, P is a GLP-1R monoagonist peptide or a GLP-1R/GIPR/GCGR triagonist peptide or an analogue thereof; more preferably, P comprises the amino acid sequence of SEQ ID NO: 20, SEQ ID NO: 21 or SEQ ID NO: 22;
L' is L^{aa}-L^{b}-L^{c}-L^{d}-L^{e}-,
L^{aa} is selected from: 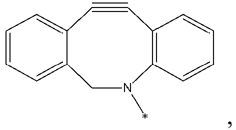 wherein the asterisk * indicates binding to L^{b};
L^{b} is selected from -C(O)-CR^{a}R^{b}-CR^{c}R^{d}-C(O)-;
L^{c} is -NR^{h}-CRⁱR^{j}-CR^{m}Rⁿ-;
L^{d} is 4 to 24 -O-CH₂-CH₂- units;
L^{e} is -C(O)-;
wherein R^{a}, R^{b}, R^{c}, R^{d}, R^{h}, Rⁱ, R^{j}, R^{m} and Rⁿ are identical or different, and are each independently a hydrogen atom or C₁₋₆ alkyl.

In some embodiments, in the compound as represented by general formula (Ib) or the salt thereof according to any one of the foregoing, P is a glucagon-like peptide-1 receptor agonist peptide or an analogue thereof; preferably, P is a GLP-1R/GIPR/GCGR triagonist peptide or an analogue thereof; more preferably, the amino acid sequence of P is as set forth in SEQ ID NO: 20 or SEQ ID NO: 21;
L' is L^{aa}-L^{b}-L^{c}-L^{d}-L^{e}-,
L^{aa} is selected from: 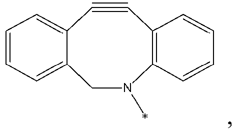 wherein the asterisk * indicates binding to L^{b};
L^{b} is selected from -C(O)-CR^{a}R^{b}-CR^{c}R^{d}-C(O)-;
L^{c} is -NR^{h}-CRⁱR^{j}-CR^{m}Rⁿ-;
L^{d} is 4 to 24 -O-CH₂-CH₂- units;
L^{e} is -C(O)-;
wherein R^{a}, R^{b}, R^{c}, R^{d}, R^{h}, Rⁱ, R^{j}, R^{m} and Rⁿ are identical or different, and are each independently a hydrogen atom or C₁₋₆ alkyl.

In some embodiments, the compound as represented by general formula (Ib) or the salt thereof according to any one of the foregoing has the structure as represented by general formula (IIb): 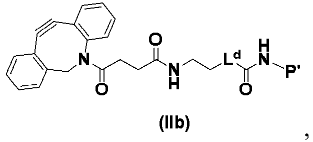 wherein P' is a moiety resulting from removal of the amino group at the P-terminus; preferably, P' is 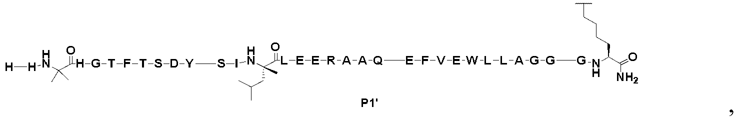 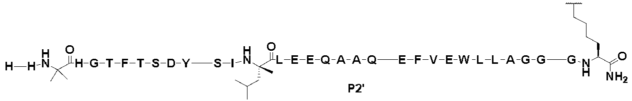 or 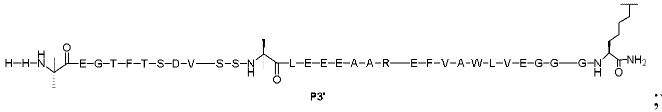 wherein L^{d} is as defined in general formula (Ib).

In some embodiments, the compound as represented by general formula (Ib) or the salt thereof according to any one of the foregoing is selected from the group consisting of the following structures: 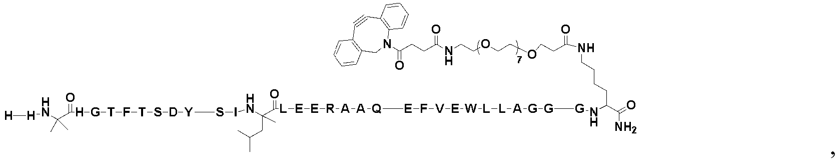 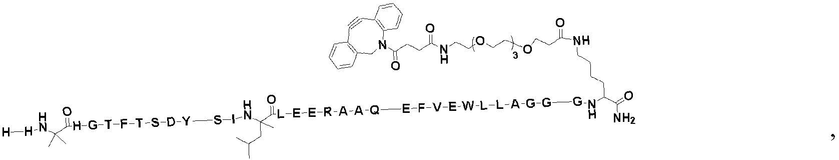 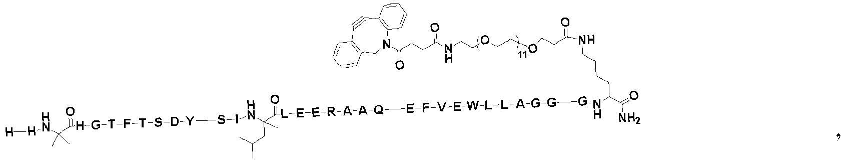 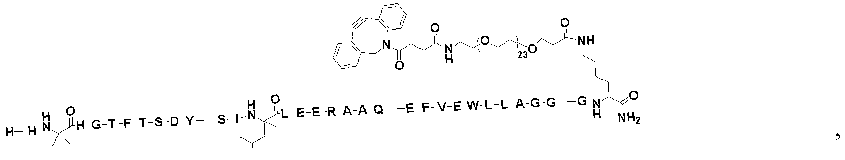 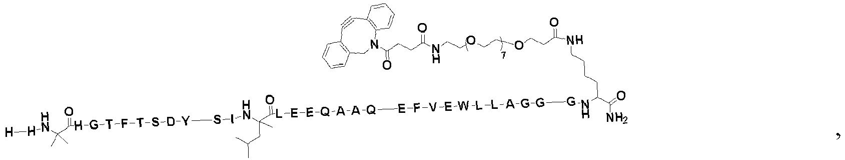 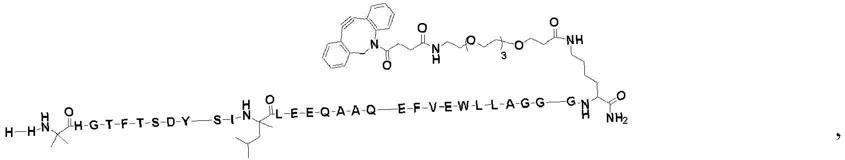 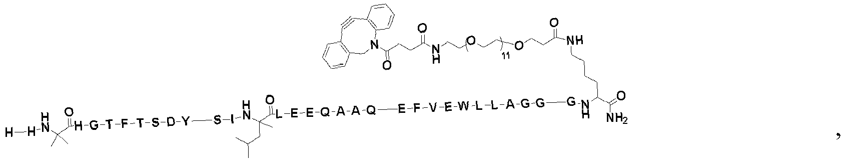 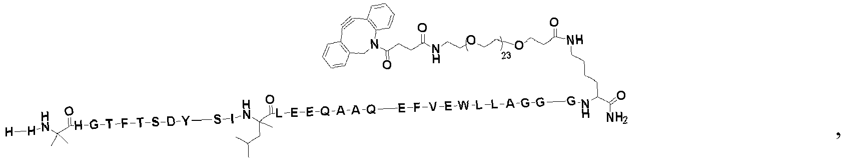 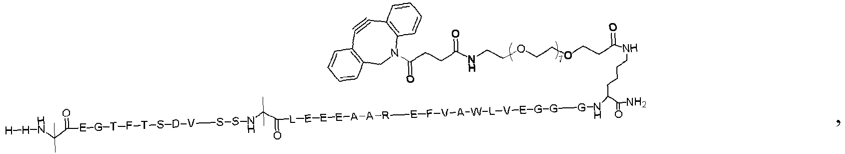 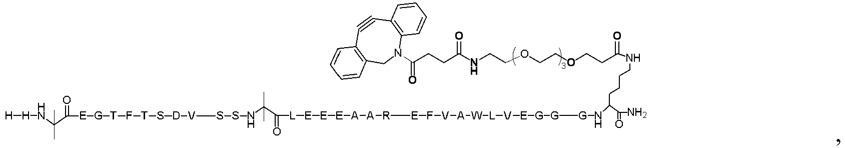 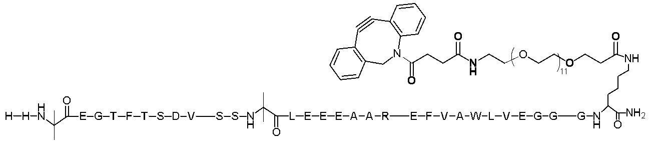 or

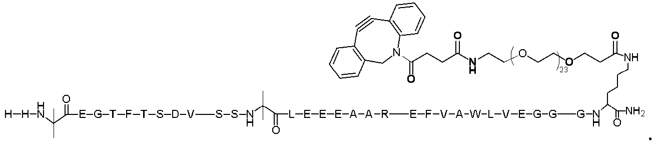

In some embodiments, the compound as represented by general formula (Ib) or the salt thereof according to any one of the foregoing is selected from the group consisting of the following structures: 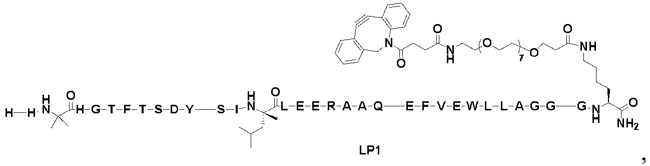 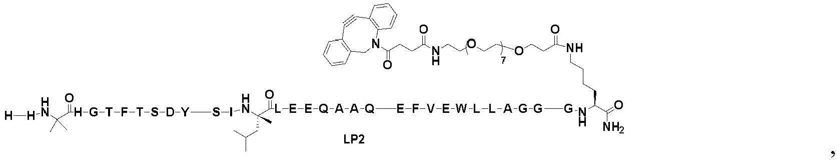 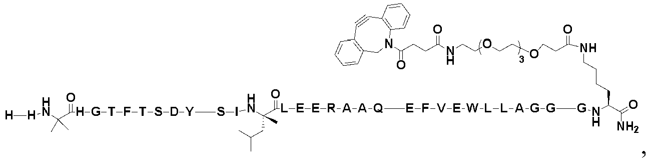 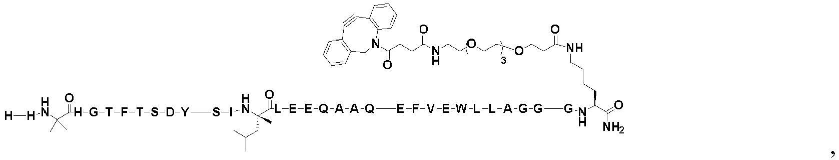 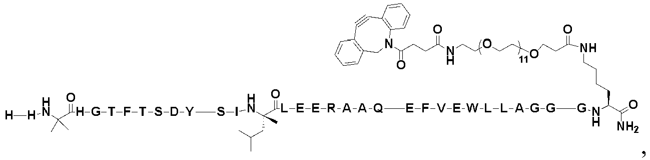 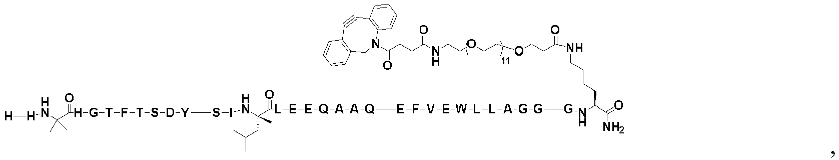 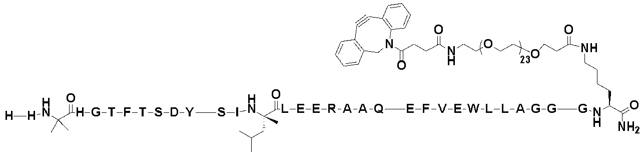 or

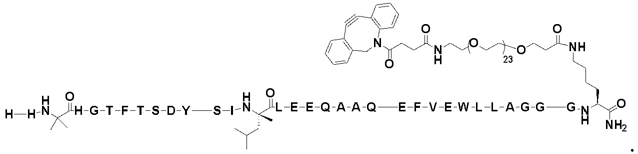

In some embodiments, the compound as represented by general formula (Ib) or the salt thereof according to any one of the foregoing is selected from the group consisting of the following structures: 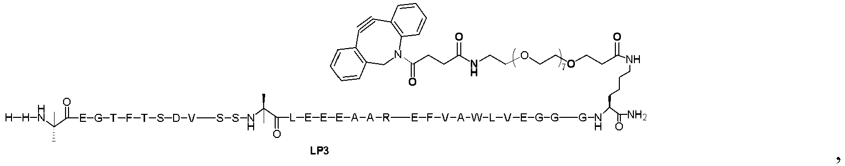 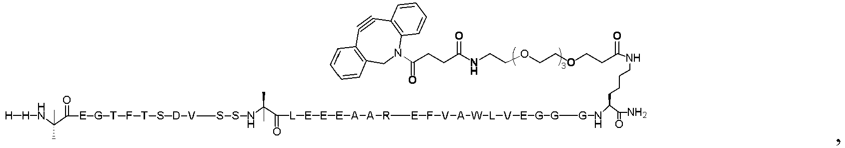 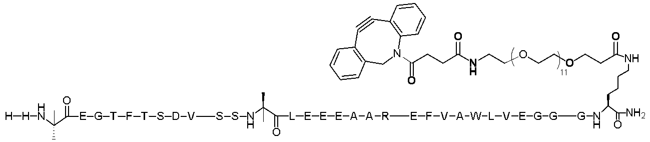 or

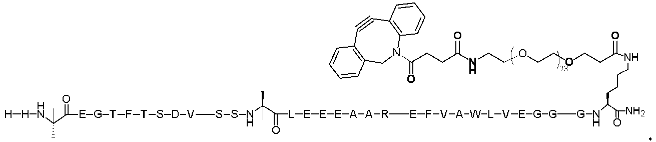

In some embodiments, the compound as represented by general formula (Ib) or the salt thereof according to any one of the foregoing is selected from the group consisting of the following structures: 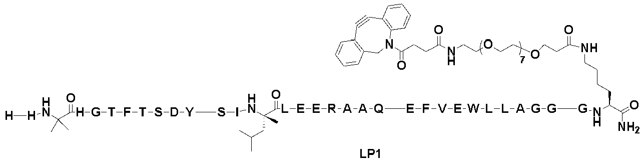 or

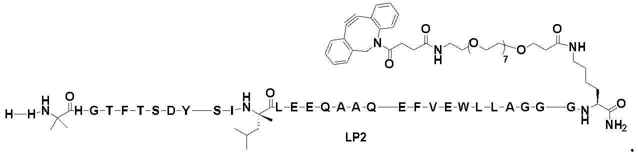

In some embodiments, the compound as represented by general formula (Ib) or the salt thereof according to any one of the foregoing is selected from the following structure:

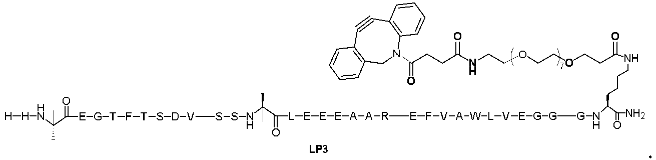

In another aspect, the present disclosure provides a method for preparing an antibody-polypeptide conjugate as represented by general formula (II) or a pharmaceutically acceptable salt thereof, comprising the following steps: 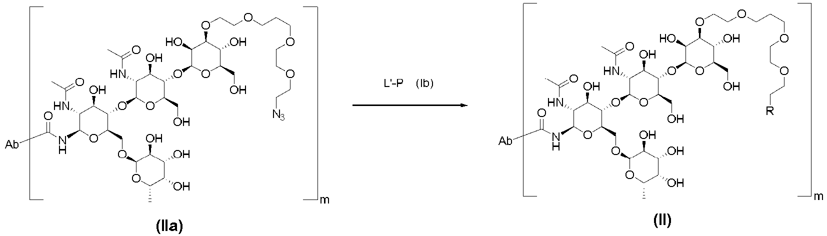
reacting a compound as represented by general formula (IIa) or a salt thereof with a compound as represented by general formula (Ib) or a salt thereof to obtain the antibody-polypeptide conjugate as represented by general formula (II) or the pharmaceutically acceptable salt thereof;
wherein R is -L-P;
Ab, L, P and m are as defined in general formula (I) or general formula (II), and L' is as defined in general formula (Ib).

In another aspect, the present disclosure provides a compound as represented by general formula (IIc) or a salt thereof: 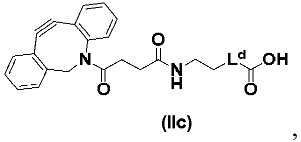 wherein L^{d} is as defined in general formula (Ib) or general formula (IIb).

In some embodiments, the compound as represented by general formula (IIc) or the salt thereof as described above is selected from the group consisting of the following structures: 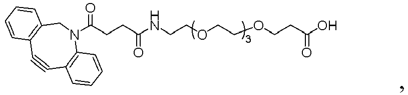 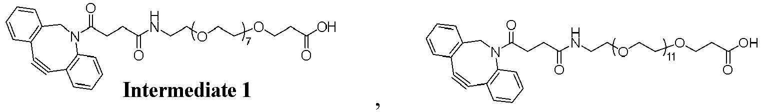 and

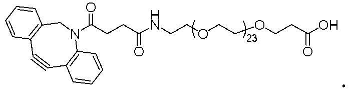

In some embodiments, the compound as represented by general formula (IIc) or the salt thereof as described above is selected from the following structure:

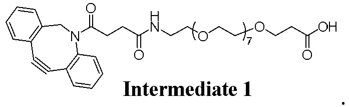

In another aspect, the present disclosure provides a method for preparing a compound as represented by general formula (IIb) or a salt thereof, comprising the following steps: 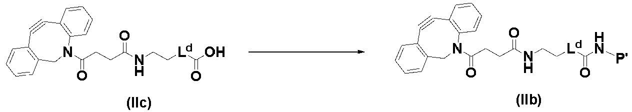
reacting a compound as represented by general formula (IIc) or a salt thereof with a carboxyl protecting reagent (such as N-hydroxysuccinimide (HOSU)) and then with P to obtain the compound as represented by general formula (IIb) or the salt thereof;
P is a glucagon-like peptide-1 receptor agonist peptide or an analogue thereof; preferably, P is a GLP-1R monoagonist peptide or a GLP-1R/GIPR/GCGR triagonist peptide or an analogue thereof; more preferably, P comprises the amino acid sequence of SEQ ID NO: 20, SEQ ID NO: 21 or SEQ ID NO: 22;
P' is a moiety resulting from removal of the amino group at the P-terminus;
wherein L^{d} is as defined in general formula (Ib) or general formula (IIb).

In another aspect, the present disclosure provides a method for preparing a compound as represented by general formula (IIb) or a salt thereof, comprising the following steps: 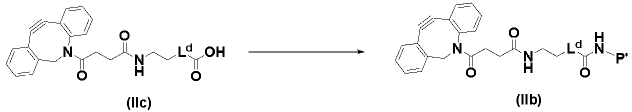
reacting a compound as represented by general formula (IIc) or a salt thereof with a carboxyl protecting reagent (such as N-hydroxysuccinimide (HOSU)) and then with P to obtain the compound as represented by general formula (IIb) or the salt thereof;
P is a glucagon-like peptide-1 receptor agonist peptide or an analogue thereof; preferably, P is a GLP-1R/GIPR/GCGR triagonist peptide or an analogue thereof; more preferably, the amino acid sequence of P is as set forth in SEQ ID NO: 20 or SEQ ID NO: 21;
P' is a moiety resulting from removal of the amino group at the P-terminus;
wherein L^{d} is as defined in general formula (Ib) or general formula (IIb).

In some embodiments, in the antibody-polypeptide conjugate as represented by general formula (I) or general formula (II) or the pharmaceutically acceptable salt thereof according to any one of the foregoing, m refers to the average number of polypeptides loaded per antibody-polypeptide conjugate molecule in the population of antibody-polypeptide conjugates, which can also be expressed as the ratio of polypeptide to antibody.

In some embodiments, in the antibody-polypeptide conjugate as represented by general formula (I) or general formula (II) or the pharmaceutically acceptable salt thereof according to any one of the foregoing, m is 1, 2, 3, 4, 5, 6, 7, 8, 9, 10, 11, 12, 13, 14, 15, 16, 17, 18, 19, 20, or a mean value of any two values therebetween; preferably, m is 1 to 10; further preferably, m is 1 to 4; more preferably, m is 1 or 2; most preferably, m is 2.

In some embodiments, in the antibody-polypeptide conjugate as represented by general formula (I) or general formula (II) or the pharmaceutically acceptable salt thereof according to any one of the foregoing, m represents the average ratio of antibody to polypeptide, for example, an integer from 1 to 10 is taken for m; preferably, an integer from 1 to 4 is taken for m, i.e., m is an integer from 1 to 4; further preferably, m is 1 or 2; more preferably, m is 2.

In some embodiments, in the compound as represented by general formula (IIa) or the salt thereof according to any one of the foregoing, m represents the average ratio of antibody to polypeptide, for example, an integer from 1 to 10 is taken for m; preferably, an integer from 1 to 4 is taken for m; further preferably, m is 1 or 2; more preferably, m is 2.

In another aspect, the present disclosure provides a pharmaceutical composition comprising the antibody-polypeptide conjugate as represented by general formula (I) or general formula (II) or the pharmaceutically acceptable salt thereof according to any one of the foregoing and one or more pharmaceutically acceptable carriers, diluents or excipients.

In another aspect, the present disclosure provides a pharmaceutical composition comprising the glucagon-like peptide-1 receptor agonist peptide or the analogue thereof according to any one of the foregoing and one or more pharmaceutically acceptable carriers, diluents or excipients.

In some embodiments, the pharmaceutical composition comprises 0.01%-99.99% of the antibody-polypeptide conjugate as represented by general formula (I) or general formula (II) or the pharmaceutically acceptable salt thereof according to any one of the foregoing or the glucagon-like peptide-1 receptor agonist peptide or the analogue thereof according to any one of the foregoing on the basis of the total weight of the pharmaceutical composition. In some embodiments, the pharmaceutical composition comprises 0.1%-99.9% of the antibody-polypeptide conjugate as represented by general formula (I) or general formula (II) or the pharmaceutically acceptable salt thereof according to any one of the foregoing or the glucagon-like peptide-1 receptor agonist peptide or the analogue thereof according to any one of the foregoing. In some embodiments, the pharmaceutical composition comprises 0.5%-99.5% of the antibody-polypeptide conjugate as represented by general formula (I) or general formula (II) or the pharmaceutically acceptable salt thereof according to any one of the foregoing or the glucagon-like peptide-1 receptor agonist peptide or the analogue thereof according to any one of the foregoing. In some embodiments, the pharmaceutical composition comprises 1%-99% of the antibody-polypeptide conjugate as represented by general formula (I) or general formula (II) or the pharmaceutically acceptable salt thereof according to any one of the foregoing or the glucagon-like peptide-1 receptor agonist peptide or the analogue thereof according to any one of the foregoing. In some embodiments, the pharmaceutical composition comprises 2%-98% of the antibody-polypeptide conjugate as represented by general formula (I) or general formula (II) or the pharmaceutically acceptable salt thereof according to any one of the foregoing or the glucagon-like peptide-1 receptor agonist peptide or the analogue thereof according to any one of the foregoing on the basis of the total weight of the pharmaceutical composition.

In some embodiments, the pharmaceutical composition comprises 0.01%-99.99% of the pharmaceutically acceptable diluent or excipient on the basis of the total weight of the pharmaceutical composition. In some embodiments, the pharmaceutical composition comprises 0.1%-99.9% of the pharmaceutically acceptable diluent or excipient. In some embodiments, the pharmaceutical composition comprises 0.5%-99.5% of the pharmaceutically acceptable excipient. In some embodiments, the pharmaceutical composition comprises 1%-99% of the pharmaceutically acceptable diluent or excipient. In some embodiments, the pharmaceutical composition comprises 2%-98% of the pharmaceutically acceptable diluent or excipient.

In another aspect, the present disclosure provides a method for preventing or treating a disease, comprising administering to a subject the antibody-polypeptide conjugate as represented by general formula (I) or general formula (II) or the pharmaceutically acceptable salt thereof or the pharmaceutical composition thereof according to any one of the foregoing.

In another aspect, the present disclosure provides a method for preventing or treating a disease, comprising administering to a subject the glucagon-like peptide-1 receptor agonist peptide or the analogue thereof according to any one of the foregoing.

In another aspect, the present disclosure provides the use in the manufacture of a medicament for preventing or treating a disease, comprising administering to a subject the antibody-polypeptide conjugate as represented by general formula (I) or general formula (II) or the pharmaceutically acceptable salt thereof or the pharmaceutical composition thereof according to any one of the foregoing.

In another aspect, the present disclosure provides the use in the manufacture of a medicament for preventing or treating a disease, comprising administering to a subject the glucagon-like peptide-1 receptor agonist peptide or the analogue thereof according to any one of the foregoing.

In another aspect, the present disclosure provides the antibody-polypeptide conjugate as represented by general formula (I) or general formula (II) or the pharmaceutically acceptable salt thereof or the pharmaceutical composition thereof according to any one of the foregoing for use as a medicament. In some embodiments, the medicament is used for preventing or treating a disease.

In another aspect, the present disclosure provides the glucagon-like peptide-1 receptor agonist peptide or the analogue thereof according to any one of the foregoing for use as a medicament. In some embodiments, the medicament is used for preventing or treating a disease.

In some embodiments, the disease according to any one of the foregoing is a GLP-1R-mediated disease.

In some embodiments, the disease according to any one of the foregoing is a GCGR-mediated disease.

In some embodiments, the disease according to any one of the foregoing is a GIPR-mediated disease.

In some embodiments, the disease according to any one of the foregoing is diabetes, obesity, liver disease, coronary artery disease or kidney disease; preferably, the disease is type II diabetes, obesity, metabolic dysfunction-associated steatotic liver disease (MASLD) and metabolic dysfunction-associated steatohepatitis (MASH, such as non-alcoholic steatohepatitis).

### Brief Description of the Drawings

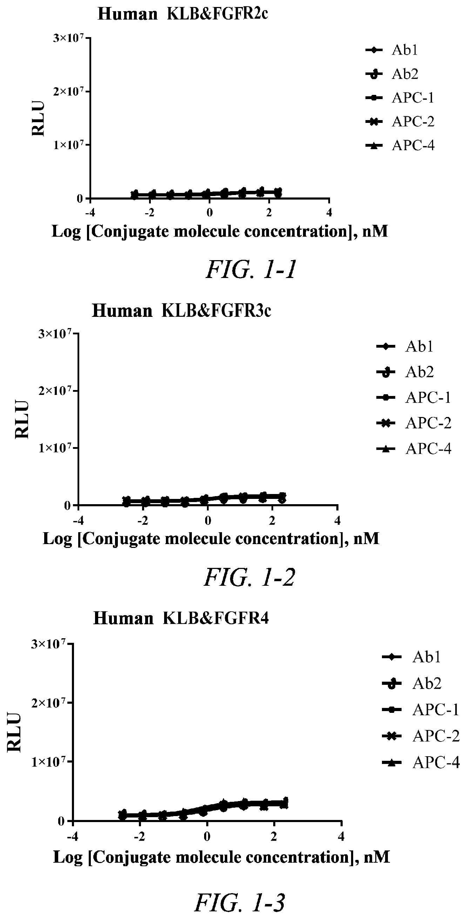

FIG. 1-1 shows a plot of KLB & FGFR2c signalling pathway activation by antibodies and conjugate molecules of the present application.
FIG. 1-2 shows a plot of KLB & FGFR3c signalling pathway activation by antibodies and conjugate molecules of the present application.
FIG. 1-3 shows a plot of KLB & FGFR4 signalling pathway activation by antibodies and conjugate molecules of the present application.
FIG. 2-1, FIG. 2-2, FIG. 2-3 and FIG. 2-4 show the effect of conjugate molecules of the present application on the glucose tolerance of wild type mice.
FIG. 3-1 shows the effect of conjugate molecules APC-2 and APC-4 of the present application on the body weight of DIO mice.
FIG. 3-2 shows the effect of conjugate molecules APC-2 and APC-4 of the present application on the food intake of DIO mice.
FIG. 3-3 shows the effect of conjugate molecules APC-2 and APC-4 of the present application on the random blood glucose of DIO mice.
FIG. 3-4 and FIG. 3-5 show the effect of conjugate molecules APC-2 and APC-4 of the present application on the glucose tolerance of DIO mice.

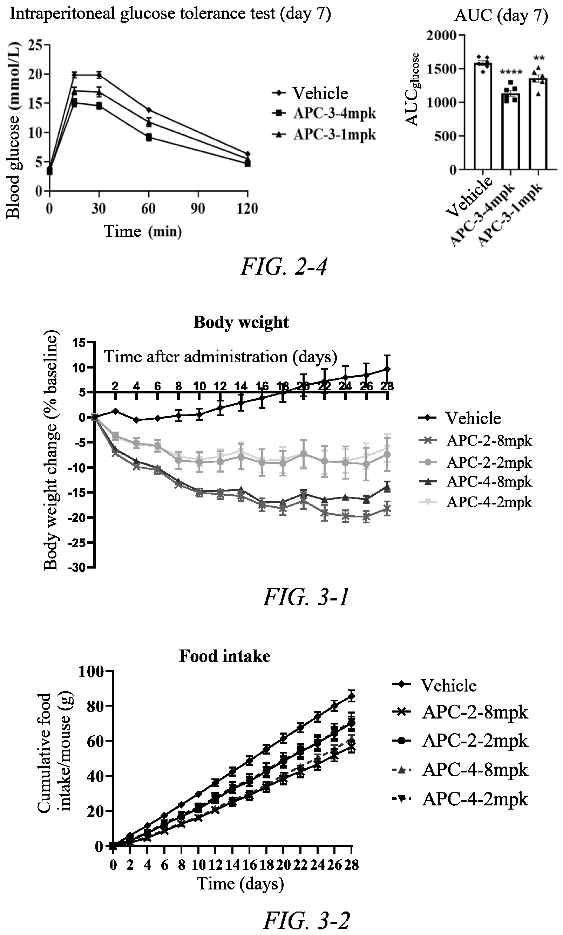

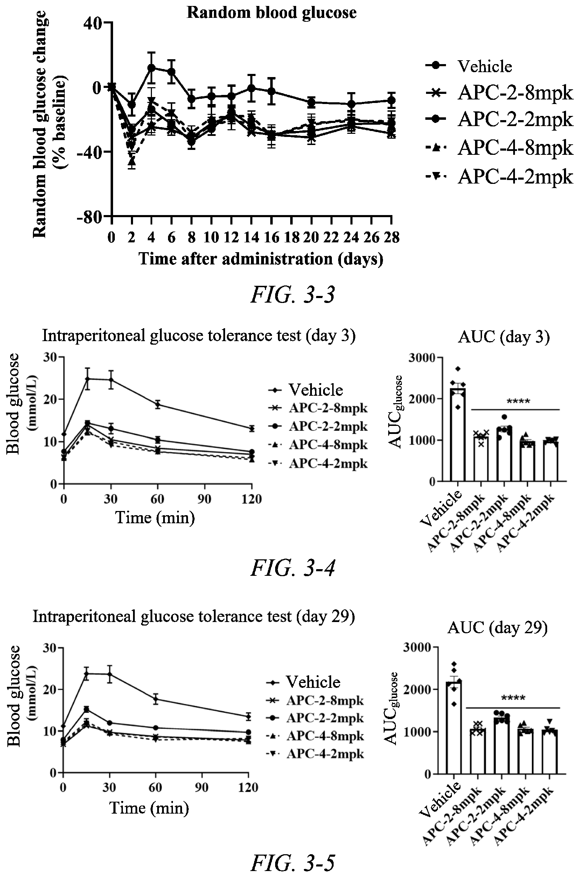

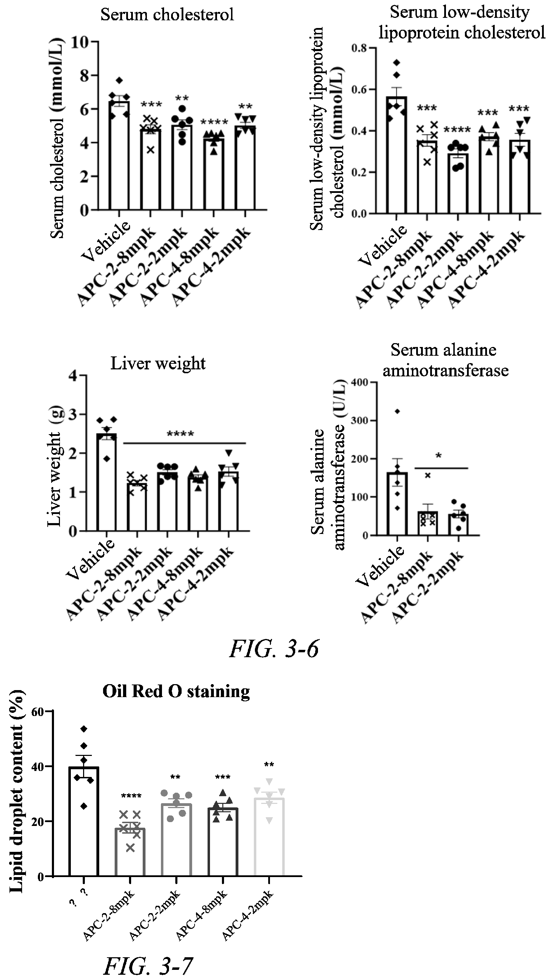

FIG. 3-6 shows the effect of conjugate molecules APC-2 and APC-4 of the present application on the serum cholesterol, serum low-density lipoprotein cholesterol, liver weight and serum alanine aminotransferase of DIO mice.
FIG. 3-7 shows the statistical results of Oil Red O staining of the effect of conjugate molecules APC-2 and APC-4 of the present application on the lipid droplets in liver tissues of DIO mice.

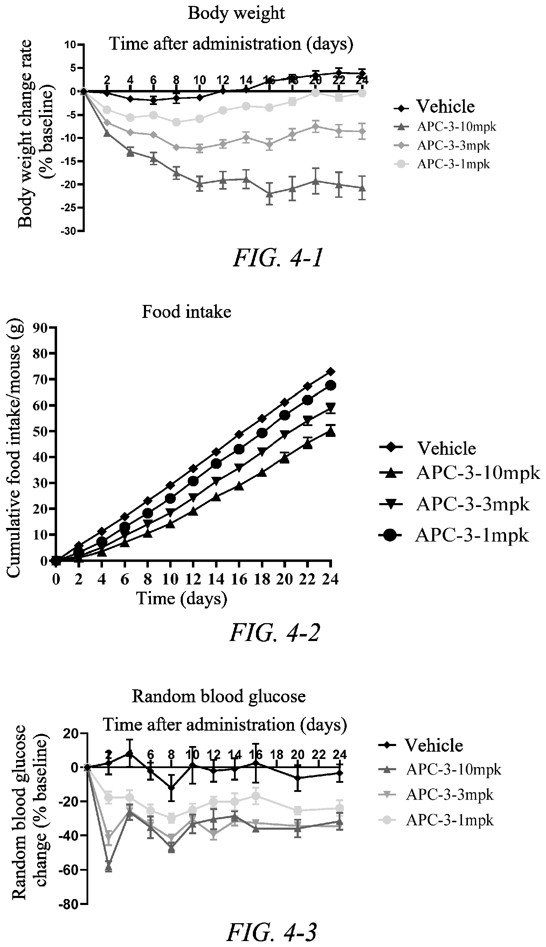

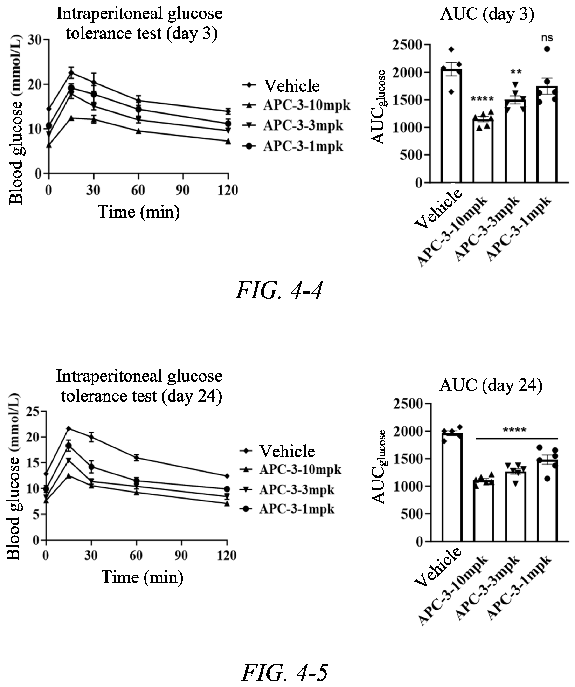

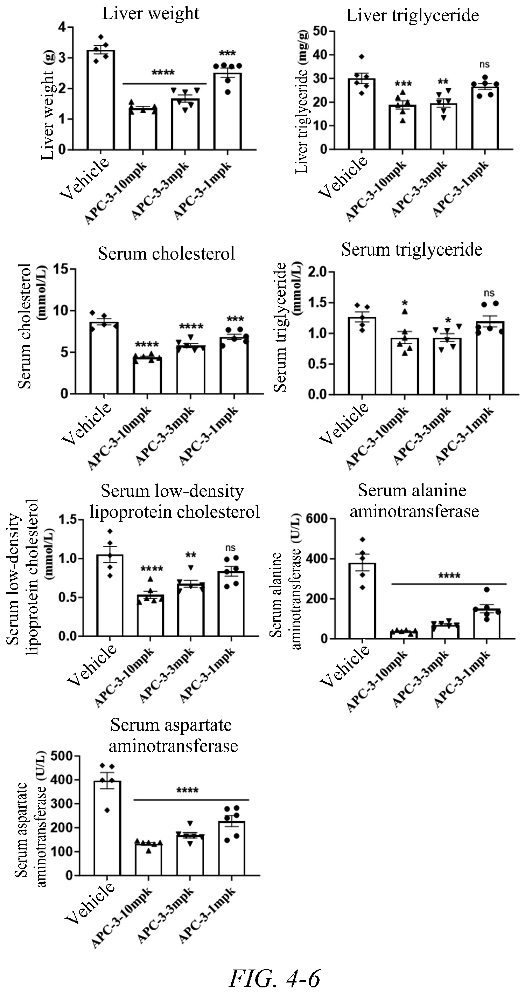

FIG. 4-1 shows the effect of conjugate molecule APC-3 of the present application on the body weight of DIO mice.
FIG. 4-2 shows the effect of conjugate molecule APC-3 of the present application on the food intake of DIO mice.
FIG. 4-3 shows the effect of conjugate molecule APC-3 of the present application on the random blood glucose of DIO mice.
FIG. 4-4 and FIG. 4-5 show the effect of conjugate molecule APC-3 of the present application on the glucose tolerance of DIO mice.
FIG. 4-6 shows the effect of conjugate molecule APC-3 of the present application on the liver weight, liver triglyceride, serum cholesterol, serum triglyceride, serum low-density lipoprotein cholesterol, serum alanine aminotransferase, and serum aspartate aminotransferase of DIO mice.

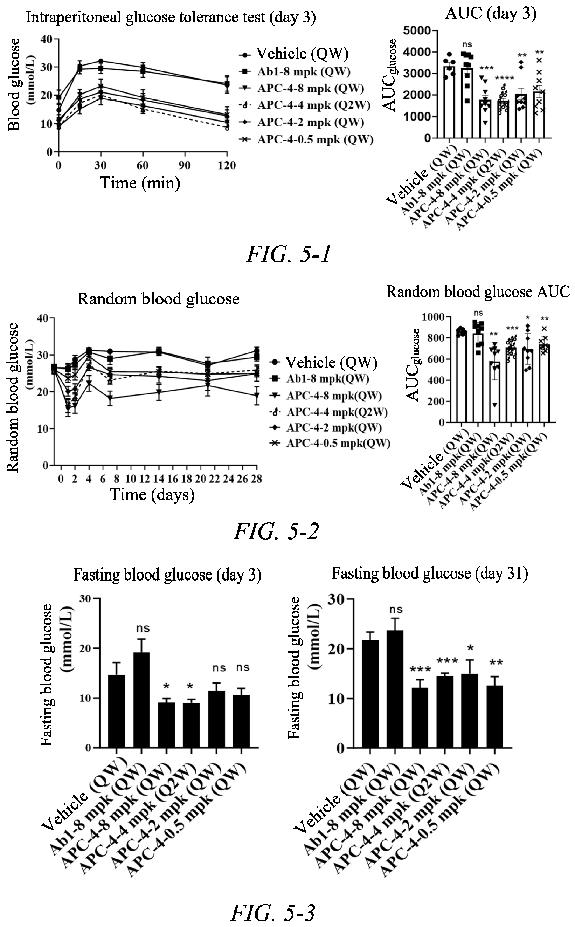

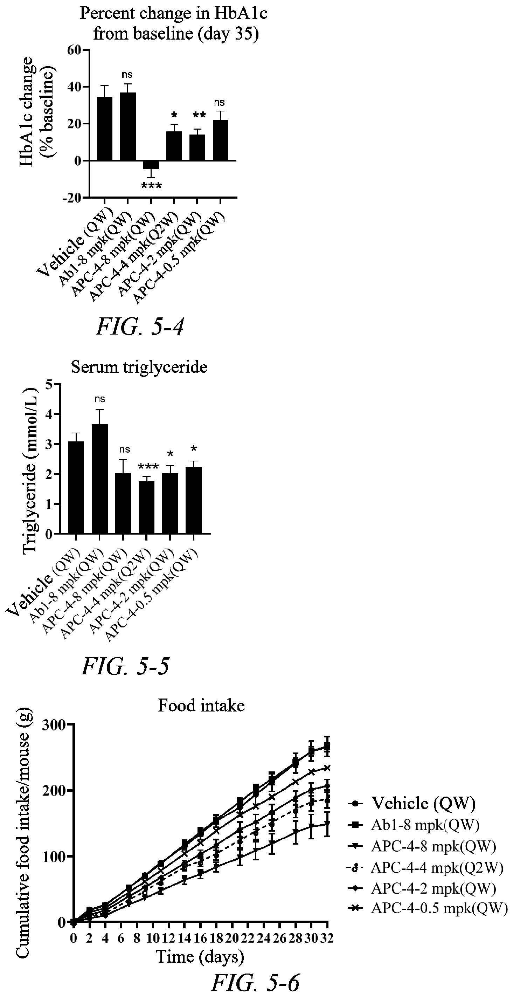

FIG. 5-1 shows the effect of antibody Ab1 and conjugate molecule APC-4 of the present application on the glucose tolerance of db/db mice.
FIG. 5-2 shows the effect of antibody Ab1 and conjugate molecule APC-4 of the present application on the random blood glucose of db/db mice.
FIG. 5-3 shows the effect of antibody Ab1 and conjugate molecule APC-4 of the present application on the fasting blood glucose on day 3 and day 31 of db/db mice.
FIG. 5-4 shows the effect of antibody Ab1 and conjugate molecule APC-4 of the present application on HbAlc of db/db mice.
FIG. 5-5 shows the effect of antibody Ab1 and conjugate molecule APC-4 of the present application on the serum triglyceride of db/db mice.
FIG. 5-6 shows the effect of antibody Ab1 and conjugate molecule APC-4 of the present application on the food intake of db/db mice.

### Detailed Description of Embodiments

### Terms

In order that the present disclosure may be more readily understood, certain technical and scientific terms are described below. Unless otherwise explicitly defined herein, all technical and scientific terms used herein have the meaning as commonly understood by those of ordinary skill in the art.

The singular forms "a", "an", and "the" used in the description and claims include plural referents unless the context clearly dictates otherwise.

Unless the context clearly requires otherwise, throughout the description and the claims, the words "comprise", "have", "include", and the like are to be construed in a sense as "including but not limited to", rather than an exclusive or exhaustive sense.

The term "cytokine" is a general term for proteins that are released by one cell population to act as an intercellular mediators on other cells. Examples of such cytokines include lymphokines, monokines, chemokines, and traditional polypeptide hormones. Exemplary cytokines include: mIL-2, IFNγ, TNFα, CCL-2 and IL-6.

The term "and/or" means including both the meanings of "and" and "or". For example, the phrase "A, B, and/or C" is intended to encompass each of the following: A, B and C; A, B or C; A or C; A or B; B or C; A and C; A and B; B and C; A (alone); B (alone); and C (alone).

The three-letter and single-letter codes for amino acids used in the present disclosure are described in J. biol. chem, 243, p 3558 (1968).

The term "native GLP-1" refers to a peptide comprising the sequence of human GLP-1 (7-36 or 7-37).

The term "native GIP" refers to a peptide comprising the sequence of human GIP (1-42).

The term "native GCG" refers to a peptide comprising the sequence of human GCG (1-29).

The terms "GLP-1", "GIP" or "GCG", if not explained further, refer to native GLP-1, native GIP or native GCG, respectively.

The term "glucagon-like peptide-1 receptor agonist peptide" i.e., a GLP-1R agonist peptide, includes GLP-1R monoagonist peptides (i.e., peptides having monoagonist activity against GLP-1R), and dual agonist peptides (including GLP-1R/GIPR dual agonist peptides (i.e., peptides having dual agonist activity against GLP-1R/GIPR) and GLP-1R/GCGR dual agonist peptides (peptide having dual agonist activity against GLP-1R/GCGR)) and triagonist peptides (GLP-1R/GIPR/GCGR triagonist peptides (i.e., peptide having triagonist activity against GLP-1R/GIPR/GCGR)).

The term "glucagon-like peptide-1 receptor agonist peptide analogue" i.e., a GLP-1R monoagonist peptide analogue, includes GLP-1R monoagonist peptide analogues, and analogues of dual agonist peptides (GLP-1R/GIPR dual agonist peptides or GLP-1R/GCGR dual agonist peptides) and analogues of triagonist peptides (GLP-1R/GIPR/GCGR triagonist peptides). For example, a peptide having an amino acid sequence comprising 1, 2, 3, 4, 5, 6, 7 or 8 amino acid substitutions, insertions, deletions, or a combination of two or more thereof, as compared to the amino acid sequence of a GLP-1R agonist peptide. GLP-1R agonist peptide analogues include amidated forms, acid forms, pharmaceutically acceptable salt forms, and any other physiologically active form of the molecule.

The term "amino acid" refers to naturally occurring and synthetic amino acids, as well as amino acid analogues and amino acid mimics that function in a manner similar to the naturally occurring amino acids. Naturally occurring amino acids are those encoded by genetic codes and those later modified, e.g., hydroxyproline, γ-carboxyglutamic acid, and O-phosphoserine. Amino acid analogues refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methionine methyl sulfonium. Such analogues have a modified R group (e.g., norleucine) or a modified peptide skeleton, but retain a substantially identical chemical structure to naturally occurring amino acids. Amino acid mimics refer to chemical compounds that have a structure that is different from the general chemical structure of amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 82aR indicates that an amino acid residue at position 82a is R. S82aR indicates that an amino acid residue at position 82a (also known as 82A) is mutated from the original S to R.

The term "antibody" may be a natural or conventional antibody in which two heavy chains are linked to each other by disulfide bonds and each heavy chain is linked to a light chain by disulfide bonds, including but not limited to monoclonal antibodies and polyclonal antibodies, monospecific antibodies, multispecific antibodies (e.g., bispecific antibodies), and full-length antibodies, as long as they exhibit the desired antigen-binding activity. Examples include chimeric antibodies, humanised antibodies, and fully human antibodies.

The term "bispecific antibody" refers to an antibody (including an antibody or antigen-binding fragment thereof, such as a single chain antibody) capable of specifically binding to two different antigens or at least two different epitopes of the same antigen. Various structures of bispecific antibodies have been disclosed in the prior art. On the basis of the integrity of the IgG molecule, bispecific antibodies can be classified into IgG-like bispecific antibodies and antibody fragment-type bispecific antibodies. On the basis of the number of antigen-binding regions, they can be classified into bivalent, trivalent, tetravalent, or higher-valent bispecific antibodies. On the basis of whether the structure is symmetric, they can be classified into symmetric bispecific antibodies and asymmetric bispecific antibodies. Among them, the bispecific antibodies based on antibody fragments, such as Fab fragments lacking Fc fragments, e.g., bispecific antibodies formed by binding two or more Fab fragments in one molecule, have low immunogenicity, small molecular weight, and high tumour tissue permeability, and typical antibody structures of this type include bispecific antibodies such as F(ab)₂, scFv-Fab, and (scFv)₂-Fab; and IgG-like bispecific antibodies (e.g., having Fc fragments) have relatively large molecular weight, wherein the Fc fragments facilitate purification of the antibody and increase their solubility and stability, and the Fc portions may further bind to the receptor FcRn and increase the serum half-life of the antibody.

"Native antibody" refers to naturally occurring immunoglobulin molecules. For example, native IgG antibodies are heterotetrameric glycoproteins of about 150,000 daltons, consisting of two identical light chains and two identical heavy chains that are disulfide-bonded. From the N-terminus to the C-terminus, each heavy chain has a variable region (VH), also known as variable heavy domain or heavy chain variable region, followed by a heavy chain constant region. A native IgG heavy chain constant region usually contains three constant domains (CH1, CH2 and CH3). Similarly, from the N-terminus to the C-terminus, each light chain has a variable region (VL), also known as variable light domain or light chain variable domain, followed by a constant light domain (light chain constant region, CL). The terms "full-length antibody", "intact antibody", and "whole antibody" are used herein interchangeably and refer to an antibody having a structure substantially similar to a native antibody structure or having heavy chains that comprise an Fc region as defined herein. A native intact antibody light chain comprises a light chain variable region VL and a constant region CL, with VL located at the amino terminus of the light chain and the light chain constant region comprising a κ chain and a λ chain; a native intact antibody heavy chain comprises a variable region VH and constant regions (CH1, CH2, and CH3), with VH located at the amino terminus of the heavy chain and the constant regions located at the carboxyl terminus, wherein CH3 is closest to the carboxyl terminus of the polypeptide. The heavy chain can be of any isotype, including IgG (including IgG1, IgG2, IgG3, and IgG4 subtypes), IgA (including IgA1 and IgA2 subtypes), IgM, and IgE.

The term "variable region" or "variable domain" of an antibody refers to a domain of an antibody heavy or light chain that is involved in the binding of the antibody to an antigen. Herein, an antibody heavy chain variable region (VH) and an antibody light chain variable region (VL) each comprise four conserved framework regions (FRs) and three complementarity determining regions (CDRs). The term "complementarity determining region" or "CDR" refers to a region within a variable domain that primarily contributes to binding to an antigen; "framework" or "FR" refers to variable domain residues other than CDR residues. VH comprises 3 CDR regions: HCDR1, HCDR2, and HCDR3; VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, FR4.

The amino acid sequence boundaries of CDRs may be determined by various well-known schemes, such as the "Kabat" numbering scheme (see Kabat et al. (1991), "Sequences of Proteins of Immunological Interest", 5th edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), the ImMunoGenTics (IMGT) numbering scheme (see Lefranc, M.P. et al., Dev. Comp. Immunol., 27, 55-77 (2003); Front Immunol. 2018 Oct 16; 9: 2278), and the like; the correspondences among various numbering systems are well known to those skilled in the art. Exemplary, as shown in Table 1.

**Table 1. Relationships among CDR numbering systems**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure.

The term "Fc region" or "fragment crystallisable region" is used to define the C-terminal region of the heavy chain of an antibody, including native Fc regions and engineered Fc regions. In some embodiments, the Fc region comprises two subunits that are identical or different. In some embodiments, the Fc region of a human IgG heavy chain is defined as extending from the amino acid residue at position Cys226 or from Pro230 to its carboxyl terminus. Suitable Fc regions for use in the antibodies described herein include Fc regions of human IgG1, IgG2 (IgG2A and IgG2B), IgG3, and IgG4. In some embodiments, the boundary of the Fc region may also vary, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" antibody refers to an antibody in which a portion of the heavy and/or light chain is derived from a particular source or species, while the remainder of the heavy and/or light chain is derived from another different source or species.

The term "humanised" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to an antibody in which the variable regions and constant regions are of human sequences. The term encompasses antibodies that are derived from human genes but have, for example, sequences that have been altered to reduce possible immunogenicity, increase affinity, and eliminate cysteines or glycosylation sites that may cause undesired folding. The term encompasses such antibodies recombinantly produced in non-human cells (that may confer glycosylation not characteristic of human cells). The term also encompasses antibodies that have been raised in transgenic mice comprising some or all of the human immunoglobulin heavy and light chain loci. The meaning of a human antibody explicitly excludes humanised antibodies comprising non-human antigen-binding residues.

The term "affinity" refers to the strength of the sum total of noncovalent interactions between a single binding site of a molecule (e.g., an antibody) and its binding partner (e.g., an antigen). Unless otherwise indicated, as used herein, binding "affinity" refers to intrinsic binding affinity which reflects a 1:1 interaction between members of a binding pair (e.g., antibody and antigen). The affinity of a molecule X for its ligand Y can generally be represented by the dissociation constant (KD). Affinity can be measured by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and is expressed as a molar concentration (M). The KD value of an antibody can be determined using methods well known in the art. For example, a biosensor system such as a system for measuring surface plasmon resonance (e.g., Biacore) is used, or affinity in solution is measured by solution equilibrium titration (SET).

The term "surface plasmon resonance" refers to an optical phenomenon that allows for the analysis of real-time interactions by detecting changes in protein concentrations within a biosensor matrix, for example, using the BIAcoreTM system (Biacore LifeSciences division of GE Healthcare, Piscataway, NJ).

The term "effector function" refers to those biological activities attributable to the Fc region of an antibody (a native sequence Fc region or an Fc region with amino acid sequence mutations) that vary with the antibody isotype. Examples of antibody effector functions include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for naturally occurring mutations that may be present in minor amounts. In contrast, polyclonal antibody formulations generally comprise a plurality of different antibodies having different amino acid sequences in their variable domains, which are generally specific for different epitopes. "Monoclonal" indicates the characteristic of an antibody obtained from a population of substantially homogeneous antibodies, and is not to be construed as requiring production of the antibody by any particular method. In some embodiments, the antibody provided by the present disclosure is a monoclonal antibody.

The term "antigen" refers to a molecule or portion of a molecule that is capable of being bound by a selective binding agent, such as an antigen-binding protein (including, for example, an antibody), and that is also capable of being used in an animal to produce an antibody that is capable of binding to the antigen. An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from a string of contiguous amino acids (linear epitopes) or comprise non-contiguous amino acids (conformational epitopes), e.g., brought into spatial proximity by folding of an antigen (i.e., by tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as, but not limited to, alanine scanning, peptide blotting, peptide cleavage analysis, epitope excision, epitope extraction, chemical modification of the antigen (see Prot. Sci. 9 (2000) 487-496), and cross-blocking.

The term "capable of specifically binding", "specifically bind", or "bind" means that an antibody is capable of binding to a certain antigen or an epitope of the antigen with higher affinity than to other antigens or epitopes. Generally, an antibody binds to an antigen or an epitope within an antigen with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD for the binding of an antibody to an antigen is 10% or less (e.g., 1%) of the KD for the binding of the antibody to a non-specific antigen (e.g., BSA or casein). KD may be measured using known methods, for example, by a BIACORE^{®} surface plasmon resonance assay. However, an antibody specifically binding to an antigen or an epitope within an antigen may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (common marmoset, marmoset).

The term "antibody-dependent cellular cytotoxicity", "antibody-dependent cell-mediated cytotoxicity", or "ADCC" is a mechanism for inducing cell death, the mechanism relies on the interaction of antibody-coated target cells with effector cells having lytic activity, such as natural killer cells (NK), monocytes, macrophages, and neutrophils, via Fcγ receptors (FcγRs) expressed on the effector cells. For example, NK cells express FcγRIIIa, while monocytes express FcγRI, FcγRII, and FcγRIIIa. The ADCC activity of the antibodies provided herein can be assessed using in vitro assays, with cells expressing the antigen as target cells and NK cells as effector cells. Cell lysis is detected on the basis of the release of a label (e.g., a radioactive substrate, a fluorescent dye, or a natural intracellular protein) from lysed cells.

The term "antibody-dependent cellular phagocytosis (ADCP)" refers to a mechanism by which antibody-coated target cells are eliminated by the internalisation of phagocytic cells (such as macrophages or dendritic cells).

The term "complement-dependent cytotoxicity" or "CDC" refers to a mechanism for inducing cell death in which the Fc effector domain of a target-binding antibody binds to and activates the complement component C1q, and C1q then activates the complement cascade, resulting in the death of the target cell. Activation of complement may also result in the deposition of complement components on the surface of target cells, and these complement components promote CDC by binding to complement receptors on leukocytes (e.g., CR3).

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the extent (percentage) to which the amino acids/nucleic acids of two sequences are identical at equivalent positions when the two sequences are optimally aligned, with gaps introduced if necessary to achieve the maximum percent sequence identity, and without considering any conservative substitutions as part of the sequence identity. The alignment for determining percent sequence identity can be achieved by known techniques in the art, for example, using publicly available computer software, such as BLAST, BLAST-2, ALIGN, ALIGN-2 or Megalign (DNASTAR) software. Those skilled in the art can determine parameters suitable for measuring alignment, including any algorithm needed to achieve the maximum alignment over the full length of the sequences being compared.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (in conjunction with the host cell) the expression of one or more heterologous coding regions operably linked thereto. An expression construct may include, but is not limited to, sequences that affect or control transcription, translation, and, if introns are present, affect RNA splicing of a coding region operably linked thereto.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be completely identical to parent cells in terms of nucleic acid content and may contain mutations. Variant progeny that have the same function or biological activity as those screened or selected in the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichia pastoris, Pichia finlandica, Pichia trehalophila, Pichia koclamae, Pichia membranaefaciens*, *Pichia minuta (Ogataea minuta, Pichia lindneri), Pichia opuntiae, Pichia thermotolerans, Pichia salictaria, Pichia guercuum, Pichia pijperi*, *Pichia stiptis, Pichia methanolica, Pichia, Saccharomyces cerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa. Pichia,* any *Saccharomyces, Hansenula polymorpha,* any *Kluyveromyces, Candida albicans,* any *Aspergillus, Trichoderma reesei, Chrysosporium lucknowense,* any *Fusarium, Yarrowia lipolytica,* and *Neurospora crassa.*

"Cell", "cell line", and "cell culture" are used interchangeably, and all such designations include progeny. Thus, the words "transformant" and "transformed cell" include primary subject cells and cultures derived therefrom, regardless of the number of passages. It is also understood that not all progeny have precisely identical DNA contents due to deliberate or inadvertent mutations. Variant progeny that have the same function or biological activity as the originally transformed cell are included.

The term "alkyl" refers to a saturated, straight or branched aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl preferably has 1 to 12 carbon atoms (i.e., C₁₋₁₂ alkyl), more preferably 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, etc. The alkyl can be substituted or unsubstituted. When substituted, the alkyl can be substituted at any available point of attachment, and the substituents are preferably one or more selected from the group consisting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples include: methoxy, ethoxy, propoxy, butoxy, etc. The alkoxy can be substituted or unsubstituted. When substituted, the alkoxy can be substituted at any available point of attachment, and the substituents are preferably one or more selected from the group consisting of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl preferably has 3 to 12 ring atoms (i.e., 3- to 12-membered cycloalkyl), more preferably 3 to 8 ring atoms (i.e., 3- to 8-membered cycloalkyl), and most preferably 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The polycyclic cycloalkyl includes: spirocycloalkyl, fused cycloalkyl and bridged cycloalkyl.

The term "spirocycloalkyl" refers to a polycyclic system in which the rings share one carbon atom (called spiro atom), wherein the ring can contain one or more double bonds, or the ring can contain one or more heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or -S-S-), provided that at least one all-carbon ring is present and the point of attachment is on the all-carbon ring. The spirocycloalkyl has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl preferably has 6 to 14 ring atoms (i.e., 6- to 14-membered spirocycloalkyl), more preferably 7 to 10 ring atoms (i.e., 7- to 10-membered spirocycloalkyl). The spirocycloalkyl includes monospirocycloalkyl and polyspirocycloalkyl (such as dispirocycloalkyl), preferably monospirocycloalkyl or dispirocycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl. Non-limiting examples include: 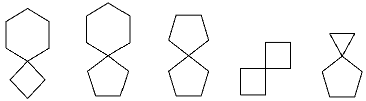 (the point of attachment can be at any position); 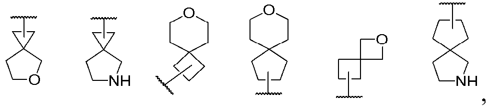 etc.

The term "fused cycloalkyl" refers to a polycyclic system in which the rings share two adjacent carbon atoms, formed by fusing monocyclic cycloalkyl with one or more monocyclic cycloalkyl, or by fusing monocyclic cycloalkyl with one or more of heterocyclyl, aryl, or heteroaryl, wherein the point of attachment is on the monocyclic cycloalkyl, and the ring can contain one or more double bonds. The fused cycloalkyl has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl preferably has 6 to 14 ring atoms (i.e., 6- to 14-membered fused cycloalkyl), more preferably 7 to 10 ring atoms (i.e., 7- to 10-membered fused cycloalkyl). The fused cycloalkyl includes bicyclic fused cycloalkyl and polycyclic fused cycloalkyl (such as tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl. Non-limiting examples include: 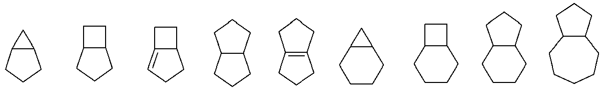 (the point of attachment can be at any position); 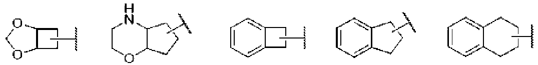 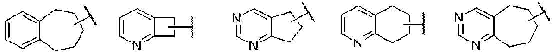 etc.

The term "bridged cycloalkyl" refers to an all-carbon polycyclic system in which the rings share two carbon atoms that are not directly linked, wherein the ring can contain one or more double bonds. The bridged cycloalkyl has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl preferably has 6 to 14 carbon atoms (i.e., 6- to 14-membered bridged cycloalkyl), more preferably 7 to 10 carbon atoms (i.e., 7- to 10-membered bridged cycloalkyl). The bridged cycloalkyl includes bicyclic bridged cycloalkyl and polycyclic bridged cycloalkyl (such as tricyclic bridged cycloalkyl and tetracyclic bridged cycloalkyl), preferably bicyclic bridged cycloalkyl or tricyclic bridged cycloalkyl. Non-limiting examples include: 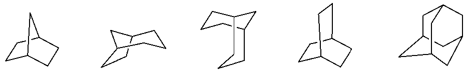 (the point of attachment can be at any position).

The cycloalkyl can be substituted or unsubstituted. When substituted, the cycloalkyl can be substituted at any available point of attachment, and the substituents are preferably one or more selected from the group consisting of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "heterocyclyl" refers to a saturated or partially unsaturated monocyclic heterocycle (i.e., monocyclic heterocyclyl) or polycyclic heterocyclic system (i.e., polycyclic heterocyclyl), wherein the ring contains at least one (e.g., 1, 2, 3 or 4) heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or -S-S-). The heterocyclyl has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl preferably has 3 to 12 ring atoms (i.e., 3- to 12-membered heterocyclyl), for example 4- to 12-membered heterocyclyl containing at least one nitrogen atom; the heterocyclyl further preferably has 3 to 8 ring atoms (i.e., 3- to 8-membered heterocyclyl); the heterocyclyl more preferably has 3 to 6 ring atoms (i.e., 3- to 6-membered heterocyclyl); the heterocyclyl most preferably has 5 or 6 ring atoms (i.e., 5- or 6-membered heterocyclyl).

Non-limiting examples of the monocyclic heterocyclyl include: pyrrolidyl, tetrahydropyranyl, 1,2,3,6-tetrahydropyridyl, piperidyl, piperazinyl, morpholinyl, thiomorpholinyl, homopiperazinyl, etc.

The polycyclic heterocyclyl includes spiroheterocyclyl, fused heterocyclyl and bridged heterocyclyl.

The term "spiroheterocyclyl" refers to a polycyclic heterocyclic system in which the rings share one atom (called spiro atom), wherein the ring can contain one or more double bonds, and the ring contains at least one (e.g., 1, 2, 3 or 4) heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or -S-S-), provided that at least one monocyclic heterocycly is present and the point of attachment is on the monocyclic heterocycly. The spiroheterocyclyl has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl preferably has 6 to 14 ring atoms (i.e., 6- to 14-membered spiroheterocyclyl), more preferably 7 to 10 ring atoms (i.e., 7- to 10-membered spiroheterocyclyl). The spiroheterocyclyl includes monospiroheterocyclyl and polyspiroheterocyclyl (such as dispiroheterocyclyl), preferably monospiroheterocyclyl or dispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocyclyl. Non-limiting examples include: 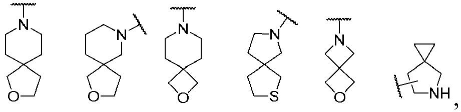 etc.

The term "fused heterocyclyl" refers to a polycyclic heterocyclic system in which the rings share two adjacent atoms, wherein the ring can contain one or more double bonds, and the ring contains at least one (e.g., 1, 2, 3 or 4) heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or - S-S-), formed by fusing monocyclic heterocyclyl with one or more monocyclic heterocyclyl, or by fusing monocyclic heterocyclyl with one or more of cycloalkyl, aryl or heteroaryl, wherein the point of attachment is on the monocyclic heterocycly. The fused heterocyclyl has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl preferably has 6 to 14 ring atoms (i.e., 6- to 14-membered fused heterocyclyl), more preferably 7 to 10 ring atoms (i.e., 7- to 10-membered fused heterocyclyl). The fused heterocyclyl includes bicyclic and polycyclic fused heterocyclyl (such as tricyclic fused heterocyclyl and tetracyclic fused heterocyclyl), preferably bicyclic fused heterocyclyl or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused heterocyclyl. Non-limiting examples include: 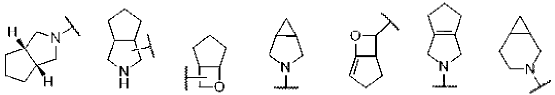 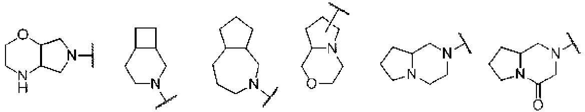 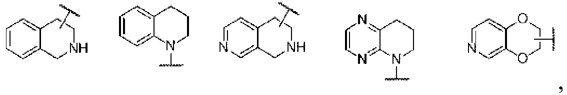 etc.

The term "bridged heterocyclyl" refers to a polycyclic heterocyclic system in which the rings share two atoms that are not directly linked, wherein the ring can contain one or more double bonds, and the ring contains at least one (e.g., 1, 2, 3 or 4) heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or -S-S-). The bridged heterocyclyl has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl preferably has 6 to 14 ring atoms (i.e., 6- to 14-membered bridged heterocyclyl), more preferably 7 to 10 ring atoms (i.e., 7- to 10-membered bridged heterocyclyl). Based on the number of constituent rings, the bridged heterocyclyl can be classified into bicyclic bridged heterocyclyl and polycyclic bridged heterocyclyl (such as tricyclic bridged heterocyclyl and tetracyclic bridged heterocyclyl), preferably bicyclic bridged heterocyclyl or tricyclic bridged heterocyclyl. Non-limiting examples include: 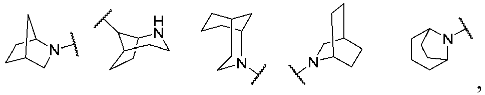 etc.

The heterocyclyl can be substituted or unsubstituted. When substituted, the heterocyclyl can be substituted at any available point of attachment, and the substituents are preferably one or more selected from the group consisting of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "aryl" refers to a monocyclic all-carbon aromatic ring (i.e., monocyclic aryl) or polycyclic aromatic ring system (i.e., polycyclic aryl) having a conjugated π-electron system. The aryl has 6 to 14 (e.g., 6, 7, 8, 9, 10, 11, 12, 13, or 14) ring atoms (i.e., 6- to 14-membered aryl). The aryl preferably has 6 to 10 ring atoms (i.e., 6- to 10-membered aryl). The aryl is monocyclic aryl, such as phenyl. Non-limiting examples of the polycyclic aryl include: naphthyl, anthryl, phenanthryl, etc. The polycyclic aryl further includes phenyl fused with one or more of heterocyclyl or cycloalkyl, or naphthyl fused with one or more of heterocyclyl or cycloalkyl, wherein the point of attachment is on the phenyl or naphthyl. In such cases, the number of ring atoms still represents the number of ring atoms in the polycyclic aromatic ring system. Non-limiting examples include: 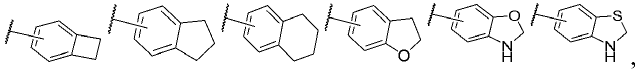 etc.

The aryl can be substituted or unsubstituted. When substituted, the aryl can be substituted at any available point of attachment, and the substituents are preferably one or more selected from the group consisting of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, wherein the ring contains at least one (e.g., 1, 2, 3 or 4) heteroatoms selected from the group consisting of nitrogen, oxygen and sulfur (the nitrogen can optionally be oxidised, i.e., to form nitrogen oxides; and the sulfur can optionally be oxo-substituted, i.e., to form sulfoxides or sulfones, but excluding -O-O-, -O-S- or -S-S-). The heteroaryl has 5 to 14 (e.g., 5, 6, 7, 8, 9, 10, 11, 12, 13, or 14) ring atoms (i.e., 5- to 14-membered heteroaryl). Preferably, the heteroaryl is heteroaryl having 5 to 10 ring atoms (i.e., 5- to 10-membered heteroaryl); more preferably, the heteroaryl is monocyclic heteroaryl having 5 or 6 ring atoms (i.e., 5- or 6-membered monocyclic heteroaryl) or bicyclic heteroaryl having 8 to 10 ring atoms (i.e., 8- to 10-membered bicyclic heteroaryl); most preferably, the heteroaryl is 5- or 6-membered monocyclic heteroaryl containing 1, 2, or 3 heteroatoms selected from the group consisting of nitrogen, oxygen, and sulfur within the ring, or 8- to 10-membered bicyclic heteroaryl containing 1, 2, or 3 heteroatoms selected from the group consisting of nitrogen, oxygen, and sulfur within the ring.

Non-limiting examples of the monocyclic heteroaryl include: furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkyl pyrrolyl, pyridyl, pyrimidyl, pyridonyl, N-alkylpyridone (such as 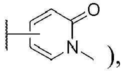 pyrazinyl, pyridazinyl, etc.

Non-limiting examples of the polycyclic heteroaryl include: indolyl, indazolyl, quinolyl, isoquinolyl, quinoxalinyl, phthalazinyl, benzoimidazolyl, benzothienyl, quinazolinyl, benzothiazolyl, carbazolyl, etc. The polycyclic heteroaryl further includes monocyclic heteroaryl fused with one or more aryl, wherein the point of attachment is on the aromatic ring. In such cases, the number of ring atoms still represents the number of ring atoms in the polycyclic heteroaromatic ring system. The polycyclic heteroaryl further includes monocyclic heteroaryl fused with one or more of cycloalkyl or heterocyclyl, wherein the point of attachment is on the monocyclic heteroaromatic ring. In such cases, the number of ring atoms still represents the number of ring atoms in the polycyclic heteroaromatic ring system. Non-limiting examples include: 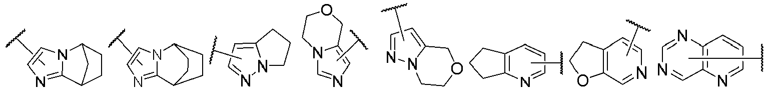 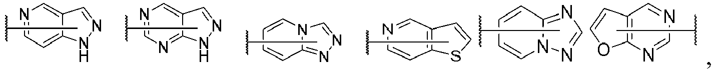 etc.

The heteroaryl can be substituted or unsubstituted. When substituted, the heteroaryl can be substituted at any available point of attachment, and the substituents are preferably one or more selected from the group consisting of a D atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The above cycloalkyl, heterocyclyl, aryl, and heteroaryl include residues derived by removing one hydrogen atom from a parent ring atom, or residues derived by removing two hydrogen atoms from the same ring atom or two different ring atoms of the parent, i.e., "divalent cycloalkyl", "divalent heterocyclyl", "arylene", and "heteroarylene".

In the chemical structures of the compounds of the present disclosure, the bond " 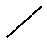 " represents an unspecified configuration; that is, if the chemical structure exists as stereoisomers, the bond " 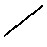 " can be " 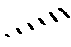 " or " 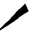 ", or both the configurations of " 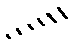 " and " 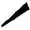 " are involved.

The compounds of the present disclosure include all suitable isotopic derivatives thereof. The term "isotopic derivative" refers to a compound in which at least one atom is replaced with an atom having the same atomic number but a different atomic mass. Examples of isotopes that can be introduced into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D),³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I , preferably deuterium.

Deuterated drugs offer advantages over their non-deuterated counterparts, such as reduced toxicity and side effects, increased drug stability, enhanced efficacy, and prolonged biological half-life. All isotopic variations of the compounds of the present disclosure, whether radioactive or not, are encompassed within the scope of the present disclosure. Each available hydrogen atom attached to a carbon atom can be independently replaced with a deuterium atom, where such deuterium replacement can be partial or complete, and partial deuterium replacement means that at least one hydrogen atom is replaced with at least one deuterium atom.

The term "optional" or "optionally" means that the subsequently described event or circumstance may but need not occur, and that the description includes instances where the event or circumstance occurs and instances in which it does not.

The term "pharmaceutical composition" denotes a mixture containing one or more antibody-polypeptide conjugates described herein or pharmaceutically acceptable salts thereof and other chemical components, such as physiologically/pharmaceutically acceptable carriers and excipients.

The term "pharmaceutically acceptable carrier" refers to an ingredient in a pharmaceutical formulation, other than an active ingredient, which is nontoxic to a subject. Pharmaceutically acceptable carriers include, but are not limited to, buffers, excipients, stabilisers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless indicated, the terms "patient" and "subject" are used interchangeably herein. In certain embodiments, the individual or subject is a human.

When applied to an animal, human, experimental subject, cell, tissue, organ, or biological fluid, "administering" or "giving" refers to the contact of an exogenous drug, therapeutic agent, diagnostic agent, or composition with the animal, human, subject, cell, tissue, organ, or biological fluid.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; etc.), tissue biopsy samples, fine needle punctures, surgically excised tissues, organ cultures, or cell cultures.

"Treatment" or "treat" and "treating" (and grammatical variations thereof) refer to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of a disease or to slow the progression of a disease.

"Effective amount" is generally an amount sufficient to reduce the severity and/or frequency of symptoms, eliminate these symptoms and/or underlying causes, prevent the appearance of symptoms and/or their underlying causes, and/or ameliorate or alleviate damages (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors: such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

Exemplary antibody-polypeptide conjugate or pharmaceutically acceptable salt thereof

Examples of the present disclosure disclose an antibody-polypeptide conjugate or a pharmaceutically acceptable salt thereof, having the structure as represented by general formula (II): 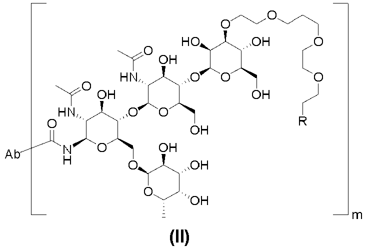 wherein 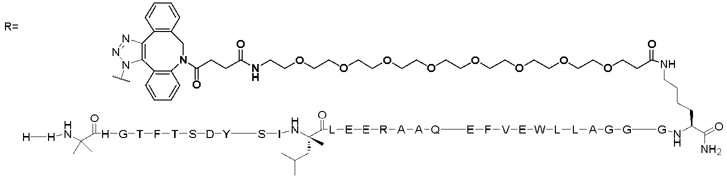 or 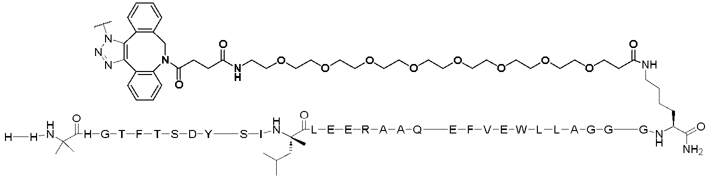
the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11;
m is 2.

Examples of the present disclosure disclose an antibody-polypeptide conjugate or a pharmaceutically acceptable salt thereof, having the structure as represented by general formula (II): 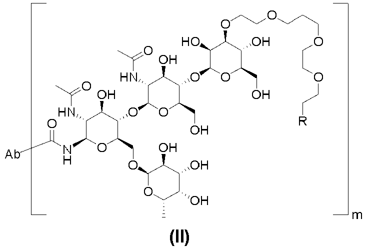 wherein 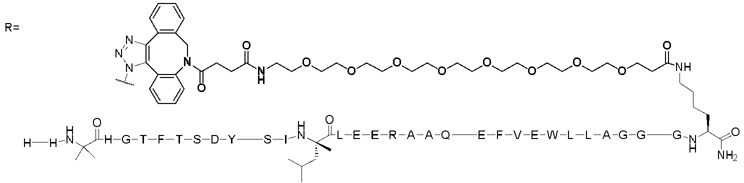 or 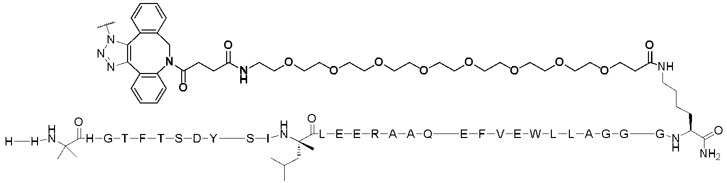
the heavy chain variable region of Ab comprises the amino acid sequence of SEQ ID NO: 12, and the light chain variable region comprises the amino acid sequence of SEQ ID NO: 13;
m is 2.

Examples of the present disclosure disclose an antibody-polypeptide conjugate or a pharmaceutically acceptable salt thereof, having the structure as represented by general formula (II): 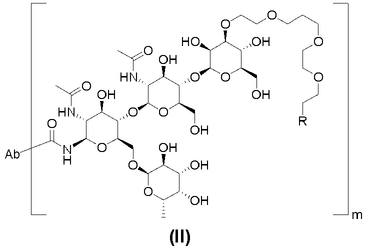 wherein 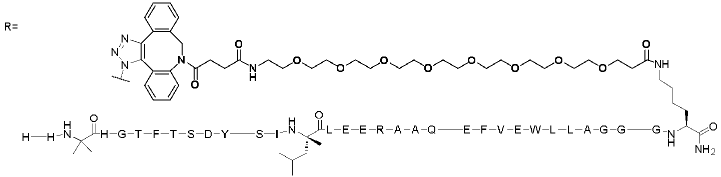 or 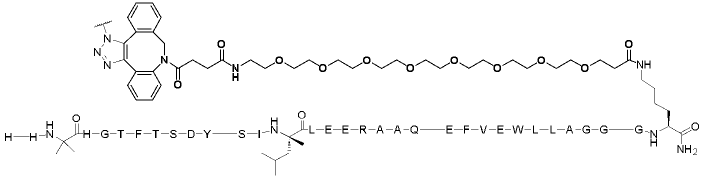
the amino acid sequence of the heavy chain of Ab is as set forth in SEQ ID NO: 16, and the amino acid sequence of the light chain is as set forth in SEQ ID NO: 17;
m is 2.

Examples of the present disclosure disclose an antibody-polypeptide conjugate or a pharmaceutically acceptable salt thereof, having the structure as represented by general formula (II): 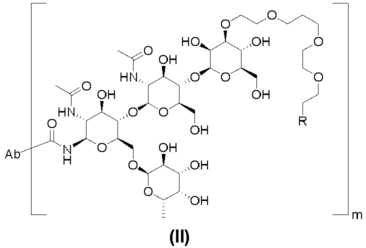 wherein 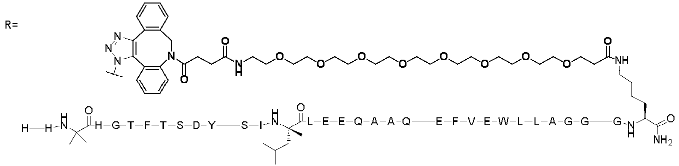 or 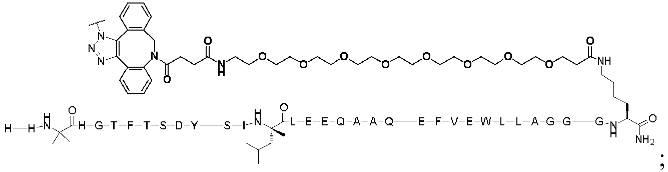
the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11;
m is 2.

Examples of the present disclosure disclose an antibody-polypeptide conjugate or a pharmaceutically acceptable salt thereof, having the structure as represented by general formula (II): 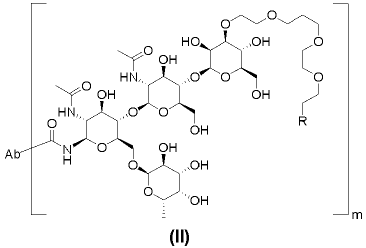 wherein 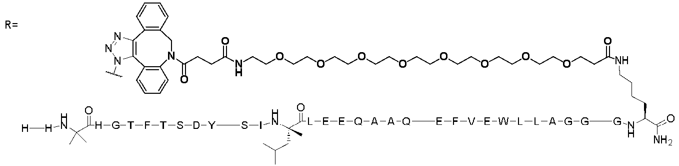 or 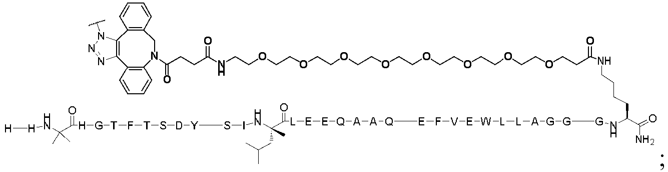
the heavy chain variable region of Ab comprises the amino acid sequence of SEQ ID NO: 12, and the light chain variable region comprises the amino acid sequence of SEQ ID NO: 13;
m is 2.

Examples of the present disclosure disclose an antibody-polypeptide conjugate or a pharmaceutically acceptable salt thereof, having the structure as represented by general formula (II): 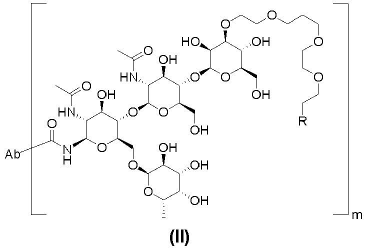 wherein 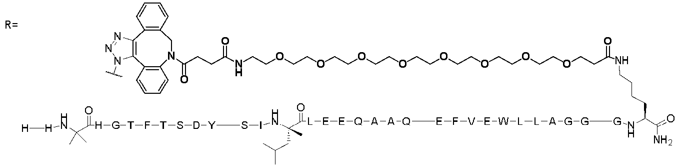 or 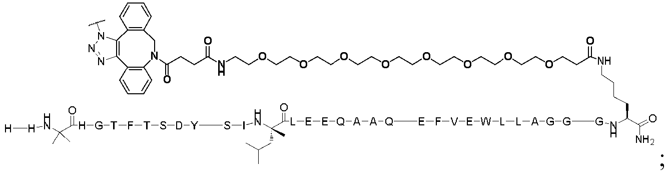
the amino acid sequence of the heavy chain of Ab is as set forth in SEQ ID NO: 16 or 19, and the amino acid sequence of the light chain is as set forth in SEQ ID NO: 17;
m is 2.

Examples of the present disclosure disclose an antibody-polypeptide conjugate or a pharmaceutically acceptable salt thereof, having the structure as represented by general formula (II): 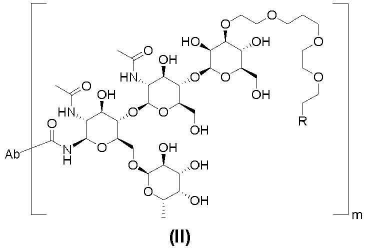 wherein 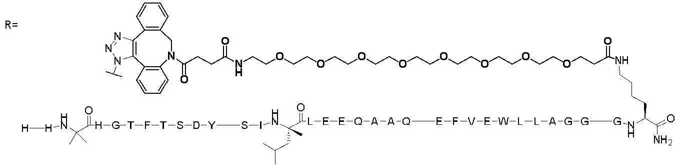 or 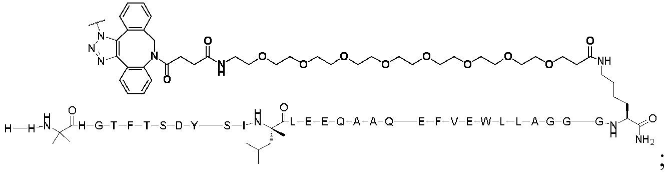
the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
m is 2.

Examples of the present disclosure disclose an antibody-polypeptide conjugate or a pharmaceutically acceptable salt thereof, having the structure as represented by general formula (II): 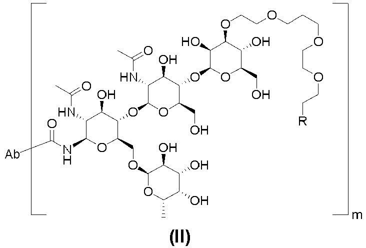 wherein 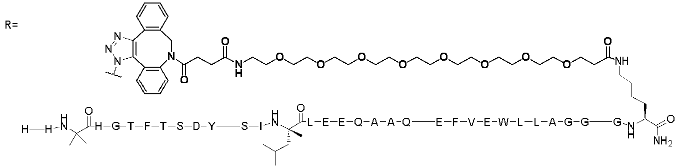 or 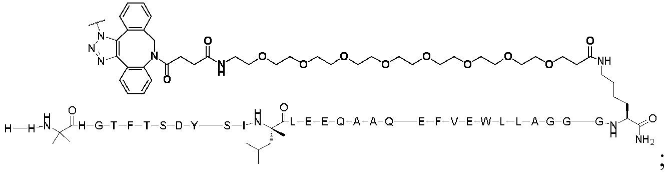
the heavy chain variable region of Ab comprises the amino acid sequence of SEQ ID NO: 29, and the light chain variable region comprises the amino acid sequence of SEQ ID NO: 30;
m is 2.

Examples of the present disclosure disclose an antibody-polypeptide conjugate or a pharmaceutically acceptable salt thereof, having the structure as represented by general formula (II): 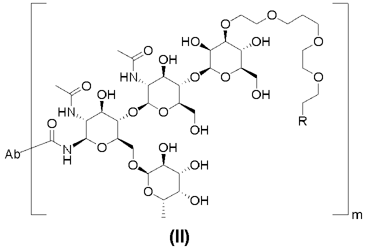 wherein 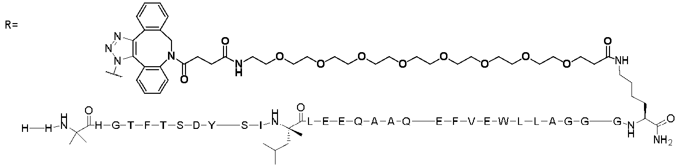 or 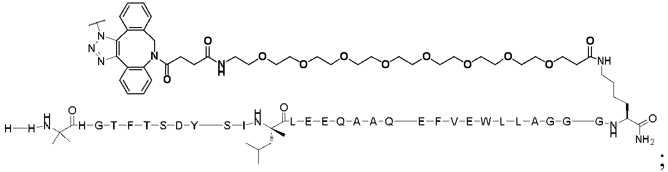
the heavy chain of Ab comprises the amino acid sequence of SEQ ID NO: 33, and the light chain comprises the amino acid sequence of SEQ ID NO: 34;
m is 2.

Examples of the present disclosure disclose an antibody-polypeptide conjugate or a pharmaceutically acceptable salt thereof, having the structure as represented by general formula (II): 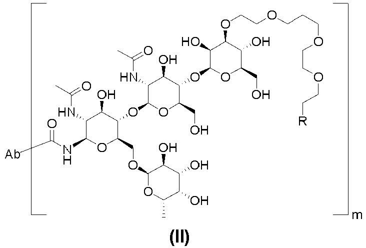 wherein 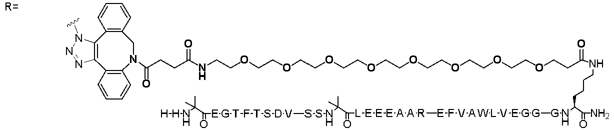 or 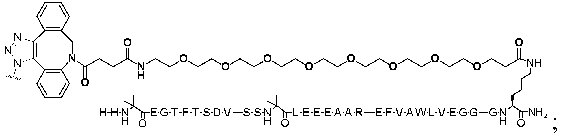
(1) the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11; or
(2) the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
m is 2.

Examples of the present disclosure disclose an antibody-polypeptide conjugate or a pharmaceutically acceptable salt thereof, having the structure as represented by general formula (II): 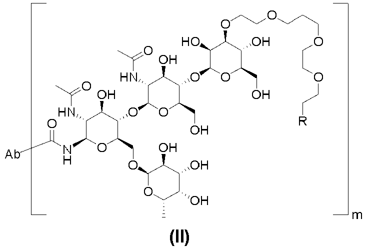 wherein 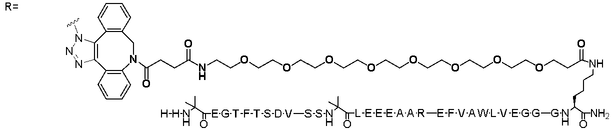 or 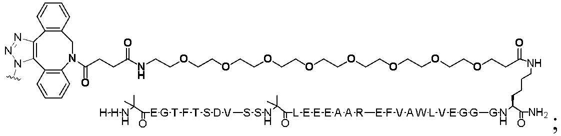
(1) the heavy chain variable region of Ab comprises the amino acid sequence of SEQ ID NO: 12, and the light chain variable region comprises the amino acid sequence of SEQ ID NO: 13; or
(2) the heavy chain variable region of Ab comprises the amino acid sequence of SEQ ID NO: 29, and the light chain variable region comprises the amino acid sequence of SEQ ID NO: 30;
m is 2.

Examples of the present disclosure disclose an antibody-polypeptide conjugate or a pharmaceutically acceptable salt thereof, having the structure as represented by general formula (II): 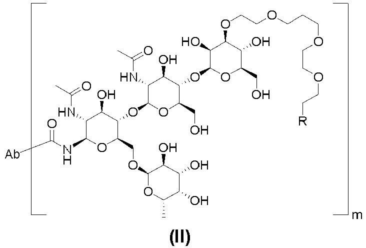 wherein 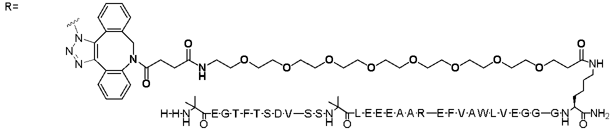 or 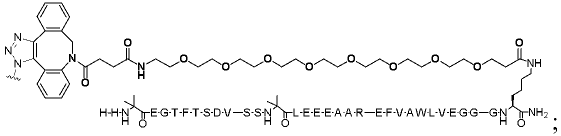
(1) the heavy chain of Ab comprises the amino acid sequence of SEQ ID NO: 16, and the light chain comprises the amino acid sequence of SEQ ID NO: 17; or
(2) the heavy chain of Ab comprises the amino acid sequence of SEQ ID NO: 33, and the light chain comprises the amino acid sequence of SEQ ID NO: 34;
[0420] m is 2.

### Detailed Description of Embodiments

The present disclosure is further described below with reference to examples and test examples. However, these examples and test examples do not limit the scope of the present disclosure. In the examples or test examples of the present disclosure, experimental methods for which specific conditions are not specified are generally performed under conventional conditions, such as those described in Antibodies: A Laboratory Manual and Molecular Cloning: A Laboratory Manual, Cold Spring Harbor Laboratory Press; or under conditions recommended by the manufacturers of the starting materials or commercial products; reagents and materials for which specific sources are not specified are commercially available.

### Examples

### Example 1-1. Construction of recombinant cell line

### 1. Construction of cell lines overexpressing human and cynomolgus monkey KLB & FGFR1c

A cell line expressing a human KLB & FGFR1c complex (see Uniprot ID: Q86Z14 for human KLB sequence and see Uniprot ID: P11362 for human FGFR1c sequence) and a cell line expressing a cynomolgus monkey KLB & FGFR1c complex (see Genebank ID: EHH53620.1 for cynomolgus monkey KLB sequence and see SEQ ID NO: 1 for cynomolgus monkey FGFR1c sequence) were constructed.

Method: Full-length genes of human KLB, monkey KLB, human FGFR1c, and monkey FGFR1c were each cloned into a lentiviral expression vector pCDH (System bioscience, 01. SBI. CD514B-1), wherein the viral expression vector for KLB and the viral expression vector for FGFR1c carried different resistance genes. Viruses for KLB and FGFR1c of different species were respectively packaged. Three plasmids, i.e., pVSV-G, pCMV-dR8.91, and pCDH-KLB or pCDH-FGFR1c, were co-transfected into HEK293T cells (ATCC^{®} CRL-11268) for virus packaging. For constructing cell lines overexpressing the complexes, the CHO-K1 cell (ATCC, CCL-61) line was co-infected with the packaged viruses containing KLB and FGFR1c genes at a certain ratio. After 48 hours of infection, the virus-containing culture supernatant was removed, and after 5 days of pressure screening with the corresponding antibiotics, CHO-K1 cell pools expressing human or monkey KLB & FGFR1c complex were obtained. The cell pools expressing human KLB & FGFR1c complex were subjected to flow cytometry sorting to obtain monoclonal cells stably expressing CHO-K1-hKLB & hFGFR1c.

### Amino acid sequence of cynomolgus monkey FGFR1c:

MWSWKCLLFWAVLVTATLCTARPSPTLPEQDALPSSEDDDDDDDSSSEEK
ETDNTKPNRMPVAPYWTSPEKMEKKLHAVPAAKTVKFKCPSSGTPNPTLR
WLKNGKEFKPDHRIGGYKVRYATWSIIMDSVVPSDKGNYTCIVENEYGSI
NHTYQLDVVERSPHRPILQAGLPANKTVALGSNVEFMCKVYSDPQPHIQW
LKHIEVNGSKIGPDNLPYVQILKTAGVNTTDKEMEVLHLRNVSFEDAGEYT
CLAGNSIGLSHHSAWLTVLEALEERPAVMTSPLYLEIIIYCTGAFLISCMVG
SVIVYKMKSGTKKSDFHSQMAVHKLAKSIPLRRQVTVSADSSASMNSGVL
LVRPSRLSSSGTPMLAGVSEYELPEDPRWELPRDRLVLGKPLGEGCFGQVV
LAEAIGLDKDKPNRVTKVAVKMLKSDATEKDLSDLISEMEMMKMIGKHK
NIINLLGACTQDGPLYVIVEYASKGNLREYLQARRPPGLEYCYNPSHNPEE
QLSSKDLVSCAYQVARGMEYLASKKCIHRDLAARNVLVTEDNVMKIADF
GLARDIHHIDYYKKTTNGRLPVKWMAPEALFDRIYTHQSDVWSFGVLLW
EIFTLGGSPYPGVPVEELFKLLKEGHRMDKPSNCTNELYMMMRDCWHAV
PSQRPTFKQLVEDLDRIVALTSNQEYLDLSMPLDQYSPSFPDTRSSTCSSGE
DSVFSHEPLPEEPCLPRHPAQLANGGLKRR
SEQ ID NO: 1

Note: In the above sequence, the signal peptide (single underlined part), the extracellular region (non-underlined part), the transmembrane region (double underlined part), and the cytoplasmic region (dotted underlined part) are arranged in sequence

### 2. Construction of cell lines overexpressing human KLB & FGFR2c, KLB & FGFR3c, and KLB & FGFR4

A cell line expressing human KLB & FGFR2c complex (see Uniprot ID: Q86Z14 for human KLB sequence and see SEQ ID NO: 2 for human FGFR2c sequence), a cell line expressing human KLB & FGFR3c complex (see SEQ ID NO: 3 for human FGFR3c sequence), and a cell line expressing human KLB & FGFR4 complex (see SEQ ID NO: 4 for human FGFR4 sequence) were constructed.

A CHO-K1-hKLB monoclonal cell line stably expressing human KLB was constructed by lentiviral infection. Then, CHO-K1-hKLB & hFGFR2c, CHO-K1-hKLB & hFGFR3c, and CHO-K1-hKLB & hFGFR4 cell pools stably expressing human FGFR2c (human FGF receptor 2c), human FGFR3c (human FGF receptor 3c), and human FGFR4 (human FGF receptor 4), respectively, were constructed by lentiviral infection.
Amino acid sequence of human FGFR2c: 















 


SEQ ID NO: 2
Amino acid sequence of human FGFR3c: 
MGAPACALALCVAVAIVAGASSESLGTEQRVVGRAAEVPGPEPGQQEQLV
FGSGDAVELSCPPPGGGPMGPTVWVKDGTGLVPSERVLVGPQRLQVLNAS
HEDSGAYSCRQRLTQRVLCHFSVRVTDAPSSGDDEDGEDEAEDTGVDTGA
PYWTRPERMDKKLLAVPAANTVRFRCPAAGNPTPSISWLKNGREFRGEHR
IGGIKLRHQQWSLVMESVVPSDRGNYTCVVENKFGSIRQTYTLDVLERSPH
RPILQAGLPANQTAVLGSDVEFHCKVYSDAQPHIQWLKHVEVNGSKVGPD
GTPYVTVLKTAGANTTDKELEVLSLHNVTFEDAGEYTCLAGNSIGFSHHS
AWLVVLPAEEELVEADEAGSVYAGILSYGVGFFLFILVVAAVTLCRLRSPP
KKGLGSPTVHKISRFPLKRQVSLESNASMSSNTPLVRIARLSSGEGPTLANV
SELELPADPKWELSRARLTLGKPLGEGCFGQVVMAEAIGIDKDRAAKPVT
VAVKMLKDDATDKDLSDLVSEMEMMKMIGKHKNIINLLGACTQGGPLYV
LVEYAAKGNLREFLRARRPPGLDYSFDTCKPPEEQLTFKDLVSCAYQVAR
GMEYLASQKCIHRDLAARNVLVTEDNVMKIADFGLARDVHNLDYYKKTT
NGRLPVKWMAPEALFDRVYTHQSDVWSFGVLLWEIFTLGGSPYPGIPVEE
LFKLLKEGHRMDKPANCTHDLYMIMRECWHAAPSQRPTFKQLVEDLDRV
LTVTSTDEYLDLSAPFEQYSPGGQDTPSSSSSGDDSVFAHDLLPPAPPSSGG
SRT
SEQ ID NO: 3
Amino acid sequence of human FGFR4:

MRLLLALLGVLLSVPGPPVLSLEASEEVELEPCLAPSLEQQEQELTVALGQP
VRLCCGRAERGGHWYKEGSRLAPAGRVRGWRGRLEIASFLPEDAGRYLC
LARGSMIVLQNLTLITGDSLTSSNDDEDPKSHRDPSNRHSYPQQAPYWTHP
QRMEKKLHAVPAGNTVKFRCPAAGNPTPTIRWLKDGQAFHGENRIGGIRL
RHQHWSLVMESVVPSDRGTYTCLVENAVGSIRYNYLLDVLERSPHRPILQ
AGLPANTTAVVGSDVELLCKVYSDAQPHIQWLKHIVINGSSFGADGFPYV
QVLKTADINSSEVEVLYLRNVSAEDAGEYTCLAGNSIGLSYQSAWLTVLPE
EDPTWTAAAPEARYTDIILYASGSLALAVLLLLAGLYRGQALHGRHPRPPA
TVQKLSRFPLARQFSLESGSSGKSSSSLVRGVRLSSSGPALLAGLVSLDLPL
DPLWEFPRDRLVLGKPLGEGCFGQVVRAEAFGMDPARPDQASTVAVKML
KDNASDKDLADLVSEMEVMKLIGRHKNIINLLGVCTQEGPLYVIVECAAK

GNLREFLRARRPPGPDLSPDGPRSSEGPLSFPVLVSCAYQVARGMQYLESR
KCIHRDLAARNVLVTEDNVMKIADFGLARGVHHIDYYKKTSNGRLPVKW
MAPEALFDRVYTHQSDVWSFGILLWEIFTLGGSPYPGIPVEELFSLLREGHR
MDRPPHCPPELYGLMRECWHAAPSQRPTFKQLVEALDKVLLAVSEEYLDL
RLTFGPYSPSGGDASSTCSSSDSVFSHDPLPLGSSSFPFGSGVQT
SEQ ID NO: 4

### 3. Construction of cell lines overexpressing human, cynomolgus monkey, and murine GLP1R, as well as CRE and luciferase reporter gene regulated by CRE

Cell lines stably expressing human GLP1R (see Uniprot ID: P43220 for human GLP1R sequence), cynomolgus monkey GLP1R (see Uniprot ID: A0A2K5WDY5 for cynomolgus monkey GLP1R sequence), murine GLP1R (see Uniprot ID: O35659 for murine GLP1R sequence), as well as CRE and a luciferase reporter gene regulated by CRE were constructed.

The expression plasmids containing the full-length genes of human GLP1R, cynomolgus monkey GLP1R, and murine GLP1R were obtained by molecular cloning. After virus packaging, the resulting viruses were used to infect CHO-K1 cells. Screening with puromycin was performed to obtain GLP1R/CHO-K1 cells highly expressing human GLP1R, cynomolgus monkey GLP1R and murine GLP1R, respectively. The pGL4.29[CRE/hygro] plasmid was transfected into hGLP1R/CHO-K1, cynoGLP1R/CHO-K1, and mGLP1R/CHO-K1 cells, respectively, using Lipofectamine 3000. Screening was performed with the addition of hygromycin B to obtain hGLP1R CRE/CHO-K1, cyno GLP1R CRE/CHO-K1, and mGLP1R CRE/CHO-K1 monoclonal stably transfected cell lines.

### 4. Construction of cell lines overexpressing human, cynomolgus monkey, and murine GIPR, as well as CRE and luciferase reporter gene regulated by CRE

Cell lines stably expressing human GIPR (see Uniprot ID: P48546 for human GIPR sequence), cynomolgus monkey GIPR (see Uniprot ID: A0A2K5VD75 for cynomolgus monkey GIPR sequence), murine GIPR (see Uniprot ID: Q0P543 for murine GIPR sequence), as well as CRE and a luciferase reporter gene regulated by CRE were constructed.

The expression plasmids containing the full-length genes of human GIPR, cynomolgus monkey GIPR, and murine GIPR were obtained by molecular cloning. After virus packaging, the resulting viruses were used to infect CHO-K1 cells. Screening with puromycin was performed to obtain GIPR/CHO-K1 cells highly expressing human GIPR, cynomolgus monkey GIPR, and murine GIPR, respectively. The pGL4.29[CRE/hygro] plasmid was transfected into hGIPR/CHO-K1, cynoGIPR/CHO-K1, and mGIPR/CHO-K1 cells, respectively, using Lipofectamine 3000. Screening was performed with the addition of hygromycin B to obtain hGIPR CRE/CHO-K1, cyno GIPR CRE/CHO-K1, and mGIPR CRE/CHO-K1 stably transfected cell pools.

### 5. Construction of cell lines overexpressing human, cynomolgus monkey, and murine GCGR, as well as CRE and luciferase reporter gene regulated by CRE

Cell lines stably expressing human GCGR (see Uniprot ID: P47871 for human GCGR sequence), cynomolgus monkey GCGR (see SEQ ID NO: 5 for cynomolgus monkey GCGR sequence), murine GCGR (see Uniprot ID: Q61606 for murine GCGR sequence), as well as CRE and a luciferase reporter gene regulated by CRE were constructed.

The expression plasmids containing the full-length genes of human GCGR, cynomolgus monkey GCGR, and murine GCGR were obtained by molecular cloning. After virus packaging, the resulting viruses were used to infect CHO-K1 cells. Screening with puromycin or G418 was performed to obtain GCGR/CHO-K1 cells highly expressing human GCGR, cynomolgus monkey GCGR, and murine GCGR, respectively. The pGL4.29[CRE/hygro] plasmid was transfected into hGCGR/CHO-K1, cynoGCGR/CHO-K1, and mGCGR/CHO-K1 cells, respectively, using Lipofectamine 3000. Screening was performed with the addition of hygromycin B to obtain hGCGR CRE/CHO-K1, cyno GCGR CRE/CHO-K1, and mGCGR CRE/CHO-K1 monoclonal stably transfected cell lines.

### Amino acid sequence of cynomolgus monkey GCGR:

MPPCQPRRPLLLLLLLLACQPQAPSAQVMDFLFEKWKLYGDQCHHNLSLL
PPPTELVCNRTFDKYSCWPDTPANTTANISCPWYLPWHHKVQHRFVFKRC
GPDGQWVRGPRGQPWRDASQCQMDGEELEVQKEVAKMYSSFQVMYTV
GYSLSLGALLLALAVLGGISKLHCTRNAIHANLFVSFVLKASSVLVIDGLLR
TRYSQKIGDDLSVSIWLSDGAVAGCRVAAVFMQYGVVANYCWLLVEGLY
LHNLLGLATLPERSFFSLYLGIGWGAPMLFIIPWVVVRCLFENIQCWTSND

NMGFWWILRFPVFLAILINFFIFIRIVHLLVAKLRAREMHHTDYKFRLAKST
LTLIPLLGVHEVVFAFVTDEHAQGTLRFAKLFFDLFLSSFQGLLVAVLYCFL
NKEVQSELRRHWHRWRLGKVLQEERGTSNHKAPSAPGQGLPGKKLQSGR
DGGSQDSSAEIPLAGGLPRLAESPFSTLLGPQLGLDSGT
SEQ ID NO: 5

### Example 1-2. Preparation of antibody

### 1. Antibody expression

The Lonza CHO-K1 cells stably overexpressing antibody Ab1 (whose amino acid sequence is derived from patent WO 2023046071 A1) were adjusted to a density of 0.5 × 10⁶/mL. The cells were further cultured, and the feed was added on days 4, 7, 9, and 11, respectively. The sugar content in the culture medium was measured before each feed addition, and the sugar concentration was supplemented to 8-12 g/L. On day 14 of culture, the expression supernatant was collected, and centrifuged at a high speed to remove impurities.

Antibody Ab2 (obtained by Fc mutation of antibody Ab1) was expressed in HEK293 6e cells: On day 0, HEK293 6e cells were adjusted to 0.8 × 10⁶/mL, and the cells were further cultured. On day 1, cell transfection was performed, and the plasmids expressing the heavy chain and light chain of Ab2 antibody were prepared into a transfection mixture at a ratio of 2:3. On day 3, the feed was added, and the cells were further cultured. On day 7 of culture, the cell supernatant was collected, and centrifuged at a high speed to remove impurities.

### 2. Purification of antibody

Purification was performed using a Protein A column. The column was rinsed with PBS until the A280 reading decreased to the baseline. The protein of interest was eluted with 100 mM acetic acid buffer at pH 3.0 and neutralized with 1 M Tris-HCl at pH 8.0. The eluted sample was appropriately concentrated and further purified by gel chromatography Superdex200 (GE) equilibrated with PBS to remove aggregates, and the eluate with monomer peak was collected and aliquoted for later use.

The sequence of antibody Ab1 is as follows:

**Table 2. CDR sequences of antibody Ab1**

| | | | |
|---|---|---|---|
| HCDR1 | NYGIN (SEQ ID NO: 6) | LCDR1 | RASKSISKFLA (SEQ ID NO: 9) |
| HCDR2 | YIYIGNVDIEYNAKFKG (SEQ ID NO: 7) | LCDR2 | SGSTLQS (SEQ ID NO: 10) |
| HCDR3 | GRGLGHFDV (SEQ ID NO: 8) | LCDR3 | QQHNEYPWT (SEQ ID NO: 11) |

Heavy chain variable region sequence of antibody Ab1: 
EVQLVQSGAEVKKPGASVKVSCKASGYTFTNYGINWVRQAPGQRLEWM
GYIYIGNVDIEYNAKFKGRVTITSDTSASTAYMELSSLRSEDTAVYYCARG
RGLGHFDVWGQGTTVTVSS
SEQ ID NO: 12
Light chain variable region sequence of antibody Ab1: 
DIQMTQSPSSLSASVGDRVTITCRASKSISKFLAWFQQKPGKTNKLLIYSGS
TLQSGVPSRFSGSGSGTDFTLTISSLQPEDFATYYCQQHNEYPWTFGGGTK
VEIK
SEQ ID NO: 13
Heavy chain constant region sequence 1: 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQ
GNVFSCSVMHEALHNHYTQKSLSLSPGK
SEQ ID NO: 14
Light chain constant region sequence 1: 



SEQ ID NO: 15
Heavy chain sequence of antibody Ab1: 
EVQLVQSGAEVKKPGASVKVSCKASGYTFTNYGINWVRQAPGQRLEWMGYIYI
GNVDIEYNAKFKGRVTITSDTSASTAYMELSSLRSEDTAVYYCARGRGLGHFDV
WGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSN
TKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVL
 
HQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLT
VDKSRWQQGNVFSCSVMHEALHNHYTQKSLSLSPGK
SEQ ID NO: 16
Light chain sequence of antibody Ab1: 
DIQMTQSPSSLSASVGDRVTITCRASKSISKFLAWFQQKPGKTNKLLIYSGSTLQS
GVPSRFSGSGSGTDFTLTISSLQPEDFATYYCQQHNEYPWTFGGGTKVEIKRTV
AAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNR
GEC
SEQ ID NO: 17
Heavy chain constant region sequence 2: 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKS
CDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQ
GNVFSCSVLHEALHSHYTQKSLSLSPGK
SEQ ID NO: 18
Heavy chain sequence of antibody Ab2: 
EVQLVQSGAEVKKPGASVKVSCKASGYTFTNYGINWVRQAPGQRLEWMGYIYI
GNVDIEYNAKFKGRVTITSDTSASTAYMELSSLRSEDTAVYYCARGRGLGHFDV
WGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSN
TKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVL
HQDWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSKLT
VDKSRWQQGNVFSCSVLHEALHSHYTQKSLSLSPGK
SEQ ID NO: 19
Light chain sequence of antibody Ab2: The same as that of antibody Ab1 (SEQ ID NO: 17).

Note: In the above antibody sequences, the single underlined part is the CDR region sequence, the italicized part is the variable region sequence, and the double underlined part is the antibody constant region sequence.

### Example 1-3. Preparation of antibody

The amino acid sequence of antibody Ab3 is derived from WHO Drug Information Vol. 27, No. 3, 2013. DNA encoding Ab3 was synthesised by Genewiz Inc., (SuZhou). The heavy chain and light chain genes of Ab3 were respectively cloned into the expression vector pcDNA3.4. The expression vectors for the heavy chain and light chain were simultaneously transfected into Expi293F^{™} cells (Thermo Fisher Scientific, Cat. No.: A14527) using PEI (Polyethylenimine) to express the antibody. 293F cells were cultured in a serum-free culture medium for about 5 days. The cell supernatant was collected, and the antibody was purified using Protein A affinity chromatography.

The purification steps are described as follows: Diatomaceous earth was added to the cell culture, and a filter was used to remove cells and impurities to obtain the cell supernatant. A MabSelect Sure (Cytiva, Cat. No.: 17543801) affinity chromatography column was first washed with 0.2 M NaOH, rinsed with pure water, and then equilibrated with PBS. The supernatant was allowed to flow through the affinity column. The affinity column was washed with PBS until the A280 decreased to the baseline. The protein of interest was eluted with 0.1 M acetate buffer at pH 3.5. The antibody solution was neutralized with 1 M Tris-HCl (pH 8.0). The protein solution was dialyzed into PBS by dialysis. The antibody concentration was determined by ultraviolet spectrophotometry, and the antibody was sterilized by filtration and stored in a refrigerator (4°C).

The sequence of antibody Ab3 is as follows:

**Table 3. CDR sequences of antibody Ab3**

| | | | |
|---|---|---|---|
| HCDR1 | SSYIN (SEQ ID NO: 23) | LCDR1 | TGTSSDVGSYNYVN (SEQ ID NO: 26) |
| HCDR2 | TINPVSGSTSYAQKFQG (SEQ ID NO: 24) | LCDR2 | GVSKRPS (SEQ ID NO: 27) |
| HCDR3 | GGWFDY (SEQ ID NO: 25) | LCDR3 | GTFAGGSYYGV (SEQ ID NO: 28) |

Heavy chain variable region sequence of antibody Ab3: 



SEQ ID NO: 29
Light chain variable region sequence of antibody Ab3: 



SEQ ID NO: 30
Heavy chain constant region sequence 3: 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKRVEPKSC
DKTHTCPPCPAPEAAGGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGN
VFSCSVMHEALHNHYTQKSLSLSPGK
SEQ ID NO: 31
Light chain constant region sequence 2: 
GQPKAAPSVTLFPPSSEELQANKATLVCLISDFYPGAVTVAWKADSSPVKA
GVETTTPSKQSNNKYAASSYLSLTPEQWKSHRSYSCQVTHEGSTVEKTVA
PTECS
SEQ ID NO: 32
Heavy chain sequence of antibody Ab3: 
QVQLVQSGAEVKKPGASVKVSCKASGYTFTSSYINWVRQAPGQGLEWMGTINP
VSGSTSYAQKFQGRVTMTRDTSISTAYMELSRLRSDDTAVYYCARGGWFDYWG
QGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKV
DKRVEPKSCDKTHTCPPCPAPEAAGGPSVFLFPPKPKDTLMISRTPEVTCVV
VDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQ
VSLTCLVKGFYPSDIAVEWESNGQPENNYKTTPPVLDSDGSFFLYSKLTVD
KSRWQQGNVFSCSVMHEALHNHYTQKSLSLSPGK
SEQ ID NO: 33
Light chain sequence of antibody Ab3:

QSALTQPASVSGSPGQSITISCTGTSSDVGSYNYVNWYQQHPGKAPKLMIYGVSK
RPSGVSNRFSGSKSGNTASLTISGLQAEDEADYYCGTFAGGSYYGVFGGGTKLT
VLGQPKAAPSVTLFPPSSEELQANKATLVCLISDFYPGAVTVAWKADSSPV
KAGVETTTPSKQSNNKYAASSYLSLTPEQWKSHRSYSCQVTHEGSTVEKT
VAPTECS
SEQ ID NO: 34

Note: In the above antibody full-length sequences, the single underlined part is the antibody CDR region sequence, the italicized part is the variable region sequence, and the double underlined part is the antibody constant region sequence.

### Example 2. Synthesis of polypeptide

The polypeptide compounds and derivatives thereof provided in the present disclosure were obtained by solid-phase synthesis using Rink-amide MBHA (Sunresin New Materials Co. Ltd.) resin as the synthesis support. During the synthesis, the α-amino groups of the amino acid derivatives used were protected by the Fmoc group (fluorenylmethoxycarbonyl), and the side chains of the amino acids were protected with the following protecting groups depending on their functional groups: The thiol group of the cysteine side chain, the amide group of the glutamine side chain, and the imidazole group of the histidine side chain were protected by Trt (trityl); the guanidino group of the arginine side chain was protected by Pbf (2,2,4,6,7-pentamethyldihydrobenzofuran-5-sulfonyl); the indolyl group of the tryptophan side chain and the amino group of the lysine side chain were protected by Boc (tert-butoxycarbonyl); the hydroxyl group of the threonine side chain, the phenol group of the tyrosine side chain, and the hydroxyl group of the serine side chain were protected by t-Bu (tert-butyl); the carboxyl group of the glutamic acid and aspartic acid side chains was protected by OtBu (tert-butyl ester), etc.

The chemical synthesis of the backbone peptide sequences of P1, P2, and P3 was completed using a fluorenylmethoxycarbonyl (Fmoc)/tert-butyl (t-Bu) synthesis method on a Prelude-X fully automated polypeptide synthesiser, wherein Rink-amide MBHA resin with a substitution degree of 0.54 mmol/g was used.

The P1 sequence is:
HAibHGTFTSDYSIαLLEERAAQEFVEWLLAGGGK-NH₂
SEQ ID NO: 20;

The P2 sequence is:
HAibHGTFTSDYSIαLLEEQAAQEFVEWLLAGGGK-NH₂
SEQ ID NO: 21;

The P3 sequence is:
HAibEGTFTSDVSSAibLEEEAAREFVAWLVEGGGK-NH₂
SEQ DI NO: 22.

Before the amino acid condensation step, the Fmoc group was removed using a DMF solution containing 20% 4-methylpiperidine (2 reactions, 8 minutes each). All standard amino acid condensations were performed using equimolar ratios of Fmoc amino acids, HATU (Suzhou Highfine Biotech Co., Ltd.), and 2 equivalents of 4-methylmorpholine, wherein the condensation was performed at room temperature for 25 minutes with a 10-fold excess over the theoretical peptide loading. Exceptions were that when coupling to the C-terminal amino acid of Cα-methylated amino acids such as Aib (2-aminoisobutyric acid) and αL (2-methyl-L-leucine), a second or third condensation was performed for 60 minutes each to ensure complete condensation. After completion of peptide backbone synthesis, the resulting resin-peptide was washed sequentially with DMF and DCM three times each, and dried in vacuum. 10 mL of freshly prepared cleavage solution (trifluoroacetic acid:triisopropylsilane: water = 90: 5: 5, v: v: v) was then added. The mixture was allowed to react at room temperature for 3-4 hours with shaking. After the reaction was completed, the mixture was filtered, and the resin was washed twice with trifluoroacetic acid. The filtrates were combined, and a large amount of methyl tert-butyl ether was added to precipitate a crude peptide solid. The mixture was centrifuged, and the supernatant was discarded to obtain crude P1, P2 and P3 backbone peptides.

The crude peptide was dissolved in a mixed solvent containing 20% acetic acid/water. The resulting solution was filtered through a 0.22 µM membrane, and then separated using a WATERS Prep150 LC reversed-phase high-performance liquid chromatography system with mobile phases A (0.1% trifluoroacetic acid and 10% acetonitrile in water) and B (0.1% trifluoroacetic acid and 90% acetonitrile in water). The chromatography column was an X-SELECT OBD C-18 (WATERS) reversed-phase chromatography column. During purification, the detection wavelength of the chromatograph was set to 220 nm, and the flow rate was 15 mL/min. Product-related fractions were collected and lyophilised to obtain pure P1, P2 and P3 backbone peptides, with a yield of 20%. The purity and the compound identity of the pure backbone peptides were determined by analytical ultra-high performance liquid chromatography and liquid chromatography/mass spectrometry, and the pure backbone peptides were used in the next reaction.

### Example 3. Chemical synthesis of polypeptide containing DBCO-PEG8 modification at C-terminus

### 1. Synthesis of LP1

### (1) Synthesis of activated intermediate 2

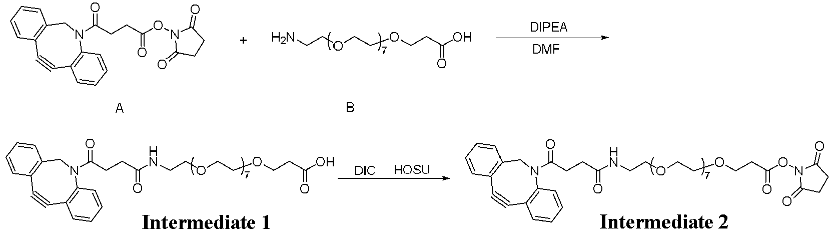

177.7 mg (0.44 mmol) of dibenzocyclooctyne-N-hydroxysuccinimidyl ester (compound A, Leyan) and 195 mg (0.44 mmol) of 1-amino-3,6,9,12,15,18,21,24-octaoxaheptacosan-27-oic acid (compound B, Bide Pharmatech Ltd.) were dissolved in N,N-dimethylformamide (2.5 mL), and 285 mg of N,N-diisopropylethylamine (2.2051 mmol, 384 µL) was added. The mixture was purged with nitrogen three times, and stirred at room temperature for 1 hour. The mixture was filtered, and purified by high-performance liquid chromatography. The corresponding sample was collected, rotary evaporated at 30°C, and dried in vacuum with an oil pump to obtain intermediate 1 (260 mg, yield: 80.8%).

200.92 mg (0.28 mmol) of intermediate 1 and 34.9 mg (0.30 mmol) of N-hydroxysuccinimide (HOSU) were dissolved in 2 mL of anhydrous THF/DMF (v:v = 3:1); under stirring, 47 µL (0.30 mmol) of N,N'-diisopropylcarbodiimide (DIC) was slowly added to the reaction system; and the mixture was reacted at room temperature for 12 hours or more. The reaction process was monitored by analytical ultra-high performance liquid chromatography. The conversion rate of the product intermediate 2 was 77%. The resulting intermediate 2 was directly used in the next reaction without purification.

### (2) P1 polypeptide conjugation modification

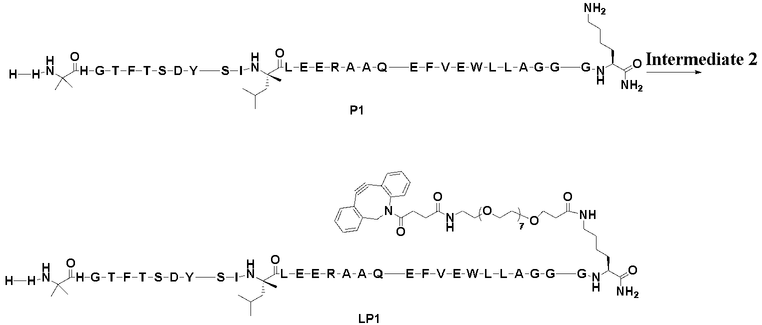

90 mg (0.025 mmol) of P1 backbone peptide (see Example 2 for synthesis and purification methods) was dissolved in 4 mL of a sodium carbonate solution (pH 10.8) and 2 mL of acetonitrile (final concentration not less than 15 mg/mL); after 10 minutes of stirring (the pH of the reaction system dropped as measured by a pH meter), the pH of the reaction system was adjusted to 10.8 using a sodium hydroxide solution, and after 10 minutes of further stirring, the pH was monitored to remain unchanged; subsequently, under stirring, 358 µL of the intermediate 2 reaction system (actually containing 31.3 mg of intermediate 2 (0.038 mmol)) was slowly added dropwise; after 20 minutes of further reaction, monitoring by ultra-high performance liquid chromatography indicated that the reaction was complete. The reaction system was adjusted to pH 3-4 with trifluoroacetic acid, and then purified using preparative high-performance liquid chromatography. Product-related fractions were collected and lyophilised to obtain a pure C-terminally modified polypeptide LP1. The purity and the compound molecular weight of the pure LP1 were determined by analytical ultra-high performance liquid chromatography and liquid chromatography/mass spectrometry, wherein the purity was 95.45%, and the found molecular weight was: 4271.17.

### 2. Synthesis of LP2

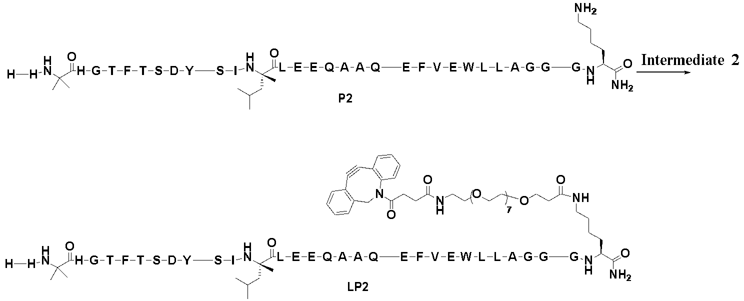

90 mg (0.025 mmol) of P2 backbone peptide (see Example 2 for synthesis and purification methods) was dissolved in 4 mL of a sodium carbonate solution (pH 10.8) and 2 mL of acetonitrile (final concentration not less than 15 mg/mL); after 10 minutes of stirring (the pH of the reaction system dropped as measured by a pH meter), the pH of the reaction system was adjusted to 10.8 using a sodium hydroxide solution, and after 10 minutes of further stirring, the pH was monitored to remain unchanged; subsequently, under stirring, 361 µL of the intermediate 2 reaction system (actually containing 31.6 mg of intermediate 2 (0.038 mmol)) was slowly added dropwise; after 20 minutes of further reaction, monitoring by ultra-high performance liquid chromatography indicated that the reaction was complete. The reaction system was adjusted to pH 3-4 with trifluoroacetic acid, and then purified using preparative high-performance liquid chromatography. Product-related fractions were then collected and lyophilised to obtain a pure C-terminally modified polypeptide LP2. The purity and the compound molecular weight of the pure LP2 were determined by analytical ultra-high performance liquid chromatography and liquid chromatography/mass spectrometry, wherein the purity was 97.56%, and the found molecular weight was: 4243.13.

### 3. Synthesis of LP3

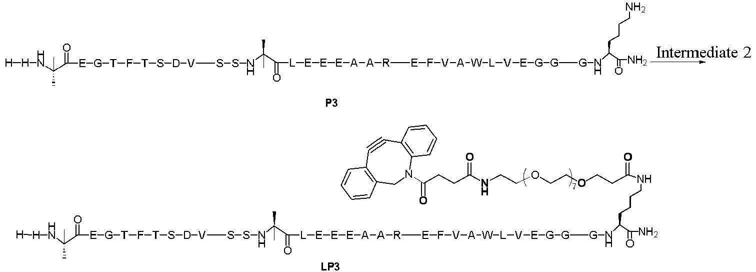

90 mg (0.026 mmol) of P3 backbone peptide (see Example 2 for synthesis and purification methods) was dissolved in 4 mL of a sodium carbonate solution (pH 10.8) and 2 mL of acetonitrile (final concentration not lower than 15 mg/mL); after 10 minutes of stirring (the pH of the reaction system dropped as measured by a pH meter), the pH of the reaction system was adjusted to 10.8 using a sodium hydroxide solution, and after 10 minutes of further stirring, the pH was monitored to remain unchanged; subsequently, under stirring, 324 µL of the intermediate 2 reaction system (actually containing 28.4 mg of intermediate 2 (0.034 mmol)) was slowly added dropwise; after 20 minutes of further reaction, monitoring by ultra-high performance liquid chromatography indicated that the reaction was complete. The reaction system was adjusted to pH 3-4 with trifluoroacetic acid, and then purified using preparative high-performance liquid chromatography. Product-related fractions were then collected and lyophilised to obtain a pure C-terminally modified polypeptide LP3. The purity and the compound molecular weight of the pure LP3 were determined by analytical ultra-high performance liquid chromatography and liquid chromatography/mass spectrometry, wherein the purity was 96.00%, and the found molecular weight was: 4117.99.

### Example 4. Synthesis of glycan chain OLS-4

### (2S,3S,4S,5R,6R)-4-(2-(3-(2-(2-Azidoethoxy)ethoxy)propoxy)ethoxy)-2-(((3aR,5R,6S,7R,7aR)-7-hydroxy-5-(hydroxymethyl)-2-methyl-5,6,7,7a-tetrahydro-3aH-pyrano[3,2-d]oxazol-6-yl)oxy)-6-(hydroxymethyl)tetrahydro-2H-pyran-3,5-diol OLS-4

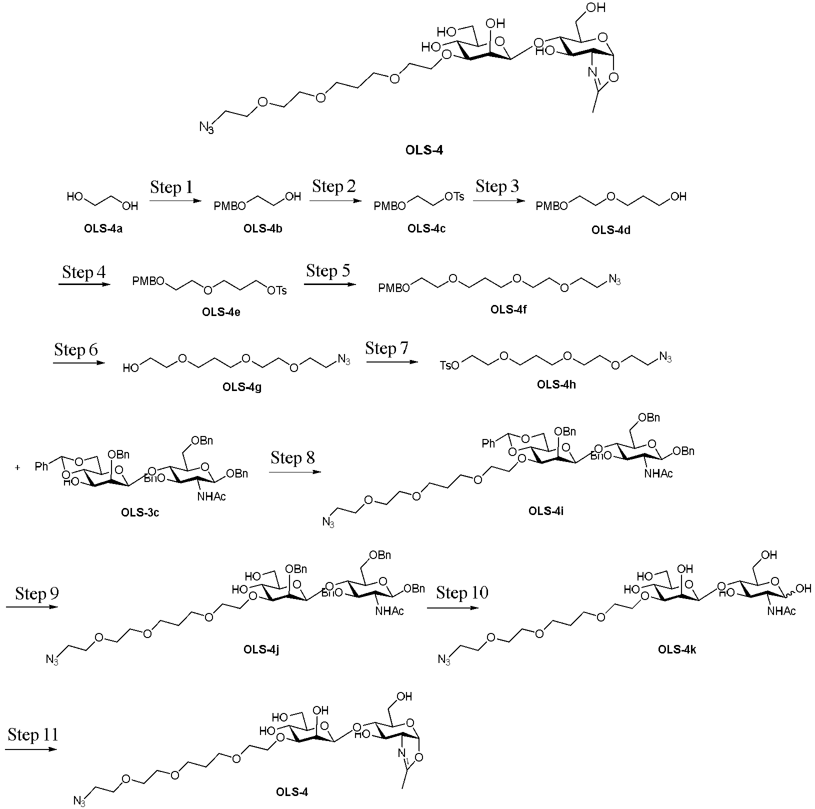

### Step 1

### 2-((4-Methoxybenzyl)oxy)ethan-1-ol OLS-4b

Ethylene glycol OLS-4a (50.00 g, 805.57 mmol) was dissolved in anhydrous tetrahydrofuran (300 mL), and sodium hydride (4.93 g, 128.89 mmol, 60% content) and tetrabutylammonium iodide (5.37 g, 16.11 mmol) were added in sequence. Under nitrogen protection, the mixture was stirred and left to react at 0°C for 30 minutes. p-Methoxybenzyl chloride (20.19 g, 128.89 mmol) was then slowly added dropwise. The dropwise addition was completed in about 30 minutes. The mixture was warmed to 80°C and stirred and left to react for 16 hours. The reaction solution was poured into ice water and quenched with stirring. Ethyl acetate (500 mL) was added, and the layers were separated. The resulting aqueous phase was extracted with ethyl acetate (150 mL × 3). The organic phases were combined. The organic phase was washed with water (250 mL × 3) and a saturated sodium chloride solution (250 mL × 2), dried over anhydrous sodium sulfate, and filtered to remove the drying agent. The filtrate was concentrated under reduced pressure. The resulting residue was purified by silica gel column chromatography with eluent system C to obtain the title product OLS-4b (22.00 g, yield: 14.9%).

MS m/z (ESI): 205.2 [M+23].

### Step 2

### 2-((4-Methoxybenzyl)oxy)ethyl 4-methylbenzenesulfonate OLS-4c

OLS-4b (22.00 g, 120.74 mmol) was dissolved in pyridine (98 mL), and p-toluenesulfonyl chloride (27.62 g, 144.88 mmol) was added at 0°C. Under nitrogen protection, the mixture was stirred and left to react at 0°C, slowly warmed to room temperature, and left to react for 16 hours. The reaction solution was diluted by the addition of ethyl acetate (300 mL), and washed with hydrochloric acid (10%, 150 mL × 3). The organic phase was washed with water (150 mL × 3) and a saturated sodium chloride solution (250 mL × 2) in sequence, dried over anhydrous sodium sulfate for 30 minutes, and filtered to remove the drying agent. The filtrate was concentrated under reduced pressure. The resulting residue was purified by silica gel column chromatography with eluent system C to obtain the title product OLS-4c (36.40 g, yield: 89.6%).

¹H NMR (400 MHz, CDCl₃) δ 7.81 (d, 2H), 7.33 (d, 2H), 7.21 (d, 2H), 6.88 (d, 2H), 4.43 (s, 2H), 4.23-4.17 (m, 2H), 3.82 (s, 3H), 3.68-3.61 (m, 2H), 2.45 (s, 3H).

### Step 3

### 3-(2-((4-Methoxybenzyl)oxy)ethoxy)propan-1-ol OLS-4d

OLS-4c (36.40 g, 108.20 mmol) and 1,3-propanediol (82.34 g, 1.08 mol) were dissolved in toluene (100 mL), and powdered potassium hydroxide (15.18 g, 270.51 mmol) was added at room temperature. The mixture was heated to 50°C under nitrogen protection, and stirred and left to react for 72 hours. The reaction solution was poured into ice water (200 mL), and concentrated hydrochloric acid was added dropwise until the pH of the reaction solution was 3-4. Ethyl acetate (300 mL) was added for extraction, and the layers were separated. The aqueous phase was extracted with ethyl acetate (100 mL × 3). The organic phases were combined, dried over anhydrous sodium sulfate for 30 minutes, and filtered to remove the drying agent. The filtrate was concentrated under reduced pressure. The resulting residue was purified by silica gel column chromatography with eluent system C to obtain the title product OLS-4d (15.60 g, yield: 60.0%).

¹H NMR (400 MHz, CDCl₃) δ 7.28 (d, 2H), 6.89 (d, 2H), 4.51 (s, 2H), 3.81 (s, 3H), 3.79 (t, 2H), 3.68 (t, 2H), 3.65-3.59 (m, 4H), 2.5i1 (br.s, 1H), 1.88-1.82 (m, 2H).

### Step 4

### 3-(2-((4-Methoxybenzyl)oxy)ethoxy)propyl 4-methylbenzenesulfonate OLS-4e

OLS-4d (15.60 g, 64.92 mmol) was dissolved in a mixed solution of pyridine (52 mL) and dichloromethane (52 mL), and p-toluenesulfonyl chloride (18.56 g, 97.38 mmol) was added at 0°C. Under nitrogen protection, the mixture was stirred and left to react at 0°C, slowly warmed to room temperature, and left to react for 16 hours. The reaction solution was diluted by the addition of ethyl acetate (300 mL), washed with hydrochloric acid (10%, 150 mL × 3), water (150 mL × 3) and a saturated sodium chloride solution (250 mL × 2) in sequence, dried over anhydrous sodium sulfate for 30 minutes, and filtered to remove the drying agent. The filtrate was concentrated under reduced pressure. The resulting residue was purified by silica gel column chromatography with eluent system C to obtain the title product OLS-4e (18.00 g, yield: 70.3%).

MS m/z (ESI): 417.3 [M+23].

¹H NMR (400 MHz, CDCl₃) δ 7.80 (d, 2H), 7.34 (d, 2H), 7.27 (d, 2H), 6.90 (d, 2H), 4.48 (s, 2H), 4.16 (t, 2H), 3.83 (s, 3H), 3.53-3.49 (m, 6H), 2.45 (s, 3H), 1.96-1.91 (m, 2H).

### Step 5

### 14-Azido-1-(4-methoxyphenyl)-2,5,9,12-tetraoxatetradecane OLS-4f

OLS-4e (8.00 g, 20.28 mmol) and azido-diethylene glycol (3.72 g , 28.39 mol, Amatek) were dissolved in toluene (33 mL), and powdered potassium hydroxide (2.84 g, 50.70 mmol) was added at room temperature. The mixture was heated to 60°C under nitrogen protection, and stirred and left to react for 16 hours. The reaction solution was poured into ice water (100 mL), and concentrated hydrochloric acid was added dropwise until the pH of the reaction solution was 3-4. Ethyl acetate (100 mL) was added for extraction, and the layers were separated. The aqueous phase was extracted with ethyl acetate (50 mL × 3). The organic phases were combined, dried over anhydrous sodium sulfate for 30 minutes, and filtered to remove the drying agent. The filtrate was concentrated under reduced pressure. The resulting residue was purified by silica gel column chromatography with eluent system C to obtain the title product OLS-4f (6.3 g, yield: 87.9%).

MS m/z (ESI): 376.3 [M+23].

¹H NMR (400 MHz, CDCl₃) δ 7.29 (d, 2H), 6.89 (d, 2H), 4.52 (s, 2H), 3.82 (s, 3H), 3.70-3.56 (m, 14H), 3.40 (t, 2H), 1.93-1.87 (m, 2H).

### Step 6

### 2-(3-(2-(2-Azidoethoxy)ethoxy)propoxy)ethan-1-ol OLS-4g

OLS-4f (6.30 g, 17.83 mmol) was dissolved in dichloromethane (31 mL) and water (3.1 mL), and dichlorodicyanobenzoquinone (4.45 g, 19.61 mmol, Aladdin) was added at room temperature. Under nitrogen protection, the mixture was stirred and left to react at 25°C for 1.5 hours. The reaction solution was filtered with diatomaceous earth. The filtrate was diluted with dichloromethane (150 mL), washed with a saturated sodium bicarbonate solution (100 mL × 2), a saturated sodium bisulfite solution (100 mL × 2), water (100 mL), and a saturated sodium chloride solution (100 mL × 2) in sequence, dried over anhydrous sodium sulfate for 30 minutes, and subjected to suction filtration. The filtrate was concentrated under reduced pressure. The resulting residue was purified by silica gel column chromatography with eluent system C to obtain the title product OLS-4g (3.2 g, yield: 76.9%).

### Step 7

### 2-(3-(2-(2-Azidoethoxy)ethoxy)propoxy)ethyl 4-methylbenzenesulfonate OLS-4h

OLS-4g (3.20 g, 13.72 mmol) was dissolved in pyridine (11 mL) and dichloromethane (11 mL) and cooled to 0°C. p-Toluenesulfonyl chloride (3.14 g, 16.46 mmol) was added. Under nitrogen protection, the mixture was stirred and left to react at 0°C, slowly warmed to room temperature, and left to react for 16 hours. The reaction solution was diluted by the addition of ethyl acetate (200 mL), washed with hydrochloric acid (10%, 150 mL × 3), water (150 mL × 3) and a saturated sodium chloride solution (250 mL × 2) in sequence, dried over anhydrous sodium sulfate for 30 minutes, and filtered to remove the drying agent. The filtrate was concentrated under reduced pressure. The resulting residue was purified by silica gel column chromatography with eluent system C to obtain the title product OLS-4h (4.80 g, yield: 90.3%).

¹H NMR (400 MHz, CDCl₃) δ 7.79 (d, 2H), 7.34 (d, 2H), 4.14 (t, 2H), 3.67-3.57 (m, 8H), 3.51-3.46 (m, 4H), 3.38 (t, 2H), 2.44 (s, 3H), 1.81-1.75 (m, 2H).

### Step 8

### N-((2R,3R,4R,5S,6R)-5-(((2R,4aR,6S,7S,8S,8aR)-8-(2-(3-(2-(2-Azidoethoxy)ethoxy)propoxy)ethoxy)-7-(benzyloxy)-2-phenylhexahydropyrano[3,2-d][1,3]dioxan-6-yl)oxy)-2,4-bis(benzyloxy)-6-((benzyloxy)methyl)tetrahydro-2H-pyran-3-yl)acetamide OLS-4i

OLS-3c (340 mg, 0.41 mmol), N,N-dimethylformamide (4.8 mL) and 2 mL of a solution of OLS-4h (398 mg, 1.03 mmol) in N,Ndimethylformamide were added to a reaction flask, and cooled to 0°C. Sodium hydride (86 mg, 2.15 mmol, 60% content) was added. The mixture was left to react at 0°C for about 0.5 hours, and then warmed to 20°C-30°C and left to react for about 0.5 hours. The reaction solution was poured into a mixed solution of a saturated ammonium chloride solution (10 mL) and water (10 mL), and extracted with ethyl acetate (10 mL × 3). The organic phases were combined, washed with water (10 mL) and a saturated sodium chloride solution (10 mL) in sequence, dried over anhydrous sodium sulfate, and filtered to remove the drying agent. The filtrate was concentrated under reduced pressure. The resulting residue was purified by thin layer chromatography with developing agent system C to obtain the title product OLS-4i (362 mg, yield: 84.6%).

MS m/z (ESI): 1047.4 [M+1].

¹H NMR (400 MHz, CDCl₃) δ 7.40-7.16 (m, 25H), 5.70 (d, 1H), 5.44 (s, 1H), 4.87 (d, 1H), 4.84-4.78 (m, 3H), 4.71-4.68 (m, 1H), 4.57-4.50 (m, 4H), 4.41-4.38 (m, 1H), 4.04-4.01 (m, 1H), 3.97 (t, 1H), 3.93-3.84 (m, 2H), 3.76-3.68 (m, 3H), 3.61-3.40 (m, 16H), 3.34-3.27 (m, 3H), 3.11-3.05 (m, 2H), 1.78-1.72 (m, 2H), 1.51 (s, 3H).

### Step 9

### N-((2R,3R,4R,5S,6R)-5-(((2S,3S,4S,5R,6R)-4-(2-(3-(2-(2-Azidoethoxy)ethoxy)propoxy)ethoxy)-3-(benzyloxy)-5-hydroxy-6-(hydroxymethyl)tetrahydro-2H-pyran-2-yl)oxy)-2,4-bis(benzyloxy)-6-((benzyloxy)methyl)tetrahydro-2H-pyran-3-yl)acetamide OLS-4j

Compound OLS-4i (360 mg, 0.34 mmol) and dichloromethane (26 mL) were added to a reaction flask in sequence. Under nitrogen protection, the temperature was lowered to -30°C to -20°C, and trifluoroacetic acid (3.6 mL) was added dropwise. After the dropwise addition was completed, the mixture was warmed to -5°C to 5°C and stirred and left to react for about 1.5 hours. The reaction was quenched by the addition of methanol (5.1 mL), and dichloromethane (12.3 mL) was added for dilution. The mixture was washed with a saturated sodium bicarbonate solution (26 mL × 2) and a saturated sodium chloride solution (13 mL) in sequence, dried over anhydrous sodium sulfate, and filtered to remove the drying agent. The filtrate was concentrated under reduced pressure. The resulting residue was purified by thin layer chromatography with developing agent system C to obtain the title product OLS-4j (234 mg, yield: 71.0%).

MS m/z (ESI): 959.7[M+1].

¹H NMR (400 MHz, CDCl₃) δ 7.31-7.17 (m, 20H), 5.64 (d, 1H), 4.87 (d, 1H), 4.84-4.79 (m, 2H), 4.70 (s, 2H), 4.58-4.50 (m, 3H), 4.43-4.39 (m, 2H), 4.05 (t, 1H), 3.82 (t, 1H), 3.74-3.36 (m, 23H), 3.30 (t, 2H), 3.10-3.07 (m, 1H), 3.00-2.97 (m, 1H), 2.21 (br.s, 1H), 1.81-1.75 (m, 2H), 1.66 (s, 3H).

### Step 10

### N-((3R,4R,5S,6R)-5-(((2S,3S,4S,5R,6R)-4-(2-(3-(2-(2-Azidoethoxy)ethoxy)propoxy)ethoxy)-3,5-dihydroxy-6-(hydroxymethyl)tetrahydro-2H-pyran-2-yl)oxy)-2,4-dihydroxy-6-(hydroxymethyl)tetrahydro-2H-pyran-3-yl)acetamide OLS-4k (a mixture of diastereomers)

OLS-4j (150 mg, 158.72 µmol) was dissolved in tetrahydrofuran (8 mL) and water (2 mL), and palladium hydroxide (50.0 mg, 15% content), palladium on carbon (61 mg, 10% content), and hydrochloric acid (1 drop) were added. The mixture was purged with hydrogen three times and stirred and left to react at room temperature for 12 hours. The catalyst was removed by filtration. The filtrate was concentrated under reduced pressure, and the resulting residue was dissolved in methanol (7 mL). Imidazole-1-sulfonyl azide hydrochloride (6 mg, 220.41 µmol), potassium carbonate (68 mg, 492.00 µmol), and copper sulfate (5.7 mg, 22.83 µmol) were added, and the mixture was stirred and left to react at room temperature for 12 hours. The inorganic salts were removed by filtration, and the solids were rinsed with methanol (3 mL). The organic phases were combined, and the filtrate was concentrated under reduced pressure to obtain the crude title product OLS-4k (161 mg). The product was directly used in the next step without purification.

MS m/z (ESI): 599.3 [M+1].

### Step 11

### (2S,3S,4S,5R,6R)-4-(2-(3-(2-(2-Azidoethoxy)ethoxy)propoxy)ethoxy)-2-(((3aR,5R,6S,7R,7aR)-7-hydroxy-5-(hydroxymethyl)-2-methyl-5,6,7,7a-tetrahydro-3aH-pyrano[3,2-d]oxazol-6-yl)oxy)-6-(hydroxymethyl)tetrahydro-2H-pyran-3,5-diol OLS-4

In an ice bath, the crude compound OLS-4k (161 mg, 148.01 µmol) was dissolved in purified water (8 mL), and 2-chloro-1,3-dimethyl-1*H*-benzimidazol-3-chloride (557 mg, 3.07 mmol) and caesium carbonate (3.00 g, 9.20 mmol) were added. The mixture was stirred and left to react at 0°C for 12 hours. The reaction solution was purified by high-performance liquid chromatography (chromatography column: Phenomenex C18, 150 × 25 mm, 10 µm; mobile phase: water (0.01% ammonia water) and acetonitrile, with gradient ratio: acetonitrile 0%-30%, flow rate: 25 mL/min) and lyophilised in a 5 mol% sodium hydroxide solution to obtain the title product OLS-4 (35 mg, total yield of step 10 and step 11: 38.2%).

MS m/z (ESI): 581.3 [M+1].

¹H NMR (400 MHz, CDCl₃) δ 6.02 (d, 1H), 4.65 (s, 2H), 4.30 (s, 2H), 4.09-3.93(m, 5H), 3.86-3.77 (m, 4H), 3.73-3.51 (m, 16H), 3.40-3.28 (m, 5H), 2.01 (s, 3H), 1.88-1.81 (m, 2H).

### Example 5-1. APC-1

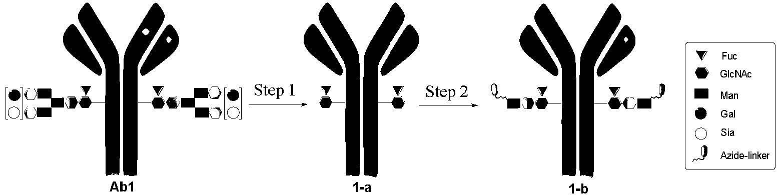

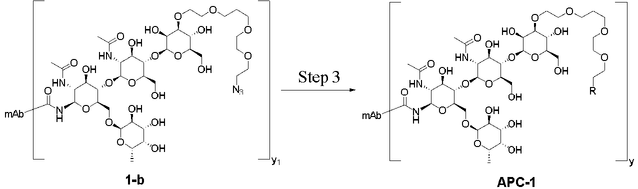

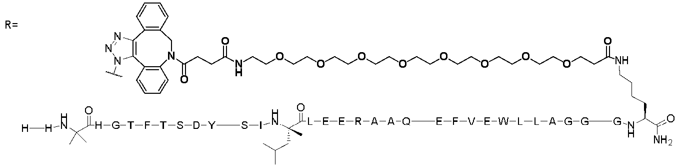

or

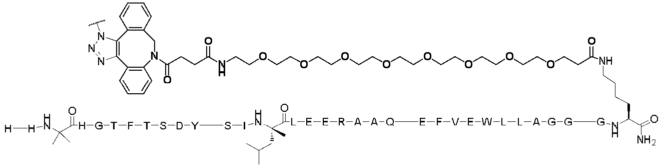

(The triazole ring formed in this step has a geometric structure, and the obtained compound comprises the two structures shown as R above.)

### Step 1

Endo S enzyme (19.16 mg/mL, 0.104 mL) was added to antibody **Ab1** in an aqueous PBS buffer solution (a pH 6.5, 0.05 M aqueous PBS buffer solution; 10 mg/mL, 20 mL) at 37°C. The mixture was shaken on a water bath shaker and left to react at 37°C for 12 hours, and then the reaction was stopped. The reaction solution was purified using a Hitrap Protein A HP protein purification column (elution phase: a pH 3.0 aqueous acetate buffer solution) to obtain an affinity eluate of 1-a, which was buffer-exchanged into a PBS buffer at pH 7.4 and refrigerated and stored at 4°C.

### Step 2

Endo S enzyme (19.16 mg/mL, 0.104 mL) and OLS-4 (23.2 mg) were added to 1-a in an aqueous PBS buffer solution (a pH 6.5, 0.05 M aqueous PBS buffer solution; 10.0 mg/mL, 20 mL) at 37°C. The mixture was shaken on a water bath shaker and left to react at 37°C for 1 hour, and then the reaction was stopped. The reaction solution was purified using a Hitrap Protein A HP protein purification column (elution phase: a pH 3.0 aqueous acetate buffer solution) to obtain an affinity eluate of 1-b, which was buffer-exchanged into an aqueous PBS buffer solution at pH 7.4 and refrigerated and stored at 4°C. y¹ represents the average number of glycan chains remodeled at the N297 positions of the heavy chains of the antibody; two glycan chains or one glycan chain may be remodeled at the two N297 positions of the heavy chains of the antibody.

### Step 3

At 25°C, 1-b in an aqueous PBS buffer solution (a pH 7.4 aqueous PBS buffer solution; 8.0 mg/mL, 10.82 mL) was taken, and then 5.0 mL of 1,2-propanediol was added thereto. After the mixture was shaken uniformly, an LP1 solution (6.83 mg, 1599 nmol, dissolved in a mixed solvent of 625 µL dimethyl sulfoxide and 625 µL 1,2-propanediol) was added; the mixture was left to react on a shaker at room temperature for 12 hours. The reaction solution was centrifuged, and the supernatant was diluted with 50 mL of a PBS buffer solution at pH 7.4 and then purified using a Hitrap Protein A HP protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-1, which was refrigerated and stored at 4°C.

The average number of conjugated polypeptides per antibody molecule was calculated by MS: y=1.96.

### Example 5-2. APC-2

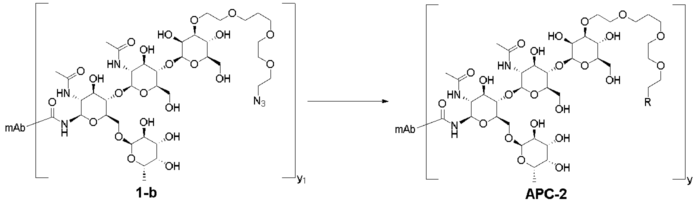

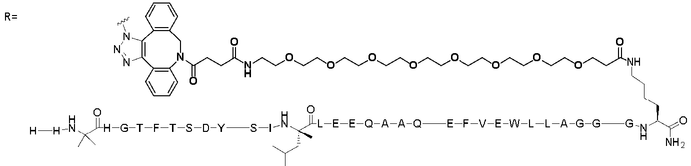

or

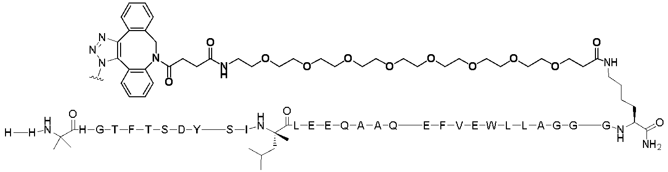

(The triazole ring formed in this step has a geometric structure, and the obtained compound comprises the two structures shown as R above.)

At 25°C, 1-b in an aqueous PBS buffer solution (a pH 7.4 aqueous PBS buffer solution; 8.0 mg/mL, 10.82 mL) was taken, and then 9.0 mL of 1,2-propanediol was added thereto. After the mixture was shaken uniformly, an LP2 solution (12.4 mg, 2922 nmol, dissolved in a mixed solvent of 1125 µL dimethyl sulfoxide and 1125 µL 1,2-propanediol) was added; the mixture was left to react on a shaker at room temperature for 12 hours. The reaction solution was centrifuged, and the supernatant was diluted with 50 mL of a PBS buffer solution at pH 7.4 and then purified using a Hitrap Protein A HP protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-2, which was refrigerated and stored at 4°C.

The average number of conjugated polypeptides per antibody molecule was calculated by MS: y=1.72.

### Example 5-3. APC-3

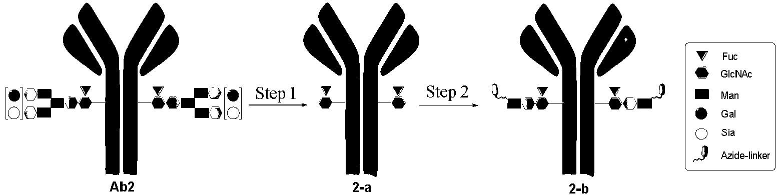

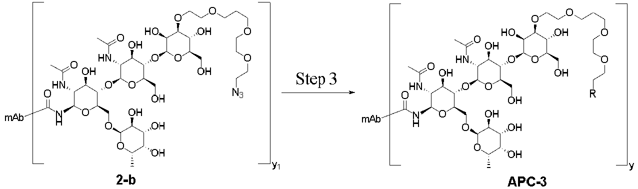

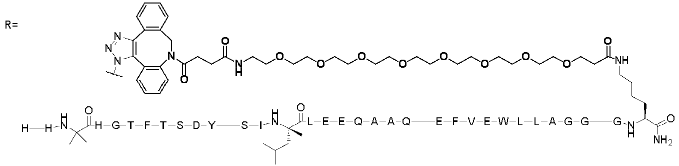

or

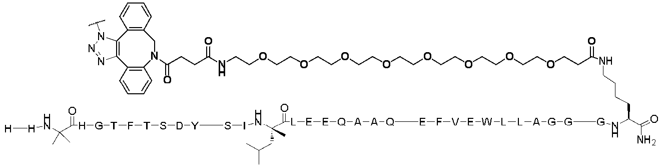

(The triazole ring formed in this step has a geometric structure, and the obtained compound comprises the two structures shown as R above.)

### Step 1

Endo S enzyme (19.16 mg/mL, 0.104 mL) was added to antibody Ab2 in an aqueous PBS buffer solution (a pH 6.5, 0.05 M aqueous PBS buffer solution; 10 mg/mL, 20 mL) at 37°C. The mixture was shaken on a water bath shaker and left to react at 37°C for 12 hours, and then the reaction was stopped. The reaction solution was purified using a Hitrap Protein A HP protein purification column (elution phase: a pH 3.0 aqueous acetate buffer solution) to obtain an affinity eluate of 2-a, which was buffer-exchanged into a PBS buffer at pH 7.4 and refrigerated and stored at 4°C.

### Step 2

Endo S enzyme (19.16 mg/mL, 0.078 mL) and OLS-4 (17.4 mg) were added to 2-a in an aqueous PBS buffer solution (a pH 6.5, 0.05 M aqueous PBS buffer solution; 10.0 mg/mL, 15 mL) at 37°C. The mixture was shaken on a water bath shaker and left to react at 37°C for 1 hour, and then the reaction was stopped. The reaction solution was purified using a Hitrap Protein A HP protein purification column (elution phase: a pH 3.0 aqueous acetate buffer solution) to obtain an affinity eluate of 2-b, which was buffer-exchanged into an aqueous PBS buffer solution at pH 7.4 and refrigerated and stored at 4°C. y¹ represents the average number of glycan chains remodeled at the N297 positions of the heavy chains of the antibody; two glycan chains or one glycan chain may be remodeled at the two N297 positions of the heavy chains of the antibody.

### Step 3

At 25°C, 2-b in an aqueous PBS buffer solution (a pH 7.4 aqueous PBS buffer solution; 8.0 mg/mL, 18.25 mL) was taken, and then 14.6 mL of 1,2-propanediol was added thereto. After the mixture was shaken uniformly, an LP2 solution (20.65 mg, 4866 nmol, dissolved in a mixed solvent of 1825 µL dimethyl sulfoxide and 1825 µL 1,2-propanediol) was added; the mixture was left to react on a shaker at room temperature for 12 hours. The reaction solution was centrifuged, and the supernatant was diluted with 50 mL of a PBS buffer solution at pH 7.4 and then purified using a Hitrap Protein A HP protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-3, which was refrigerated and stored at 4°C.

The average number of conjugated polypeptides per antibody molecule was calculated by MS: y=1.89.

### Example 5-4. APC-4

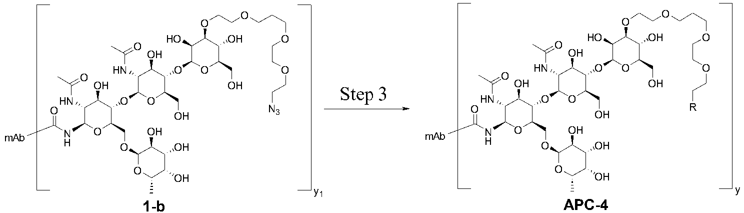

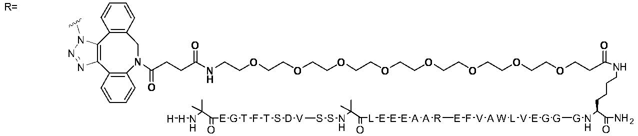

or

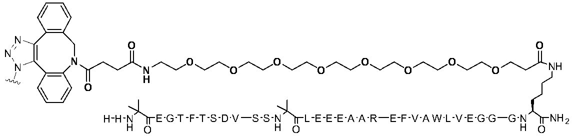

(The triazole ring formed in step 3 has a geometric structure, and the obtained compound comprises the two structures shown as R above.)
5 mL of a solution of crude 1-b was taken, and then 4 mL of 1,2-propanediol was added thereto. After the mixture was shaken uniformly, an LP-3 solution (5.56 mg, 1350 nmol, dissolved in a mixed solvent of 500 µL dimethyl sulfoxide and 500 µL 1,2-propanediol) was added; the mixture was left to react on a shaker at room temperature for 12 hours. The reaction solution was centrifuged, and the supernatant was diluted with 50 mL of a PBS 7.4 buffer solution and then purified using a MabSelect protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-4, which was refrigerated and stored at 4°C.

The average number of conjugated drugs per antibody molecule was calculated by MS: y=1.94.

### Example 5-5. APC-5

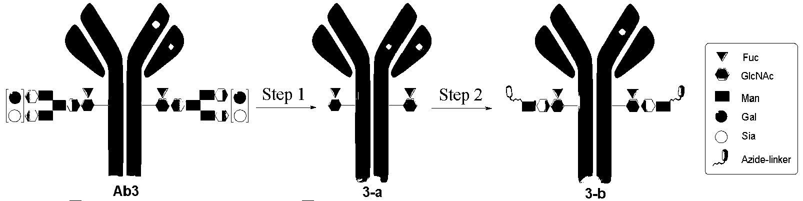

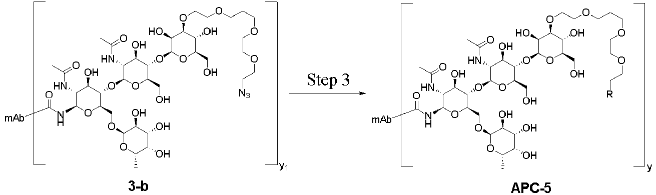

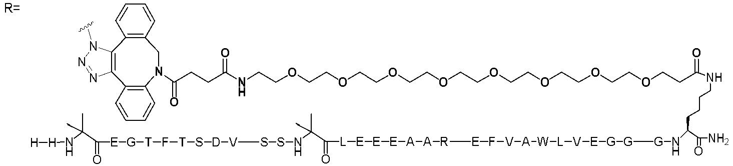

or

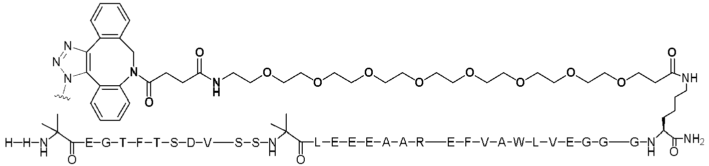

(The triazole ring formed in this step has a geometric structure, and the obtained compound comprises the two structures shown as R above.)

### Step 1

Endo S enzyme (5.06 mg/mL, 0.061 mL) was added to antibody Ab3 in an aqueous PBS buffer solution (a pH 6.5, 0.05 M aqueous PBS buffer solution; 10 mg/mL, 3.1 mL) at 37°C. The mixture was shaken on a water bath shaker and left to react at 37°C for 12 hours, and then the reaction was stopped. The reaction solution was purified using a MabSlect protein purification column (elution phase: a pH 3.0 aqueous acetate buffer solution) to obtain an affinity eluate of 3-a, which was buffer-exchanged into a PBS buffer at pH 6.5 and refrigerated and stored at 4°C.

### Step 2

Endo S enzyme (5.06 mg/mL, 0.061 mL) and OLS-4 (3.72 mg) were added to 3-a in an aqueous PBS buffer solution (a pH 6.5, 0.05 M aqueous PBS buffer solution; 10.0 mg/mL, 3.1 mL) at 37°C. The mixture was shaken on a water bath shaker and left to react at 37°C for 1.5 hours, and then the reaction was stopped. The reaction solution was purified using a MabSlect protein purification column (elution phase: a pH 3.0 aqueous acetate buffer solution) to obtain an affinity eluate of 3-b, which was buffer-exchanged into an aqueous PBS buffer solution at pH 7.4 and refrigerated and stored at 4°C. y¹ represents the average number of glycan chains remodeled at the N297 positions of the heavy chains of the antibody; two glycan chains or one glycan chain may be remodeled at the two N297 positions of the heavy chains of the antibody.

### Step 3

At 25°C, 3-b in an aqueous PBS buffer solution (a pH 7.4 aqueous PBS buffer solution; 8.0 mg/mL, 0.875 mL) was taken, and then 700 µL of 1,2-propanediol was added thereto. After the mixture was shaken uniformly, an LP3 solution (0.93 mg, 226 nmol, dissolved in a mixed solvent of 87.5 µL dimethyl sulfoxide and 87.5 µL 1,2-propanediol) was added; the mixture was left to react on a shaker at room temperature for 12 hours. The reaction solution was centrifuged, and the supernatant was diluted with 10 mL of a PBS buffer solution at pH 7.4 and then purified using a MabSlect protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-5, which was refrigerated and stored at 4°C.

The average number of conjugated polypeptides per antibody molecule was calculated by MS: y=1.93.

### Example 5-6. APC-6

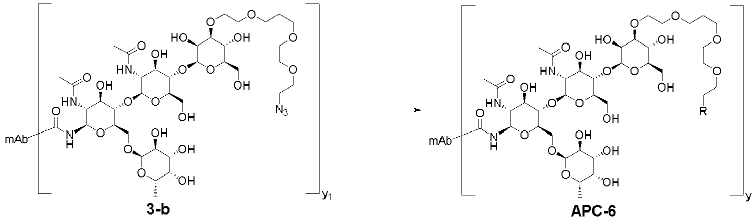

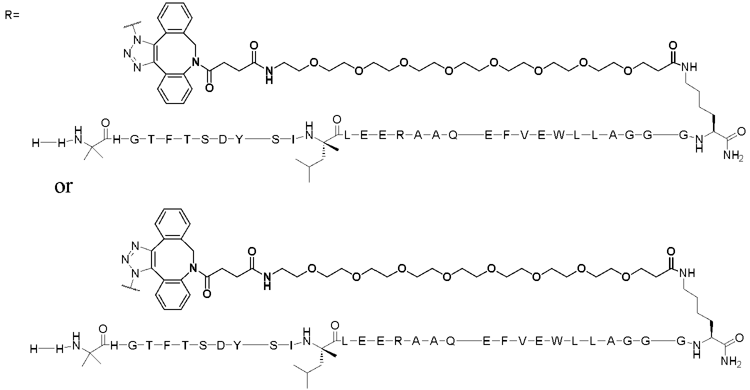

(The triazole ring formed in this step has a geometric structure, and the obtained compound comprises the two structures shown as R above.)
At 25°C, 3-b in an aqueous PBS buffer solution (a pH 7.4 aqueous PBS buffer solution; 8.0 mg/mL, 0.875 mL) was taken, and then 700 µL of 1,2-propanediol was added thereto. After the mixture was shaken uniformly, an LP2 solution (0.96 mg, 226 nmol, dissolved in a mixed solvent of 87.5 µL dimethyl sulfoxide and 87.5 µL 1,2-propanediol) was added; the mixture was left to react on a shaker at room temperature for 12 hours. The reaction solution was centrifuged, and the supernatant was diluted with 10 mL of a PBS buffer solution at pH 7.4 and then purified using a MabSlect protein purification column (elution phase: a pH 3.0 aqueous acetate buffer solution). The pH value of the resulting solution was adjusted to about 5.0 with a tris(hydroxymethyl)aminomethane hydrochloride solution (1 M, pH = 8.0) to obtain an affinity eluate of the title product APC-6, which was refrigerated and stored at 4°C.

The average number of conjugated polypeptides per antibody molecule was calculated by MS: y=1.89.

### Test example

### Test example 1. Affinity experiment

### 1-1. Detection of affinity of KLB conjugate molecule for human KLB antigen by BIAcore

The antibody molecule was affinity-captured using a Protein A biosensor chip (Cat. # 29127556, Cytiva), and an antigen molecule was allowed to flow through the surface of the chip. The reaction signals were detected in real time by the Biacore 8K instrument to obtain an association and dissociation curve. After the dissociation in each experimental cycle was complete, the biosensor chip was washed with 10 mM Glycine-HCl (pH 1.5) for regeneration. The data were fitted using a 1:1 model to obtain antibody affinity data. The specific experimental results are shown in Table 4-1 below.

**Table 4-1. Affinity test results of samples for hKLB antigen**

| **Sample** | **ka (1/Ms)** | **kd (1/s)** | **KD (M)** |
|---|---|---|---|
| Ab1 | 1.99E+05 | 4.80E-04 | 2.42E-09 |
| Ab2 | 1.40E+05 | 3.50E-04 | 2.49E-09 |
| APC-1 | 1.63E+05 | 4.24E-04 | 2.59E-09 |
| APC-2 | 1.38E+05 | 2.73E-04 | 1.98E-09 |
| APC-3 | 1.40E+05 | 2.72E-04 | 1.93E-09 |
| APC-4 | 2.34E+05 | 2.54E-04 | 1.09E-09 |

Experimental results indicated that the antibodies or the conjugate molecules of the present disclosure had good binding function to human KLB antigen and that the conjugate molecules had comparable binding ability to that of antibodies Ab1 and Ab2.

### 1-2. Detection of affinity of conjugate molecule for human ActRIIA/B by BIAcore

Antibodies and APC molecules of the present disclosure were tested for affinity for human ActRIIA and ActRIIB with Biacore. The antibody was affinity-captured by a Protein A biosensor chip (Cytiva, 29127556), and then the antigen ActRIIA (Sino Biological) and ActRIIB (Acro Biosystems) were allowed to flow through the surface of the chip. The reaction signals were detected in real time by an instrument to obtain an association and dissociation curve. After the dissociation in each experimental cycle was complete, the biosensor chip was washed with 10 mM Glycine-HCl pH 1.5 for regeneration. The data were fitted using a 1:1 model. The specific experimental results are shown in Table 4-2 and Table 4-3 below.

**Table 4-2. Affinity testing results of samples for ActRIIA antigen**

| **Sample** | **ka (1/Ms)** | **kd (1/s)** | **KD (M)** |
|---|---|---|---|
| Ab3 | 1.98E+05 | 7.24E-04 | 3.66E-09 |
| APC-5 | 2.28E+05 | 7.45E-04 | 3.27E-09 |
| APC-6 | 1.96E+05 | 6.98E-04 | 3.57E-09 |

**Table 4-3. Affinity test results of samples for ActRIIB antigen**

| **Sample** | **ka (1/Ms)** | **kd (1/s)** | **KD (M)** |
|---|---|---|---|
| Ab3 | 4.65E+05 | 2.00E-04 | 4.31E-10 |
| APC-5 | 4.71E+05 | 2.17E-04 | 4.61E-10 |
| APC-6 | 4.27E+05 | 1.75E-04 | 4.11E-10 |

Experimental results indicated that conjugate molecules APC-5 and APC-6 of the present disclosure had comparable affinity for receptors ActRIIA and ActRIIB to that of antibody Ab3.

### Test example 2. Binding experiment

### 2-1. Detection of binding ability of conjugate molecule to cell line overexpressing KLB by flow cytometry

In this test example, flow cytometry was used to detect the binding ability of the conjugate molecules to CHO-K1 cell lines overexpressing human KLB & FGFR1c and monkey KLB & FGFR1c (see Example 1 for specific preparation). Specifically: The above cells were cultured in a culture medium containing 10% FBS in a 37°C, 5% CO₂ incubator for 2 days, then added to a cell plate at 0.8 × 10⁵ cells per well, centrifuged at 300 g for 5 min, and washed once with PBS. The final concentrations of the conjugate molecule or antibody were 11 concentration points obtained by 3-fold serial dilution starting from 50 nM. The conjugate molecule or antibody was added to the cell plate at 100 µL per well, followed by incubation at 4°C for 1 hour. After washing twice with PBS, Alexa488-Goat Anti-human IgG (H+L) fluorescent secondary antibody dilution (1:1000) was added at 100 µL per well, followed by incubation at 4°C for 30 min. The plate was washed three times with PBS, PBS was added at 100 µL per well, and plate reading was performed. The experimental results are shown in Table 5-1.

**Table 5-1. Binding ability of samples to cell lines overexpressing KLB**

| **Sample** | **EC₅₀ (nM)** | |
|---|---|---|
| | **Human KLB** | **Cynomolgus monkey KLB** |
| Ab1 | 0.09511 | 0.2707 |
| Ab2 | 0.09520 | 0.2648 |
| APC-1 | 0.07766 | 0.2570 |
| APC-2 | 0.09288 | 0.2755 |
| APC-4 | 0.06922 | 0.2418 |

Experimental results showed that the conjugate molecules of the present disclosure retained the binding ability of antibodies Ab1 and Ab2 to human and cynomolgus monkey KLB antigens.

### 2-2. Detection of binding ability of conjugate molecule to ActRIIA/B by flow cytometry

A 96-well plate (Thermo, 442404) was coated with human ActRIIA (Sino Biological) and ActRIIB (Acro Biosystems) proteins at 50 ng/well in PBS and incubated overnight at 4°C. After washing the plate, 1% BSA was added, and the plate was incubated at room temperature for 1 hour for blocking. After washing the plate, serially-diluted antibodies were added, and the plate was incubated at room temperature for 1 hour. After washing the plate, HRP-goat anti-human Fc secondary antibody (Goat Anti-Human IgG Antibody, Fc-specific, HRP-conjugated, Sigma) was added, and the plate was incubated at room temperature for 0.5 hours. After washing the plate, a TMB chromogenic substrate (KPL, 52-00-03) was added, and the plate was incubated at room temperature for 5-10 minutes. 1 M H₂SO₄ was added to stop the reaction. The OD450 was read using a VERSAmax plate reader (Molecular Devices). The experimental results are shown in Table 5-2.

**Table 5-2. Binding ability of samples to ActRIIA/B antigens**

| **Sample** | **ActRIIA EC50 (nM)** | **ActRIIB EC50 (nM)** |
|---|---|---|
| Ab3 | 0.217 | 0.555 |
| APC-5 | 0.678 | 0.974 |
| APC-6 | 0.578 | 1.219 |

The experimental results indicated that the binding ability of conjugate molecules APC-5 and APC-6 of the present disclosure to receptors ActRIIA and ActRIIB was slightly weaker than that of antibody Ab3.

### Test example 3: Detection experiment of activation activity of conjugate molecule on KLB & FGFR1c

The activation effect of the conjugate molecules on recombinant cell lines CHO-K1-hKLB & hFGFR1c and CHO-K1-cynoKLB & cynoFGFRlc was tested by in vitro cell experiments. A luciferase reporter gene regulated by GAL4 and a GAL4-Elk1 fusion protein gene were stably transfected into the recombinant cell lines, and the activation activity was expressed as EC₅₀ value.

The experimental method was as follows:
On day 1 of the experiment, CHO-K1-hKLB & hFGFR1c or CHO-K1-cynoKLB & cynoFGFRIc cells were seeded into a 96-well plate (Corning, #3903) at a density of 15000 cells/well using a DMEM/F12 culture medium containing 10% FBS, 10 µg/mL puromycin, and 800 µg/mL G418, with 100 µL of cell suspension per well. Only 100 µL of PBS was added to the periphery of the 96-well plate. The plate was placed in a cell culture incubator at 37°C with 5% CO₂ and incubated overnight. On day 2, the culture medium was discarded, and starvation medium (DMEM/F12 without FBS) was added at 50 µL per well. The cells were transfected with pFA2-Elk1 and pFR-Luc at a ratio of 1:6 using Lipofectamine 3000, and a mixture of the plasmid and Lipofectamine 3000 was added at 10 µL per well. After transfection, the well plate was placed and incubated in an incubator at 37°C with 5% CO₂ for 24 hours. On day 3, the test conjugate molecule or antibody serially diluted with starvation medium was added at 60 µL per well. The final concentrations of the conjugate molecule or antibody were 4-fold serial dilution starting from 100 nM. Starvation medium was set as blank control wells. The well plate was placed and incubated in a cell culture incubator at 37°C with 5% CO₂ for 24 hours. On day 4, the 96-well cell culture plate was taken out, a signal activation fluorescence detection reagent (One-glo Luciferase Assay System, Promega, E6120) was added at 60 µL per well, and the luminescence signal value was read using a multimode microplate reader (EnVision2015, PerkinElmer). Curve fitting was performed using GraphPad Prism based on the logarithmic concentrations of the antibodies and the signal values, and EC₅₀ values were calculated. The experimental results are shown in Table 6.

**Table 6. Experimental results of activation activity of samples on KLB & FGFR1c**

| **Sample** | **EC₅₀ (nM)** | |
|---|---|---|
| | **Human KLB & FGFR1c** | **Cynomolgus monkey KLB & FGFR1c** |
| Ab1 | 0.3213 | 1.258 |
| Ab2 | 0.3125 | 1.556 |
| APC-1 | 0.2341 | 1.27 |
| APC-2 | 0.2006 | 2.013 |
| APC-3 | 0.2861 | 1.525 |
| APC-4 | 0.2737 | 2.189 |

The experimental results indicated that the conjugate molecules of the present disclosure had comparable activation activity on human KLB & FGFR1c and cynomolgus monkey KLB & FGFR1c to that of antibodies Ab1 and Ab2.

### Test example 4: Detection of activation activity of conjugate molecule on CHO-K1/hKLB cells expressing FGFR2c/FGFR3c/FGFR4

By determining the activation effect of the test conjugate molecules on CHO-K1/hKLB cell lines expressing human FGFR2c, FGFR3c, or FGFR4 by in vitro cell experiments, the selectivity of the conjugate molecules for FGFR2c, FGFR3c, or FGFR4 was determined. The specific method was as follows:

On day 1 of the experiment, CHO-K1-hKLB & hFGFR2c, CHO-K1-hKLB & hFGFR3c and CHO-K1-hKLB & hFGFR4 cells were separately seeded into a 96-well plate (Corning, #3903) at a density of 15000 cells/well using a DMEM/F12 culture medium containing 10 µg/mL puromycin with 10% FBS and 800 µg/mL G418, with 100 µL of cell suspension per well. Only 100 µL of PBS was added to the periphery of the 96-well plate. The plate was placed in a cell culture incubator at 37°C with 5% CO₂ and incubated overnight. On day 2, the culture medium was discarded, and starvation medium (DMEM/F12 without FBS) was added at 50 µL per well. The cells were transfected with pFA2-Elk1 and pFR-Luc at a ratio of 1:6 using Lipofectamine 3000, and a mixture of the plasmid and Lipofectamine 3000 was added at 10 µL per well. After transfection, the well plate was placed and incubated in an incubator at 37°C with 5% CO₂ for 24 hours. On day 3, the test conjugate molecule or antibody serially diluted with starvation medium was added at 60 µL per well. The final concentrations of the conjugate molecule or antibody were 9 concentration points obtained by 4-fold serial dilution starting from 200 nM. Starvation medium was set as blank control wells. The well plate was placed and incubated in a cell culture incubator at 37°C with 5% CO₂ for 24 hours. On day 4, the 96-well cell culture plate was taken out, a signal activation fluorescence detection reagent (One-glo Luciferase Assay System, Promega, E6120) was added at 60 µL per well, and the luminescence signal value was read using a multimode microplate reader (EnVision2015, PerkinElmer). Curve fitting was performed using GraphPad Prism based on the logarithmic concentrations of the antibodies and the signal values, and EC₅₀ values were calculated. The experimental results are shown in FIGs. 1-1 to 1-3.

Experimental results showed that the conjugate molecules of the present disclosure had no activation effect on hKLB & FGFR2c, hKLB & FGFR3c, and hKLB & FGFR4, which was consistent with those of antibodies Ab1 and Ab2.

### Test example 5: Detection experiment of agonist activity of conjugate molecule on GLP1R

The agonist effect of the conjugate molecules on recombinant cell lines CHO-K1-hGLP1R/CRE, CHO-K1-cynoGLP1R/CRE, and CHO-K1-mouseGLP1R/CRE was tested by in vitro cell experiments. CRE and a luciferase reporter gene regulated thereby were stably transfected into the recombinant cell lines, and the agonist activity was expressed as EC₅₀ value. The experimental method was as follows:

On day 1 of the experiment, CHO-K1-hGLP1R/CRE or CHO-K1-cynoGLP1R/CRE or CHO-K1-mouseGLP1R/CRE cells were seeded into a 96-well plate (Corning, #3903) at a density of 18000 cells/well using a DMEM/F12 culture medium containing 10% FBS, 10 µg/mL puromycin and 200 µg/mL hygromycin B, with 90 µL of cell suspension per well. Only 100 µL of PBS was added to the periphery of the 96-well plate. The plate was placed in a cell culture incubator at 37°C with 5% CO₂ and incubated overnight. On day 2, the test conjugate molecule serially diluted with PBS was added at 10 µL per well. The final concentrations of the conjugate molecule were 4-fold serial dilution starting from 400 nM. Starvation medium was set as blank control wells. The well plate was placed and incubated in a cell culture incubator at 37°C with 5% CO₂ for 5 hours. The 96-well cell culture plate was taken out, a signal activation fluorescence detection reagent (One-glo Luciferase Assay System, Promega, E6120) was added at 50 µL per well, and the luminescence signal value was read using a multimode microplate reader (EnVision2015, PerkinElmer). Curve fitting was performed using GraphPad Prism based on the logarithmic concentrations of the antibodies and the signal values, and EC₅₀ values were calculated. The experimental results are shown in Table 7-1 and Table 7-2.

**Table 7-1. Experimental results of agonist activity of conjugate molecules APC-1 to APC-3 on GLP1R**

| **Sample** | **Human GLP1R EC₅₀ (nM)** | **Cynomolgus monkey GLP1R EC₅₀ (nM)** | **Murine GLP1R EC₅₀ (nM)** |
|---|---|---|---|
| APC-1 | 0.3640 | 0.09524 | 0.2366 |
| APC-2 | 0.2135 | 0.05605 | 0.1049 |
| APC-3 | 0.1501 | 0.04528 | 0.1016 |

**Table 7-2. Experimental results of agonist activity of conjugate molecules APC-4 to APC-6 on GLP1R**

| **Sample** | **Human GLP1R EC₅₀ (nM)** | **Cynomolgus monkey GLP1R EC₅₀ (nM)** | **Murine GLP1R EC₅₀ (nM)** |
|---|---|---|---|
| APC-4 | 0.1668 | 0.08589 | 0.2136 |
| APC-5 | 0.04467 | 0.04767 | 0.07339 |
| APC-6 | 0.044 | 0.0326 | 0.0593 |

### Test example 6: Detection experiment of agonist activity of conjugate molecule on GIPR

The agonist effect of the conjugate molecules on recombinant cell lines CHO-K1-hGIPR/CRE, CHO-K1-mouseGIPR/CRE and CHO-K1-cynoGIPR/CRE was tested by in vitro cell experiments (Cisbio, 62AM4PEB), and the agonist activity was expressed as EC₅₀ value. The experimental method was as follows:
On day 1 of the experiment, the cells were cultured using a DMEM/F12 culture medium containing 10% FBS, puromycin resistance, and hygromycin B resistance. When the cells grew to 70%-80%, the cells were digested with trypsin. The digestion was stopped using a complete culture medium, and the cells were resuspended and centrifuged. The old medium was discarded, and the cells were resuspended using Buffer A (HBSS + 20 mM HEPES + 0.1% casein), counted, and adjusted to a cell density of 2 × 10⁵/mL. A 2-fold concentration of the antibody was prepared with Buffer B (Buffer A + 2 mM IBMX). 1000 cells were taken and added to a 384-well plate, and 5 µL of the prepared 2-fold concentration of the antibody was added to each well. Centrifugation at low speed was performed, and the plate was incubated at 25°C for 30 min. cAMP-d2 and anti-cAMP-Eu-Cryptate were prepared in the dark, and mixed well with cAMP lysis buffer separately according to a ratio of 1:4 or a ratio of 1:19. The prepared cAMP-d2 solution was added at 5 µL/well, and the anti-cAMP-Eu-Cryptate solution was added at 5 µL/well. The plate was incubated at 25°C for 1 h in the dark. The luminescence signal value was read using a multimode microplate reader (EnVision2015, PerkinElmer). The experimental results are shown in Table 8-1 and Table 8-2.

**Table 8-1. Experimental results of agonist activity of conjugate molecules APC-1 to APC-3 on GIPR**

| **Sample** | **Human GIPR EC₅₀ (nM)** | **Cynomolgus monkey GIPR EC₅₀ (nM)** | **Murine GIPR EC₅₀ (nM)** |
|---|---|---|---|
| APC-1 | 0.01294 | 0.2766 | 0.1592 |
| APC-2 | 0.004450 | 0.7613 | 0.5810 |
| APC-3 | 0.004992 | 0.1696 | 0.2619 |

**Table 8-2. Experimental results of agonist activity of conjugate molecule APC-6 on GIPR**

| **Sample** | **Human GIPR EC₅₀ (nM)** | **Cynomolgus monkey GIPR EC₅₀ (nM)** | **Murine GIPR EC₅₀ (nM)** |
|---|---|---|---|
| APC-6 | 0.01331 | 2.04 | 1.856 |

### Test example 7: Detection experiment of agonist activity of conjugate molecule on GCGR

The agonist effect of the conjugate molecules on recombinant cell lines CHO-K1-hGCGR/CRE, CHO-K1-cynoGCGR/CRE, and CHO-K1-mouseGCGR/CRE was tested by in vitro cell experiments. CRE and a luciferase reporter gene regulated thereby were stably transfected into the recombinant cell lines, and the agonist activity was expressed as EC₅₀ value. The experimental method was as follows:
On day 1 of the experiment, CHO-K1-hGCGR/CRE cells were cultured using a DMEM/F12 culture medium containing 10% FBS, 1 mg/mL G418 and 200 µg/mL hygromycin B (CHO-K1-cynoGCGR/CRE and CHO-K1-mouseGCGR/CRE cells were cultured using a DMEM/F12 culture medium containing 10 µg/mL puromycin and 200 µg/mL hygromycin B). The cells were seeded into a 96-well plate (Corning, #3903) at a density of 18000 cells/well, with 90 µL of cell suspension per well. Only 100 µL of PBS was added to the periphery of the 96-well plate. The plate was placed in a cell culture incubator at 37°C with 5% CO₂ and incubated overnight. On day 2, the test conjugate molecule serially diluted with PBS was added at 10 µL per well. The final concentrations of the conjugate molecule were 4-fold serial dilution starting from 1600 nM. Starvation medium was set as blank control wells. The well plate was placed and incubated in a cell culture incubator at 37°C with 5% CO₂ for 5 hours. The 96-well cell culture plate was taken out, a signal activation fluorescence detection reagent (One-glo Luciferase Assay System, Promega, E6120) was added at 50 µL per well, and the luminescence signal value was read using a multimode microplate reader (EnVision2015, PerkinElmer). Curve fitting was performed using GraphPad Prism based on the logarithmic concentrations of the antibodies and the signal values, and EC₅₀ values were calculated. The experimental results are shown in Table 9-1 and Table 9-2.

**Table 9-1. Experimental results of agonist activity of conjugate molecules APC-1 to APC-3 on GCGR**

| **Sample** | **Human GGCR EC₅₀ (nM)** | **Cynomolgus monkey GCGR EC₅₀ (nM)** | **Murine GCGR EC₅₀ (nM)** |
|---|---|---|---|
| APC-1 | 10.80 | 19.93 | 73.97 |
| APC-2 | 5.543 | 11.12 | 26.72 |
| APC-3 | 2.898 | 8.391 | 48.87 |

**Table 9-2. Experimental results of agonist activity of conjugate molecule APC-6 on GCGR**

| **Sample** | **Human GCGR EC₅₀ (nM)** | **Cynomolgus monkey GCGR EC₅₀ (nM)** | **Murine GCGR EC₅₀ (nM)** |
|---|---|---|---|
| APC-6 | 0.1204 | 0.1809 | 0.4926 |

### Test example 8: Detection experiment of agonist activity of conjugate molecule on MIN-6 cells

The agonist activity of the conjugate molecules on mouse GLP1R, GIPR, and GCGR positive cells MIN-6 was tested by in vitro cell experiments, and the agonist activity was expressed as EC₅₀ value. The experimental method was as follows:

On day 1 of the experiment, MIN-6 cells were cultured using a DMEM (high glucose) culture medium containing 15% FBS and 0.05 mM β-mercaptoethanol. When the cells grew to 70%-80%, the MIN-6 cells were digested with trypsin. The cells were resuspended using HBSS + 20 mM HEPES + 0.1% BSA (buffer A) and centrifuged. The old medium was discarded, and the cells were resuspended using Buffer A, counted, and adjusted to a cell density of 2 × 10⁵/mL. A 2-fold concentration of the antibody (3200 nM) was prepared with Buffer B (Buffer A + 2 mM IBMX). 1000 cells were added to a 384-well plate, and 5 µL of the prepared 2-fold concentration of the antibody was added. Quick centrifugation at low speed was performed, and the plate was incubated at 25°C for 30 min. cAMP-d2 and anti-cAMP-Eu-Cryptate were prepared in the dark, and mixed well with cAMP lysis buffer separately according to a ratio of 1:4. The prepared cAMP-d2 solution was added at 5 µL/well, and the anti-cAMP-Eu-Cryptate solution was added at 5 µL/well. The plate was incubated at 25°C for 1 h in the dark. The luminescence signal value was read using a multimode microplate reader (EnVision2015, PerkinElmer). The experimental results are shown in Table 10.

**Table 10. Detection experiment results of agonist activity of conjugate molecules on MIN-6 cells**

| **Sample** | **EC₅₀ (nM)** |
|---|---|
| APC-1 | 25.76 |
| APC-2 | 26.50 |
| APC-4 | 7.387 |

### Test example 9: Stability determination of peptide in human serum

4 µL of a solution of P2, P3, and a control peptide (HGEGTFTSDVSSYLEEEAAKEFVAWLVKGGG (SEQ ID NO: 35), whose sequence was derived from patent WO 2020125744 A1) was added to 396 µL of pre-incubated serum to a final concentration of 10 µM, and 30 µL of the peptide-serum mixture was taken and placed in a new sterile centrifuge tube, and incubated with shaking at 37°C for 24 hours in a metal bath. Immediately after incubation, 120 µL of a room temperature quenching solution (methanol with internal standard (verapamil, 10 ng/mL)) was added to the peptide-serum mixture sample to stop the reaction. Vortexing was performed for 5 minutes. The sample in the plate was centrifuged at 4°C at 4000 rpm for 15 minutes or at 12000 rpm for 10 minutes to precipitate proteins. 80 µL or 100 µL of the supernatant was transferred to a new 96-well plate, and 80 µL or 100 µL of water was added. LC-MS/MS analysis was performed. The specific experimental results are shown in Table 11.

**Table 11. Stability determination results of P2 and P3 in human serum**

| Time | Residual amount of P2 (%) | Residual amount of P3 (%) | Residual amount of control peptide (%) |
|---|---|---|---|
| 0 hour | 100 | 100 | 100 |
| 24 hours | 75.5 | 109 | 43.1 |

The experimental results indicated that the stability of both P2 and P3 in human serum was superior to that of the control peptide.

### Test example 10: Stability determination of conjugate molecule in human, rhesus monkey, and rat serum

300 µg of the conjugate molecule was dissolved in 1.5 mL of human, rhesus monkey, and rat serum, respectively, and stored in a 37°C constant temperature incubator. Samples were taken on days 1, 2, 7, and 14, respectively, and the changes in the concentrations of the KLB terminus and the peptide terminus of the conjugate molecules were detected by ELISA or HTRF method. The specific experimental results are shown in Table 12.

**Table 12. Stability determination results of conjugate molecules in serum**

| Sample | | Human | | | | Rhesus monkey | | | | Rat | | | |
|---|---|---|---|---|---|---|---|---|---|---|---|---|---|
| | | Day 1 | Day 2 | Day 7 | Day 14 | Day 1 | Day 2 | Day 7 | Day 14 | Day 1 | Day 2 | Day 7 | Day 14 |
| APC-1 | Antibody terminus | 100% | 104% | 98% | 88% | 100% | 95% | 74% | 73% | 100% | 92% | 91% | 88% |
| | Peptide terminus | 100% | 94% | 91% | 85% | 100% | 88% | 74% | 73% | 100% | 95% | 96% | 92% |
| APC-2 | Antibody terminus | 100% | 96% | 92% | 95% | 100% | 103% | 101% | 95% | 100% | 98% | 87% | 89% |
| | Peptide terminus | 100% | 98% | 95% | 107% | 100% | 102% | 95% | 94% | 100% | 96% | 88% | 89% |
| APC-3 | Antibody terminus | 100% | 95% | 91% | 92% | 100% | 95% | 91% | 91% | 100% | 77% | 76% | 85% |
| | Peptide terminus | 100% | 99% | 95% | 94% | 100% | 97% | 109% | 96% | 100% | 90% | 86% | 95% |

The results indicated that APC-1, APC-2, and APC-3 had good stability in serum.

### Test example 11.1: Simultaneous efficacy and PK detection experiment of single administration of conjugate molecule on acute glucose tolerance in C57BL/6J mice

In this experiment, male C57BL/6J mice were selected and administered a single dose. The effect on acute glucose tolerance in mice was detected using IPGTT method. The specific experimental protocol was as follows:

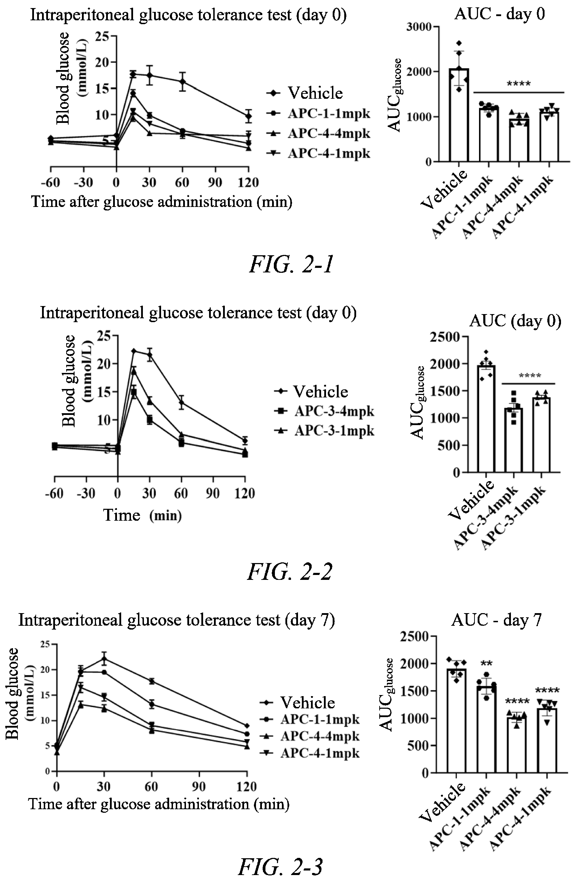

All experimental animals received a single intraperitoneal injection. The administration dose of conjugate molecule APC-1 was 1 mpk, the administration doses of APC-3 were 4 mpk and 1 mpk, and the administration doses of APC-4 were 4 mpk and 1 mpk. One day before the experiment, the animals were randomly grouped according to body weight (n = 6). After fasting for 16 h, fasting blood glucose was measured. Drugs were administered intraperitoneally at 60 min before intraperitoneal glucose injection (-60 min), respectively. At 0 min, blood glucose of the mice in each group was measured, followed by immediate intraperitoneal injection of glucose solution (2 g/kg). Blood glucose of the mice in each group was measured at 15 min, 30 min, 60 min, and 120 min. On day 7, a second IPGTT assay was performed in mice in each group. PK sampling: for the same batch of animals in the IPGTT assay, 100 µL of serum was collected at 3 h, 48 h, 168 h, 336 h, 504 h, and 672 h after administration for serum concentration analysis of the conjugate molecules. The experimental results are shown in Table 13 and FIGs. 2-1 to 2-4.

**Table 13. Pharmacokinetic experimental results in mice**

| C57BL/6J mouse | | APC-1 (1 mpk) | APC-3 (1 mpk) | APC-4 (1 mpk) |
|---|---|---|---|---|
| t_{1/2} (days) | Antibody terminus | 10.3±2.0 | 11.7±1.8 | 12.4±2.4 |
| | Peptide terminus | 9.0±1.2 | 9.5±1.2 | 7.7±1 |
| AUC 0-t | Antibody terminus | 1346±106 | 2587±214 | 1836±156 |
| (ug/mL*h) | Peptide terminus | 1231±94 | 2292±203 | 1527±157 |
| CL (mL/day/kg) | Antibody terminus | 15±1.4 | 7.7±0.9 | 10.4±1.5 |
| | Peptide terminus | 17±1.4 | 9.2±0.9 | 14.6±1.7 |

The experimental results indicated that conjugate molecules APC-1, APC-3, and APC-4 of the present disclosure, when administered as a single dose in wild-type mice, had highly significant acute glucose-lowering ability on day 0 compared with the vehicle group (FIGs. 2-1 and 2-2); and on day 7 after administration, APC-1, APC-3, and APC-4 still had significant acute glucose-lowering ability compared with the vehicle group (FIGs. 2-3 and 2-4).

The PK detection results showed that APC-1, APC-3, and APC-4 all had good stability in mice. Test example 11.2: In vivo stability detection experiment of single administration of conjugate molecule in C57BL/6J mice

In this experiment, male C57BL/6J mice were selected and received a single intraperitoneal injection. The administration doses of conjugate molecules APC-1 and APC-3 were both 20 mpk, and the administration dose of APC-4 was 40 mpk. One day before the experiment, the animals were randomly grouped according to body weight (n = 4 or 6). 100 µL of serum was collected at 3 h, 48 h, 168 h, 336 h, 504 h and 672 h after administration for stability analysis of the conjugate molecules. The experimental results are shown in Table 14.

**Table 14. PAR value analysis of conjugate molecules**

| Sample | PAR value | | | | | |
|---|---|---|---|---|---|---|
| | 3 hours | 48 hours | 168 hours | 336 hours | 504 hours | 672 hours |
| APC-1 | 2 | 2 | 2 | 2 | 2 | 2 |
| APC-3 | 2.1 | 2.0 | 2.0 | 1.9 | 2.0 | 2.0 |
| APC-4 | 1.9 | 2.0 | 1.8 | 1.7 | 1.5 | 1.5 |

The experimental results indicated that conjugate molecules APC-1, APC-3, and APC-4 had good stability in mice.

### Test example 12.1: Efficacy detection experiment of conjugate molecule in DIO mice

Male high-fat diet-induced DIO mice were purchased from GemPharmatech, housed under a 12/12-hour light/dark cycle, and given free access to food and water for acclimatization. Based on body weight and random blood glucose, the mice were divided into 3 groups, with 6 mice in each group. On the day before administration (day -1), blood glucose and body weight of the mice were measured. The experimental animals were administered via intraperitoneal injection. The administration doses of APC-2 were 8 mpk and 2 mpk (once a week), and the administration doses of APC-4 were 8 mpk and 2 mpk (once a week). The detection results are shown in FIGs. 3-1 to 3-7. Statistical analysis was performed using one-way ANOVA (****: P < 0.0001, ***: P < 0.001, **: P < 0.01, *: P < 0.05, relative to the vehicle control group).

The experimental results indicated that after multiple administrations, conjugate molecules APC-2 and APC-4 of the present disclosure demonstrated dose-dependent reductions in body weight (FIG. 3-1), food intake (FIG. 3-2), and random blood glucose (FIG. 3-3) in DIO mice, improved mouse glucose tolerance (FIGs. 3-4 and 3-5), and decreased serum cholesterol, serum low-density lipoprotein cholesterol, liver weight, or serum alanine aminotransferase (FIG. 3-6), and liver lipids (FIG. 3-7).

### Test example 12.2: Efficacy detection experiment of multiple doses of conjugate molecule in DIO mice

Male high-fat diet-induced DIO mice were purchased from GemPharmatech, housed under a 12/12-hour light/dark cycle, and given free access to food and water for acclimatization. After the DIO mice were acclimatized until their body weight stabilised, on day -1, the mice were weighed, random blood glucose was detected, and the mice were randomly grouped (n = 6) based on body weight and food intake (and with reference to random blood glucose). On day 0, the mice in each group were administered the conjugate molecule. The administration doses of APC-3 were 10 mpk, 3 mpk, and 1 mpk (once a week). The detection results are shown in FIGs. 4-1 to 4-6. Data were expressed as mean and standard error of the mean (S.E.M.). Statistical analysis was performed using one-way ANOVA (****: P < 0.0001, ***: P < 0.001, **: P < 0.01, *: P < 0.05, relative to the vehicle control group).

The experimental results indicated that after multiple administrations, conjugate molecule APC-3 of the present disclosure demonstrated dose-dependent reductions in body weight (FIG. 4-1), food intake (FIG. 4-2), and random blood glucose (FIG. 4-3) in DIO mice, improved mouse glucose tolerance (FIGs. 4-4 and 4-5), and reduced mouse liver weight, liver triglycerides, serum cholesterol, serum triglyceride, serum low-density lipoprotein cholesterol, serum alanine aminotransferase, and serum aspartate aminotransferase (FIG. 4-6).

### Test example 13: Efficacy detection experiment of conjugate molecule in db/db mice

Male db/db (BKS) experimental animals were purchased from GemPharmatech, housed under a 12/12-hour light/dark cycle, and given free access to food and water. After acclimatization for 17 days, administration was started. On the day before administration (day -1), HbA1c, blood glucose, and body weight of the mice were detected, and the mice were randomly grouped according to blood glucose (or HbA1c) and body weight, with 6 mice per vehicle administration group and 9 mice per remaining administration group, housed 3 mice per cage. The administration doses of APC-4 were 8 mpk, 2 mpk, and 0.5 mpk (once a week, respectively), and 4 mpk (once every 2 weeks). The detection results are shown in FIGs. 5-1 to 5-6. Data were expressed as mean and standard error of the mean (S.E.M.). Statistical analysis was performed using t-test (****: P < 0.0001, ***: P < 0.001, **: P < 0.01, *: P < 0.05, relative to the vehicle control group).

The experimental results indicated that after first administration, conjugate molecule APC-4 of the present disclosure demonstrated dose-dependent improvement in glucose tolerance of db/db mice (FIG. 5-1); after multiple administrations, APC-4 significantly reduced random blood glucose (FIG. 5-2), fasting blood glucose (FIG. 5-3), HbAlc (FIG. 5-4), serum triglyceride (FIG. 5-5), and food intake (FIG. 5-6) of db/db mice; and the 4 mpk (Q2W) and 2 mpk (QW) administration groups of APC-4 molecule demonstrated comparable efficacy in improving glucose tolerance and reducing random blood glucose, fasting blood glucose, HbA1c, serum triglyceride, and food intake, indicating that APC-4 molecule has good PK in db/db mice.

## Claims

1. An antibody-polypeptide conjugate or a pharmaceutically acceptable salt thereof, having the structure as represented by general formula (I):
Ab-(L-P)ₘ (I)
wherein
Ab is an antibody;
P is a glucagon-like peptide-1 receptor agonist peptide or an analogue thereof;
L is a linker connecting Ab and P;
wherein Ab binds to L via the remodeled glycan chain at Asn297 thereof;
m represents an integer from 1 to 10.

2. The antibody-polypeptide conjugate or the pharmaceutically acceptable salt thereof according to claim 1, wherein the structure of the remodeled glycan chain is: 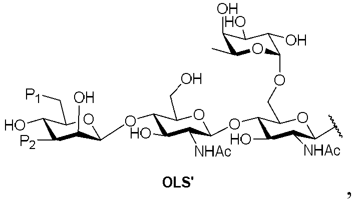
in the formula, the wavy line 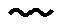 indicates binding to Asn at position 297 of the Pc heavy chain;
P₁ and P₂ are identical or different, and are each independently selected from the group consisting of hydroxyl, *-(CR^{p}R^{q}-CR^{s}R^{t}-O)s¹- and *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino; the asterisk * indicates binding to the linker L;
s¹ is 1-10, preferably 1-5; s² is 0-10, preferably 1-5; s³ is 1-10, preferably 1-5; s⁴ is 0-10, preferably 1-5;
provided that P₁ and P₂ are not both hydroxyl or *-(CH₂CH₂O)s¹-;
preferably, P₁ is hydroxyl, and P₂ is *-(CR^{p}R^{q}-CR^{s}R^{t}-O)s²-(CR^{p}R^{q}-CR^{s}R^{t}-CR^{x}R^{y}-O)s³-(CR^{p}R^{q}-CR^{s}R^{t}-O)s⁴-, wherein R^{p}, R^{q}, R^{s}, R^{t}, R^{x} and R^{y} are identical or different, and are each independently selected from the group consisting of hydrogen atom, halogen, alkyl and haloalkyl, s² is 1, s³ is 1, and s⁴ is 1;
more preferably, the structure of the remodeled glycan chain is:

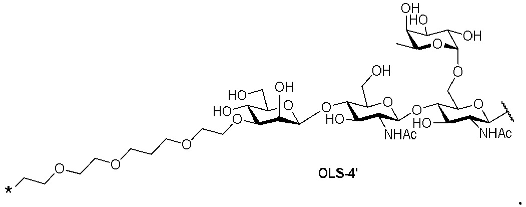

3. The antibody-polypeptide conjugate or the pharmaceutically acceptable salt thereof according to claim 1 or 2, having the structure as represented by general formula (II): 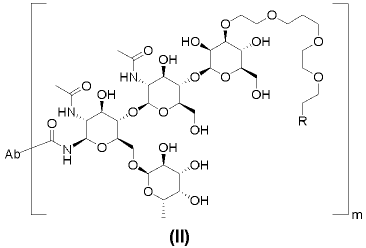
wherein R is -L-P;
Ab, L, P and m are as defined in claim 1.

4. The antibody-polypeptide conjugate or the pharmaceutically acceptable salt thereof according to any one of claims 1 to 3, wherein P is a GLP-1R monoagonist peptide or a GLP-1R/GIPR/GCGR triagonist peptide or an analogue thereof; preferably, P comprises the amino acid sequence of SEQ ID NO: 20, SEQ ID NO: 21 or SEQ ID NO: 22.

5. The antibody-polypeptide conjugate or the pharmaceutically acceptable salt thereof according to any one of claims 1 to 4, wherein Ab is selected from the group consisting of IgG1, IgG2, IgG3, and IgG4 antibodies; preferably, Ab is an IgG1 antibody.

6. The antibody-polypeptide conjugate or the pharmaceutically acceptable salt thereof according to any one of claims 1 to 5, wherein Ab is a KLB antibody or an ActRIIA/B antibody;
preferably, (1) the HCDR1 of the heavy chain variable region of Ab comprises the amino acid sequence of SEQ ID NO: 6, the HCDR2 comprises the amino acid sequence of SEQ ID NO: 7, the HCDR3 comprises the amino acid sequence of SEQ ID NO: 8, the LCDR1 of the light chain variable region comprises the amino acid sequence of SEQ ID NO: 9, the LCDR2 comprises the amino acid sequence of SEQ ID NO: 10, and the LCDR3 comprises the amino acid sequence of SEQ ID NO: 11; or (2) the HCDR1 of the heavy chain variable region of Ab comprises the amino acid sequence of SEQ ID NO: 23, the HCDR2 comprises the amino acid sequence of SEQ ID NO: 24, the HCDR3 comprises the amino acid sequence of SEQ ID NO: 25, the LCDR1 of the light chain variable region comprises the amino acid sequence of SEQ ID NO: 26, the LCDR2 comprises the amino acid sequence of SEQ ID NO: 27, and the LCDR3 comprises the amino acid sequence of SEQ ID NO: 28;
more preferably, (1) the heavy chain variable region of Ab comprises the amino acid sequence of SEQ ID NO: 12, and the light chain variable region comprises the amino acid sequence of SEQ ID NO: 13; or (2) the heavy chain variable region of Ab comprises the amino acid sequence of SEQ ID NO: 29, and the light chain variable region comprises the amino acid sequence of SEQ ID NO: 30;
most preferably, (1) the heavy chain of Ab comprises the amino acid sequence of SEQ ID NO: 16 or 19, and the light chain comprises the amino acid sequence of SEQ ID NO: 17; or (2) the heavy chain of Ab comprises the amino acid sequence of SEQ ID NO: 33, and the light chain comprises the amino acid sequence of SEQ ID NO: 34.

7. The antibody-polypeptide conjugate or the pharmaceutically acceptable salt thereof according to any one of claims 1 to 6, wherein L is -L^{a}-L^{b}-L^{c}-L^{d}-L^{e}-,
L^{a} is selected from the group consisting of: 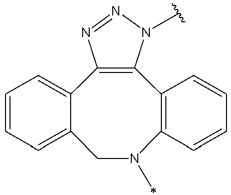
and 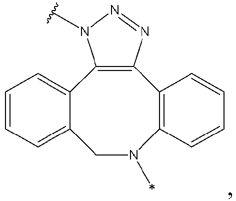 wherein the asterisk * indicates binding to L^{b}, and the wavy line 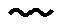 indicates binding to the remodeled glycan chain of Ab;
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, - C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and - O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino;
alternatively, R^{a} and R^{b}, together with the carbon atom to which they are attached, form cycloalkyl or heterocyclyl, R^{c} and R^{d}, together with the carbon atom to which they are attached, form cycloalkyl or heterocyclyl, R^{f} and R^{g}, together with the carbon atom to which they are attached, form cycloalkyl or heterocyclyl, Rⁱ and R^{j}, together with the carbon atom to which they are attached, form cycloalkyl or heterocyclyl, and R^{m} and Rⁿ, together with the carbon atom to which they are attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino;
alternatively, R^{a} and R^{c}, together with the carbon atoms to which they are respectively attached, form cycloalkyl or heterocyclyl, and Rⁱ and R^{m}, together with the carbon atoms to which they are respectively attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino.

8. The antibody-polypeptide conjugate or the pharmaceutically acceptable salt thereof according to claim 7, wherein L^{b} is -C(O)-CR^{a}R^{b}-CR^{c}R^{d}-C(O)-, and R^{a}, R^{b}, R^{c} and R^{d} are identical or different, and are each independently a hydrogen atom or C₁₋₆ alkyl; preferably, L^{b} is -C(O)-CH₂-CH₂-C(O)-.

9. The antibody-polypeptide conjugate or the pharmaceutically acceptable salt thereof according to claim 7, wherein L^{c} is -NR^{h}-CRⁱR^{j}-CR^{m}Rⁿ-, and R^{h}, Rⁱ, R^{j}, R^{m} and Rⁿ are identical or different, and are each independently a hydrogen atom or C₁₋₆ alkyl; preferably, L^{c} is -NH-CH₂-CH₂-.

10. The antibody-polypeptide conjugate or the pharmaceutically acceptable salt thereof according to claim 7, wherein L^{d} is 1 to 36 -O-CH₂-CH₂- units; preferably, L^{d} is 4 to 24 -O-CH₂-CH₂- units; more preferably, L^{d} is 4, 8 , 12 or 24 - O-CH₂-CH₂- units; most preferably, L^{d} is 8 -O-CH₂-CH₂- units.

11. The antibody-polypeptide conjugate or the pharmaceutically acceptable salt thereof according to any one of claims 1 to 10, wherein L is 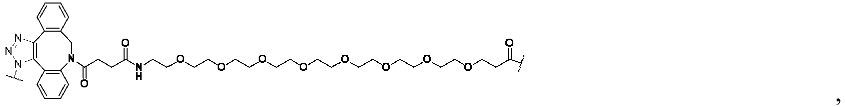 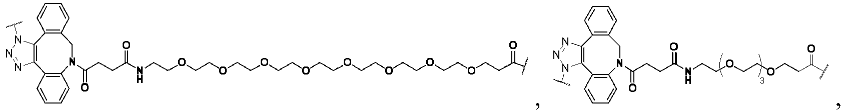 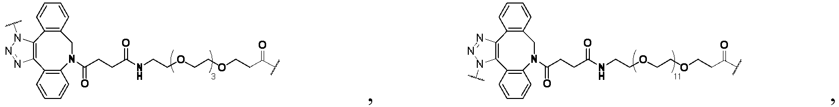 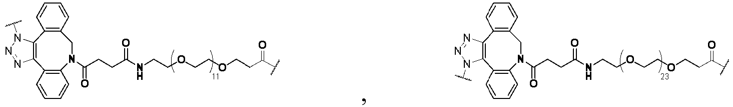 or

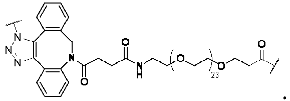

12. The antibody-polypeptide conjugate or the pharmaceutically acceptable salt thereof according to any one of claims 1 to 11, having the structure as represented by general formula (II): 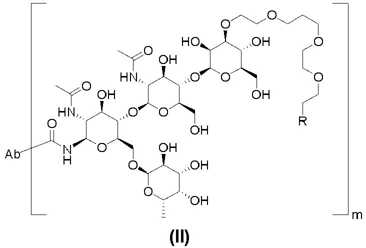 wherein 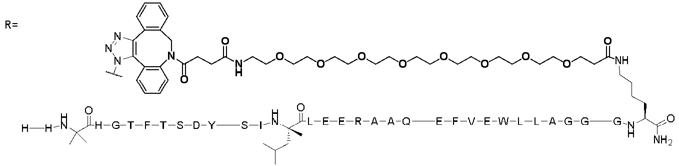 or 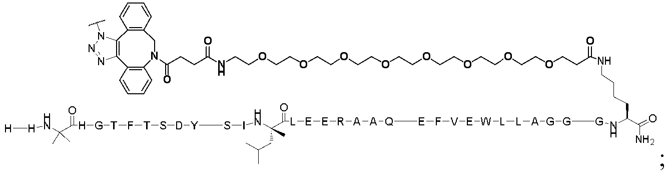 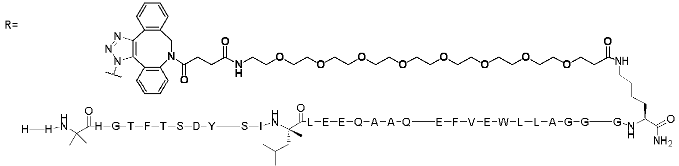 or 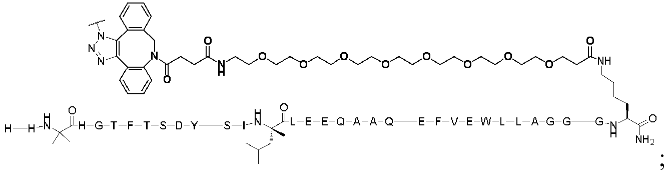 or 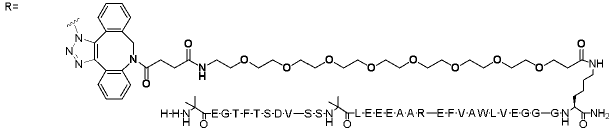 or 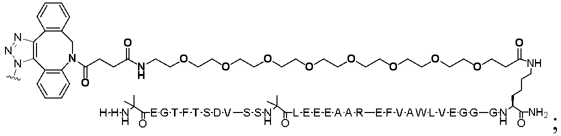 Ab and m are as defined in claim 1.

13. A glucagon-like peptide-1 receptor agonist peptide or an analogue thereof, comprising the amino acid sequence of SEQ ID NO: 20, SEQ ID NO: 21 or SEQ ID NO: 22.

14. A compound as represented by general formula (Ib) or a salt thereof:
L'-P (Ib)
wherein
P is a glucagon-like peptide-1 receptor agonist peptide or an analogue thereof; preferably, P is a GLP-1R monoagonist peptide or a GLP-1R/GIPR/GCGR triagonist peptide or an analogue thereof; more preferably, P comprises the amino acid sequence of SEQ ID NO: 20, SEQ ID NO: 21 or SEQ ID NO: 22;
L' is L^{aa}-L^{b} -L^{c}-L^{d}-L^{e}-,
L^{aa} is selected from: 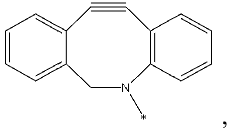 wherein the asterisk * indicates binding to L^{b};
L^{b} is selected from the group consisting of -C(O)-CR^{a}R^{b}-CR^{c}R^{d}-C(O)-, -C(O)-(CR^{a}R^{b}-CR^{c}R^{d})₂-C(O)-, -C(O)-CR^{a}R^{b}-CR^{c}R^{d}-C(O)-NR^{e}-(CR^{a}R^{b}-CR^{c}R^{d})₂-C(O)-, - C(O)-CR^{a}R^{b}-CR^{c}R^{d}-C(O)-NR^{e}-(CR^{a}R^{b}-CR^{c}R^{d}-O)₂-CR^{f}R^{g}-C(O)-, -C(O)-CR^{a}R^{b}-CR^{c}R^{d}-NR^{e}-C(O)-(CR^{a}R^{b}-CR^{c}R^{d}-O)₄-CR^{a}R^{b}-CR^{c}R^{d}-C(O)-, -CR^{f}R^{g}-O-C(O)- and - O-C(O)-;
L^{c} is -NR^{h}-CRⁱR^{j}-CR^{m}Rⁿ-;
L^{d} is a PEG unit;
L^{e} is -C(O)-;
R^{a}, R^{b}, R^{c}, R^{d}, R^{e}, R^{f}, R^{g}, R^{h}, Rⁱ, R^{j}, R^{m} and Rⁿ are identical or different, and are each independently selected from the group consisting of hydrogen atom, halogen, alkyl, haloalkyl, alkoxy, hydroxyalkyl, hydroxyl, cyano, amino, cycloalkyl, heterocyclyl, aryl and heteroaryl, the cycloalkyl, heterocyclyl, aryl and heteroaryl being optionally substituted with one or more substituents selected from the group consisting of oxo, halogen, alkyl, haloalkyl, alkoxy, hydroxyalkyl, hydroxyl, cyano and amino;
alternatively, R^{a} and R^{b}, together with the carbon atom to which they are attached, form cycloalkyl or heterocyclyl, R^{c} and R^{d}, together with the carbon atom to which they are attached, form cycloalkyl or heterocyclyl, R^{f} and R^{g}, together with the carbon atom to which they are attached, form cycloalkyl or heterocyclyl, R' and R^{j}, together with the carbon atom to which they are attached, form cycloalkyl or heterocyclyl, and R^{m} and Rⁿ, together with the carbon atom to which they are attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino;
alternatively, R^{a} and R^{c}, together with the carbon atoms to which they are respectively attached, form cycloalkyl or heterocyclyl, and Rⁱ and R^{m}, together with the carbon atoms to which they are respectively attached, form cycloalkyl or heterocyclyl, the cycloalkyl or heterocyclyl being optionally substituted with one or more substituents selected from the group consisting of oxo, halogen, alkyl, haloalkyl, alkoxy, hydroxyalkyl, hydroxyl, cyano and amino.

15. The compound as represented by general formula (Ib) or the salt thereof according to claim 14, wherein L^{aa} is selected from: 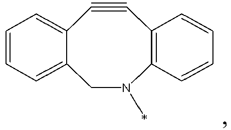 wherein the asterisk * indicates binding to L^{b};
and/or L^{b} is -C(O)-CR^{a}R^{b}-CR^{c}R^{d}-C(O)-, and R^{a}, R^{b}, R^{c} and R^{d} are identical or different, and are each independently a hydrogen atom or C₁₋₆ alkyl; preferably, L^{b} is -C(O)-CH₂-CH₂-C(O)-;
and/or L° is -NR^{h}-CRⁱR^{j}-CR^{m}Rⁿ-, and R^{h}, Rⁱ, R^{j}, R^{m} and Rⁿ are identical or different, and are each independently a hydrogen atom or C₁₋₆ alkyl; preferably, L^{c} is -NH-CH₂-CH₂-;
and/or L^{d} is 1 to 36 -O-CH₂-CH₂- units; preferably, L^{d} is 4 to 24 -O-CH₂-CH₂-units; more preferably, L^{d} is 4, 8 , 12 or 24 -O-CH₂-CH₂- units; most preferably, L^{d} is 8 -O-CH₂-CH₂- units;
and/or L^{e} is -C(O)-.

16. The compound as represented by general formula (Ib) or the salt thereof according to claim 14 or 15, wherein the compound is selected from the group consisting of the following structures: 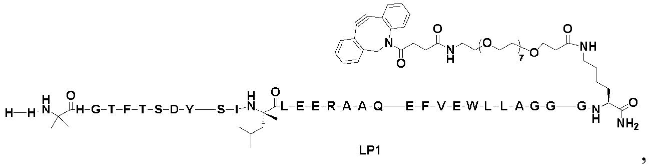 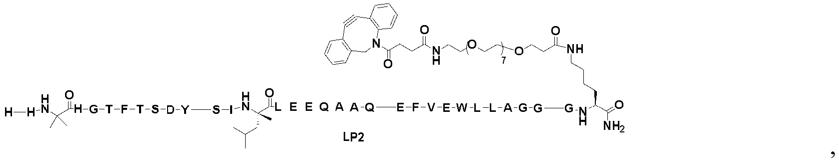 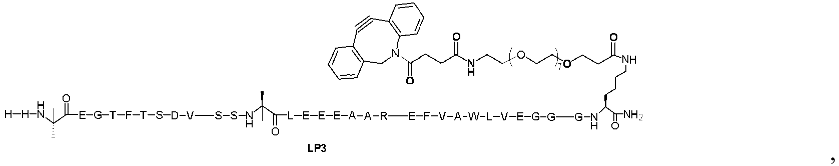 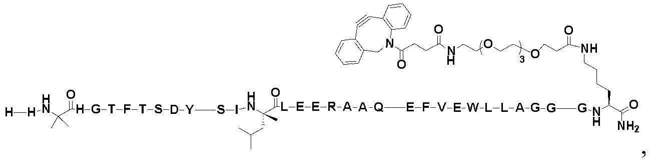 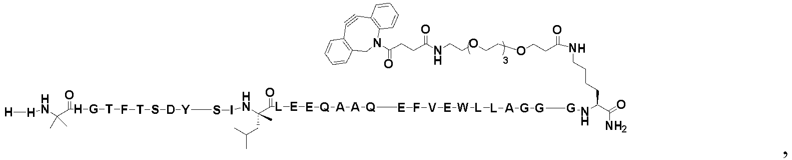 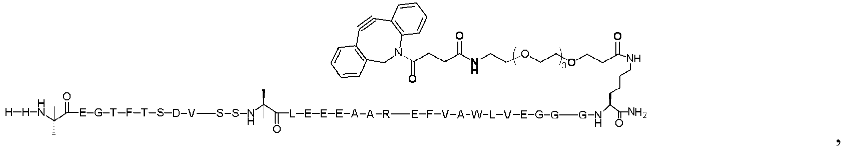 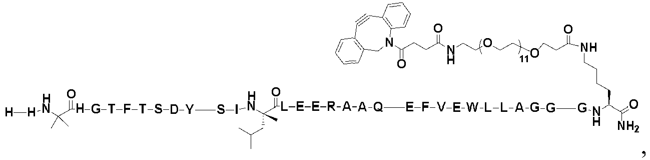 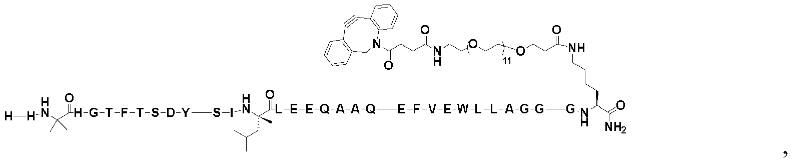 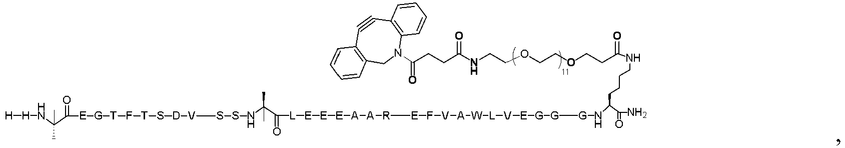 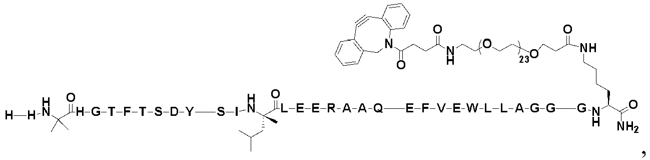 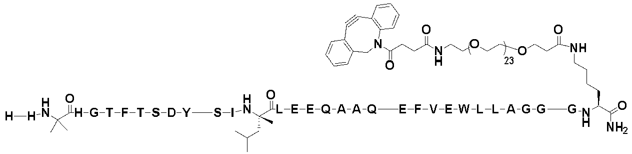 or

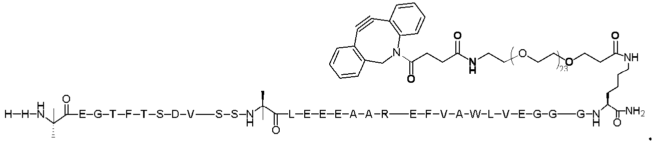

17. A method for preparing an antibody-polypeptide conjugate as represented by general formula (II) or a pharmaceutically acceptable salt thereof, comprising the following steps: 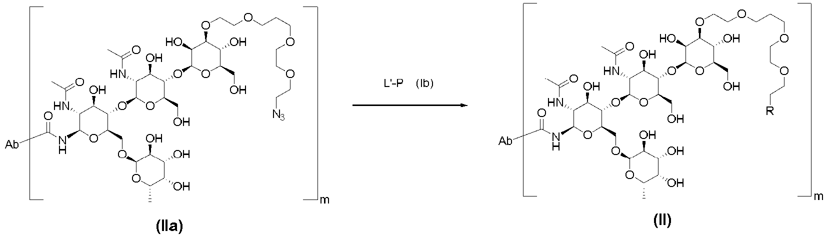
reacting a compound as represented by general formula (IIa) or a salt thereof with a compound as represented by general formula (Ib) or a salt thereof to obtain the antibody-polypeptide conjugate as represented by general formula (II) or the pharmaceutically acceptable salt thereof;
wherein R is -L-P;
Ab, L, P and m are as defined in claim 1, and L' is as defined in claim 14.

18. A pharmaceutical composition, comprising the antibody-polypeptide conjugate or the pharmaceutically acceptable salt thereof according to any one of claims 1 to 12, the glucagon-like peptide-1 receptor agonist peptide or the analogue thereof according to claim 13, and one or more pharmaceutically acceptable carriers, diluents or excipients.

19. A method for preventing or treating a disease, comprising administering to a subject the antibody-polypeptide conjugate or the pharmaceutically acceptable salt thereof according to any one of claims 1 to 12, the glucagon-like peptide-1 receptor agonist peptide or the analogue thereof according to claim 13, or the pharmaceutical composition according to claim 18; preferably, the disease is diabetes, obesity, liver disease, coronary artery disease or kidney disease; more preferably, the disease is type II diabetes, obesity, metabolic dysfunction-associated steatotic liver disease and metabolic dysfunction-associated steatohepatitis (non-alcoholic steatohepatitis).
